(19) Europäisches Patentamt
European Patent Office
Office européen des brevets

(11) Veröffentlichungsnummer: **0 144 290**
**A2**

# (12) EUROPÄISCHE PATENTANMELDUNG

(21) Anmeldenummer: 84810575.5

(22) Anmeldetag: 26.11.84

(51) Int. Cl.⁴: **C 07 K 5/06**
C 07 K 5/08, A 61 K 37/02
A 61 K 37/64

(30) Priorität: 01.12.83 CH 6436/83

(43) Veröffentlichungstag der Anmeldung:
12.06.85 Patentblatt 85/24

(84) Benannte Vertragsstaaten:
AT BE CH DE FR GB IT LI LU NL SE

(71) Anmelder: CIBA-GEIGY AG
Postfach
CH-4002 Basel(CH)

(72) Erfinder: Rasetti, Vittorio, Dr.
Passwangstrasse 6
CH-4059 Basel(CH)

(72) Erfinder: Bühlmayer, Peter, Dr.
Im Baumgarten 3
CH-4144 Arlesheim(CH)

(72) Erfinder: Fuhrer, Walter, Dr.
Munzacherweg 11
CH-4402 Frenkendorf(CH)

(72) Erfinder: Andreatta, Rudolf Heinrich, Dr.
Ochsengasse 22
CH-4123 Allschwil(CH)

(72) Erfinder: Caselli, Anthony
Gundeldingerstrasse 65
CH-4053 Basel(CH)

(72) Erfinder: Renner, Ulrich, Dr.
Auf der Bischoffhöhe 26
CH-4125 Riehen(CH)

## (54) Substituierte Aethylendiaminderivate.

(57) Aethylendiaminderivate der Formel

$$R_1-X_1-X_2-\underset{R_3}{\overset{R_2}{N}}-CH-CH_2-\overset{R_4}{N}-\overset{R_5}{CH}-\overset{O}{\overset{\|}{C}}-R_6 \quad (I),$$

worin $R_1$ Wasserstoff oder Acyl, $X_1$ einen gegebenenfalls N-alkylierten natürlichen Aminosäurerest, der N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2-$ verbunden ist, $R_2$ Wasserstoff oder Niederalkyl, $R_3$ und $R_5$ unabhängig voneinander Wasserstoff, unsubstituiertes oder substituiertes Niederalkyl, unsubstituiertes oder substituiertes Arylniederalkyl oder Aryl, $R_4$ Wasserstoff, Niederalkyl oder Acyl und $R_6$ substituiertes Amino oder substituiertes Hydroxy darstellen, und Salze von solchen Verbindungen hemmen die blutdrucksteigernde Wirkung des Enzyms Renin und können als Antihypertensiva verwendet werden.

EP 0 144 290 A2

CIBA-GEIGY AG 4-14674/+

Basel (Schweiz)

## Substituierte Aethylendiaminderivate

Die Erfindung betrifft substituierte Aethylendiaminderivate der Formel

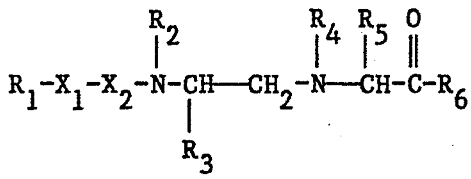

(I),

worin $R_1$ Wasserstoff oder Acyl, $X_1$ einen gegebenenfalls N-alkylierten natürlichen Aminosäurerest, der N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2-$ verbunden ist, $R_2$ Wasserstoff oder Niederalkyl, $R_3$ und $R_5$ unabhängig voneinander Wasserstoff, unsubstituiertes oder substituiertes Niederalkyl, unsubstituiertes oder substituiertes Arylniederalkyl oder Aryl, $R_4$ Wasserstoff, Niederalkyl oder Acyl und $R_6$ substituiertes Amino oder substituiertes Hydroxy darstellen, mit Ausnahme der Verbindungen, worin $R_1$ einen gegebenenfalls N-acylierten Rest der Aminosäure L-Prolin oder worin $X_1$ und $X_2$ zusammen -Arg-Pro-, -Tyr-Gly- oder -Gly-Gly- bedeuten, ferner Salze von solchen Verbindungen mit salzbildenden Gruppen, Verfahren zur Herstellung dieser Verbindungen, pharmazeutische Präparate mit diesen Verbindungen und die Verwendung dieser Verbindungen als Arzneimittel oder zur Herstellung von pharmazeutischen Präparaten, sowie Zwischenprodukte.

In der Beschreibung der vorliegenden Erfindung bedeutet der bei der Definition von Gruppen oder Resten, z.B. Niederalkyl, Niederalkoxy,

Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Die durch $R_3$ und $R_5$ substituierten C-Atome können die R-, S- oder R,S-Konfiguration haben. Bevorzugt sind Verbindungen der Formel I, worin diese C-Atome die S-Konfiguration aufweisen.

Die in der Beschreibung der vorliegenden Erfindung verwendeten allgemeinen Ausdrücke und Bezeichnungen haben vorzugsweise die folgenden Bedeutungen:

Acyl $R_1$ oder $R_4$ hat z.B. bis zu 19 C-Atome und ist in erster Linie die Acylgruppe einer Carbonsäure, eines Halbesters der Kohlensäure, der Carbaminsäure, einer N-substituierten Carbaminsäure, der Thiocarbaminsäure, einer Sulfonsäure, der Amidosulfonsäure oder einer N-substituierten Amidosulfonsäure.

Acyl $R_1$ oder $R_4$ hat beispielsweise die Teilformeln $R^b$-CO-, $R^a$-O-CO-($R^b$)($R^b$)N-CO-, ($R^b$)($R^b$)N-CS-, $R^b$-$SO_2$- oder ($R^b$)($R^b$)N-$SO_2$-, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18, bevorzugt bis einschliesslich 10, C-Atomen oder einen unsubstituierten oder substituierten aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen oder einen unsubstituierten oder substituierten, gesättigten, fünf- oder sechsgliedrigen Heterocyclus darstellt und $R^b$ Wasserstoff bedeutet oder die Bedeutungen von $R^a$ hat. In Gruppen, in denen $R^b$ zweimal vorkommt, können beide Reste $R^b$ gleich oder verschieden sein.

Ein unsubstituierter oder substituierter, gesättigter oder ungesättigter, aliphatischer, cycloaliphatischer oder cycloaliphatischaliphatischer Kohlenwasserstoffrest $R^a$ oder $R^b$ ist beispielsweise unsubstituiertes oder substituiertes Alkyl, z.B. Niederalkyl, Niederalkenyl, Niederalkinyl, Mono-, Bi- oder Tricycloalkyl, Monocycloalkenyl, Bicycloalkenyl, Cycloalkylniederalkyl, Cycloalkylniederalkenyl oder Cycloalkenylniederalkyl.

Alkyl $R^a$ oder $R^b$ hat vorzugsweise 1-10 C-Atome und ist z.B. Niederalkyl mit 1-7 C-Atomen oder n-Octyl, n-Nonyl oder n-Decyl.

Niederalkyl $R^a$ oder $R^b$ hat vorzugsweise 1-7 C-Atome und ist z.B. Methyl, Aethyl, n-Propyl, Isopropyl, n-Butyl, tert-Butyl oder n-Hexyl, welche durch eine oder mehrere funktionelle Gruppen, beispielsweise Hydroxy, veräthertes Hydroxy, z.B. Niederalkoxy, wie Methoxy oder Aethoxy, verestertes Hydroxy, z.B. Niederalkanoyloxy, wie Acetoxy, Halogen, z.B. Chlor oder Brom, Hydroxysulfonyloxy, Carboxy, verestertes Carboxy, z.B. Niederalkoxycarbonyl, wie Methoxy- oder Aethoxycarbonyl, oder amidiertes Carboxy, z.B. Carbamoyl oder Mono- oder Diniederalkylcarbamoyl, wie Methyl- oder Dimethylcarbamoyl, substituiert sein können, wobei die Substituenten nur dann in 1-Stellung des Niederalkylrests stehen, wenn dieser in der Teilformel $R^b$-CO- an die Carbonylgruppe gebunden ist.

Substituiertes Niederalkyl $R^a$ oder $R^b$ ist beispielsweise Hydroxyniederalkyl, z.B. 2-Hydroxyäthyl, Niederalkoxyniederalkyl, z.B. Niederalkoxymethyl oder Niederalkoxyäthyl, wie Methoxymethyl oder 2-Methoxyäthyl, Niederalkoxyniederalkoxyniederalkyl, z.B. 2-Methoxyäthoxymethyl, Niederalkoxyniederalkoxyniederalkoxyniederalkyl, z.B. 2-(2-Methoxyäthoxy)-äthoxymethyl, Niederalkanoyloxyniederalkyl, z.B. Niederalkanoyloxymethyl oder Niederalkanoyloxyäthyl, wie Acetoxymethyl oder 2-Acetoxyäthyl, Halogenniederalkyl, z.B. Halogenmethyl oder Halogenäthyl, wie 2-Chlor- oder 2-Bromäthyl oder 1,1-Dimethyl-2,2,2-trichloräthyl, Hydroxysulfonyloxyniederalkyl, z.B. Hydroxysulfonyloxymethyl oder 2-Hydroxysulfonyloxyäthyl, Carboxyniederalkyl, z.B. Carboxymethyl oder 2-Carboxyäthyl, Niederalkoxycarbonylniederalkyl, z.B. Niederalkoxycarbonylmethyl oder Niederalkoxycarbonyläthyl, wie Methoxycarbonylmethyl, 2-Methoxycarbonyläthyl, Aethoxycarbonylmethyl oder 2-Aethoxycarbonyläthyl, Carbamoylniederalkyl, z.B. Carbamoylmethyl oder 2-Carbamoyläthyl, Niederalkylcarbamoylniederalkyl, z.B. Methylcarbamoylmethyl, Diniederalkylcarbamoylniederalkyl, z.B. Dimethylcarbamoylmethyl, Aminoniederalkyl, z.B. Aminomethyl oder 3-Aminopropyl, Niederalkylaminoniederalkyl, z.B. Methylaminomethyl, Hydroxyniederalkylaminoalkyl, z.B. 2-Hydroxyäthylaminomethyl, oder Diniederalkylaminoniederalkyl, z.B. Dimethylaminomethyl.

Niederalkenyl $R^a$ oder $R^b$ enthält z.B. 2-7, insbesondere 2-4, C-Atome, wobei die Doppelbindung nur dann in 1-Stellung des Niederalkenylrests steht, wenn dieser in der Teilformel $R^b$-CO- an die Carbonylgruppe gebunden ist, und ist z.B. Vinyl, Allyl oder 2- oder 3-Butenyl. Niederalkenyl $R^a$ oder $R^b$ kann durch die gleichen Substituenten substituiert sein wie Niederalkyl, z.B. durch Hydroxy, veräthertes Hydroxy, z.B. Methoxy, verestertes Hydroxy, z.B. Acetoxy, Halogen, z.B. Chlor oder Brom, Carboxy, verestertes Carboxy, z.B. Methoxycarbonyl oder Aethoxycarbonyl, oder amidiertes Carboxy, z.B. Carbamoyl.

Niederalkinyl $R^a$ oder $R^b$ enthält z.B. 2-7, insbesondere 2-4, C-Atome und ist beispielsweise Aethinyl, 1-Propinyl oder 2-Propinyl.

Cycloalkyl $R^a$ oder $R^b$ enthält z.B. 3-8, insbesondere 3-6, C-Atome und ist z.B. Cyclopropyl, Cyclobutyl, Cyclopentyl oder Cyclohexyl.

Bicycloalkyl $R^a$ oder $R^b$ enthält z.B. 5-10, insbesondere 6-9, C-Atome und ist z.B. Bicyclohexyl, -heptyl, -octyl, -nonyl oder -decyl, z.B.

Bicyclo[3.1.0]hex-1-, -2- oder -3-yl, Bicyclo[4.1.0]hept-1- oder -4-yl, Bicyclo[2.2.1]hept-2-yl, z.B. endo- oder exo-Norbornyl, Bicyclo[3.2.1]oct-2-yl, Bicyclo[3.3.0]oct-3-yl oder Bicyclo[3.3.1]-non-9-yl.

Tricycloalkyl $R^a$ oder $R^b$ enthält z.B. 8-10 C-Atome und ist z.B. Tricyclo[$5.2.1.0^{2,6}$]dec-8-yl oder Adamantyl, wie 1-Adamantyl.

Monocycloalkenyl $R^a$ oder $R^b$ enthält z.B. 3-8, insbesondere 3-6, C-Atome und ist beispielsweise Cyclohexenyl, z.B. 1-Cyclohexenyl, oder Cyclohexadienyl, z.B. 1,4-Cyclohexadienyl.

Bicycloalkenyl $R^a$ oder $R^b$ enthält z.B. 5-10, insbesondere 7-10, C-Atome und ist z.B. Bicyclo[2.2.1]hept-5-en-yl, z.B. 5-Norbornen-2-yl, Bicyclo[2.2.2]octen-2-yl oder Hexahydro-4,7-methanoind-1-en-6-yl.

Cycloalkylniederalkyl $R^a$ oder $R^b$ enthält z.B. 4-10, insbesondere 4-7, C-Atome und ist beispielsweise Cyclopropylmethyl, Cyclobutylmethyl, Cyclopentylmethyl oder Cyclohexylmethyl.

Cycloalkylniederalkenyl $R^a$ oder $R^b$ enthält z.B. 5-10, insbesondere 5-9, C-Atome und ist beispielsweise Cyclohexylvinyl oder Cyclohexylallyl.

Cycloalkenylniederalkyl $R^a$ oder $R^b$ enthält z.B. 4-10, insbesondere 4-7, C-Atome und ist beispielsweise 1-Cyclohexenylmethyl oder 1,4-Cyclohexadienylmethyl.

Die genannten cycloaliphatischen oder cycloaliphatisch-aliphatischen Reste können durch die gleichen Substituenten wie Niederalkyl $R^a$ sowie durch Oxo substituiert sein.

Ein aromatischer bzw. aromatisch-aliphatischer Kohlenwasserstoffrest $R^a$ oder $R^b$ ist beispielsweise unsubstituiertes oder substituiertes

Phenyl bzw. Phenylniederalkyl, z.B. Halophenyl, z.B. 4-Chlorphenyl, Niederalkoxyphenyl, z.B. 4-Methoxyphenyl, oder Nitrophenyl, Benzyl, Niederalkylbenzyl, z.B. 4-Methylbenzyl, Niederalkoxybenzyl, z.B. 4-Methoxybenzyl, Carboxybenzyl, z.B. 3-Carboxybenzyl, Niederalkoxycarbonylbenzyl, z.B. 3-Methoxy- oder 3-tert-Butoxycarbonylbenzyl, Carbamoylbenzyl, z.B. 3-Carbamoylbenzyl, 2-Phenyläthyl, Diphenylmethyl, Di-(4-methoxyphenyl)-methyl, oder Trityl, oder Phenylniederalkenyl, z.B. 3-Phenylallyl.

In einem heteroaromatischen bzw. heteroaromatisch-aliphatischen Kohlenwasserstoffrest $R^a$ oder $R^b$ ist der Heterocyclus fünf- oder sechsgliedrig und enthält ein bis zwei Stickstoffatome und/oder ein Sauerstoff- oder Schwefelatom und ist mit einem seiner Ringkohlenstoffatome mit der Gruppe -CO- bzw. -O-CO-, >N-CO-, >N-CS-, $-SO_2-$ oder $>N-SO_2-$ verknüpft. Dieser Heterocyclus kann an einem Stickstoffatom durch Niederalkyl, z.B. Methyl oder Aethyl, Phenyl, oder Phenylniederalkyl, z.B. Benzyl, substituiert und/oder benzanelliert sein und ist beispielsweise 2- oder 3-Pyrrolyl, 2-Furanyl, 2-Thiophenyl, 4-Imidazolyl, 2-, 3- oder 4-Pyridyl, 3-Indolyl, 2-, 3- oder 4-Chinolinyl, 1-, 3- oder 4-Isochinolinyl, 2-Benzoxazolyl oder 2-Benzthiazolyl.

Die aromatisch-aliphatischen oder heteroaromatisch-aliphatischen Reste können im aliphatischen Teil durch die gleichen Substituenten wie Niederalkyl $R^a$ substituiert sein.

Ein gesättigter, fünf- oder sechsgliedriger Heterocyclus $R^a$ oder $R^b$ hat mindestens ein C-Atom, 1-3 Stickstoffatome und gegebenenfalls ein Sauerstoff- oder Schwefelatom als Ringglieder und ist mit einem seiner Ring-C-Atome mit der Gruppe -CO- bzw. -OCO-, >N-CO-, >N-CS-, $-SO_2-$ oder $>N-SO_2-$ verknüpft. Dieser Hetero-

cyclus kann an einem seiner C-Atome oder an einem Ringstickstoffatom durch Niederalkyl, z.B. Methyl oder Aethyl, Phenyl oder Phenylniederalkyl, z.B. Benzyl, oder an einem seiner C-Atome durch Hydroxy oder Oxo substituiert sein.

Ein solcher Heterocyclus ist beispielsweise Pyrrolidin-3-yl, Hydroxypyrrolidin-2- oder -3-yl, z.B. 4-Hydroxypyrrolidin-2-yl, Oxopyrrolidin-2-yl, z.B. 5-Oxopyrrolidin-2-yl, Piperidin-2- oder -3-yl, 1-Niederalkylpiperidin-2-, -3- oder -4-yl, z.B. 1-Methylpiperidin-2-, -3- oder -4-yl, Morpholin-2- oder -3-yl, Thiomorpholin-2- oder -3-yl- oder 1- und/oder 4-Niederalkylpiperazin-2- oder -3-yl, z.B. 1,4-Dimethylpiperazin-2-oder -3-yl. Ausgeschlossen als Heterocyclus $R^b$ in einem Acylrest $R^b$-CO- ist gegebenenfalls N-acyliertes Pyrrolidin-2-yl, d.h. der Rest der Aminosäure Prolin.

Bevorzugte Acylgruppen $R_1$ oder $R_4$ sind beispielsweise Alkanoyl, z.B. n-Decanoyl oder Niederalkanoyl, z.B. Formyl, Acetyl oder Pivaloyl, Niederalkoxyniederalkanoyl, z.B. Methoxyacetyl, Niederalkoxyniederalkoxyniederalkoxyalkanoyl, z.B. 2-(2-Methoxyäthoxy)-äthoxyacetyl, Halogenniederalkanoyl, z.B. 2-Halogenacetyl, wie 2-Chlor-, 2-Brom-, 2-Jod- oder 2,2,2-Trichloracetyl, Carboxyniederalkanoyl, z.B. Carboxyacetyl, Niederalkoxycarbonylniederalkanoyl, z.B. Methoxycarbonylacetyl, Diniederalkylaminoniederalkanoyl, z.B. Dimethylaminoacetyl, Niederalkenoyl, z.B. Acryloyl oder α-Carboxmethyl-ß,ß-dimethyl-acryloyl, Niederalkinoyl, z.B. Propiolyl, Cycloalkylcarbonyl, z.B. Cyclopropyl-, Cyclopentyl- oder Cyclohexylcarbonyl, Benzoyl oder durch Halogen, z.B. Chlor, Niederalkoxy, z.B. Methoxy, und/oder Nitro substituiertes Benzoyl, z.B. 4-Chlor-, 4-Methoxy-oder 4-Nitrobenzoyl, Pyrrolyl-2-carbonyl, 5-Oxopyrrolidinyl-2-carbonyl, Niederalkoxycarbonyl, z.B. Methoxycarbonyl oder tert-Butoxycarbonyl, 2-Halogenniederalkoxycarbonyl, z.B. 2-Chlor-, 2-Brom-, 2-Jod- oder 2,2,2-Trichloräthoxycarbonyl oder 1,1-Dimethyl-2,2,2-trichloräthoxycarbonyl, 1-Adamantyloxycarbonyl, Arylniederalkoxycarbonyl mit einem oder zwei Arylresten, worin Aryl gegebenenfalls z.B. durch Niederalkyl, wie Methyl oder tert-Butyl, Niederalkoxy, wie Methoxy,

Hydroxy, Halogen, wie Chlor und/oder Nitro mono-, di-oder trisubstituiertes Phenyl ist, z.B. Benzyloxycarbonyl, 4-Nitrobenzyloxycarbonyl, Diphenylmethoxycarbonyl oder Di-(4-methoxyphenyl)-methoxycarbonyl, ferner Niederalkylaminocarbonyl, z.B. tert-Butylaminocarbonyl.

Die Aminosäurereste $X_1$ und $X_2$ sind durch eine Peptidbindung miteinander verbunden und leiten sich von den natürlichen Aminosäuren ab, die normalerweise in Eiweissen vorkommen, und die beispielsweise in "Houben-Weyl, Methoden der Organischen Chemie", Band XV/1 (1974), "Synthese von Peptiden", Seite 2, aufgezählt sind. Diese Aminosäuren sind α-Aminosäuren mit der L-Konfiguration und umfassen insbesondere die 20 α-Aminosäuren, welche besonders häufig in Proteinen vorkommen, nämlich Glycin (H-Gly-OH), Alanin (H-Ala-OH), Prolin (H-Pro-OH), Serin (H-Ser-OH), Cystein (H-Cys-OH), Tyrosin (H-Tyr-OH), Asparagin (H-Asn-OH), Glutamin (H-Gln-OH), Asparaginsäure (H-Asp-OH), Glutaminsäure (H-Glu-OH), Arginin (H-Arg-OH), Histidin (H-His-OH), einschliesslich jene 8 Aminosäuren, die für den Menschen essentiell sind, nämlich Valin (H-Val-OH), Leucin (H-Leu-OH), Isoleucin (H-Ile-OH), Lysin (H-LysOH), Phenylalanin (H-Phe-OH), Tryptophan (H-Trp-OH), Methionin (H-Met-OH) und Threonin (H-Thr-OH), ferner trans-3- und trans-4-Hydroxyprolin, α-Aminobuttersäure, α,ß-Diaminopropionsäure, α,γ-Diaminobuttersäure, Ornithin, δ-Hydroxylysin und 4-Amino-3-hydroxy-6-methylheptansäure (Statin, hier abgekürzt "H-Sta-OH"). Wenn nicht anders angegeben, bezeichnen die genannten Abkürzungen die Aminosäuren in ihrer natürlichen, d.h. L-Konfiguration.

Die Aminosäurereste $X_1$ und $X_2$ können N-terminal zur Erhöhung der Stabilität der Verbindung der Formel I gegen enzymatischen Abbau durch Niederalkyl, z.B. Methyl oder Aethyl, substituiert sein.

Eine Carboxy-Funktion in der Seitenkette eines Aminosäurerestes $X_1$ und $X_2$, wie sie in Glutaminsäure und Asparaginsäure vorliegt, ist gewünschtenfalls mit einem Niederalkanol, z.B. mit Methanol oder

tert-Butanol verestert. Eine Amino-Funktion in der Seitenkette eines Aminosäurerestes $X_1$ und $X_2$, wie sie in Lysin, α,ß-Diaminopropionsäure, α,γ-Diaminobuttersäure, Ornithin und δ-Hydroxylysin vorliegt, ist gewünschtenfalls durch Niederalkoxycarbonyl. z.B. tert-Butoxycarbonyl, oder Arylniederalkoxycarbonyl, z.B. Benzyloxycarbonyl, acyliert.

$X_1$ ist vorzugsweise der bivalente Rest des Phenylalanins und $X_2$ vorzugsweise der bivalente Rest des Histidins, Glycins, Alanins oder Phenylalanins.

Niederalkyl $R_2$ oder $R_4$ hat die weiter vorn für Niederalkyl $R^a$ oder $R^b$ genannten Bedeutungen und ist vorzugsweise Methyl oder Aethyl.

Unsubstituiertes oder substituiertes Niederalkyl $R_3$ hat die weiter vorn für Niederalkyl $R^a$ oder $R^b$ genannten Bedeutungen und ist beispielsweise durch Hydroxy, veräthertes Hydroxy, z.B. Niederalkoxy, wie Methoxy, oder Arlyniederalkoxy, wie Benzyloxy, oder verestertes Hydroxy, z.B. Niederalkanoyloxy, wie Acetoxy, substituiert. Unsubstituiertes oder substituiertes Niederalkyl $R_3$ oder $R_5$ ist vorzugsweise Methyl, Isopropyl, Isobutyl, 2-Butyl, Hydroxymethyl, 2-Hydroxyäthyl, Benzyloxymethyl oder 2-Benzyloxyäthyl.

Unsubstituiertes oder substituiertes Arylniederalkyl $R_3$ oder $R_5$ hat die weiter vorn für Arylniederalkyl $R^a$ oder $R^b$ genannten Bedeutungen und ist im Niederalkylrest beispielsweise durch Hydroxy, veräthertes Hydroxy, z.B. Niederalkoxy, wie Methoxy, oder Arlyniederalkoxy, wie Benzyloxy, oder verestertes Hydroxy, z.B. Niederalkanoyloxy, wie Acetoxy, substituiert. Unsubstituiertes oder substituiertes Arylniederalkyl $R_3$ oder $R_5$ ist vorzugsweise Benzyl oder α-Hydroxybenzyl.

Aryl $R_3$ oder $R_5$ ist vorzugsweise Phenyl.

$R_6$ mit der Bedeutung "substituiertes Amino" oder "substituiertes Hydroxy" ist beispielsweise eine Amino- oder Hydroxygruppe, welche

durch einen oder gegebenenfalls zwei unsubstituierte oder substituierte, gesättigte oder ungesättigte, aliphatische Kohlenwasserstoffreste bis einschliesslich 18, bevorzugt bis einschliesslich 10, C-Atomen oder einen unsubstituierten oder substituierten, aromatischen, heteroaromatischen, aromatisch-aliphatischen oder heteroaromatisch-aliphatischen Kohlenwasserstoffrest mit bis zu 18, bevorzugt bis einschliesslich 10, C-Atomen substituiert ist.

$R_6$ mit der Bedeutung "substituiertes Amino" oder "substituiertes Hydroxy" kann ausserdem einen N-terminal mit der Gruppe -CO- verbundenen und C-terminal gegebenenfalls amidierten oder veresterten Rest einer natürlichen α-Aminosäure oder einen N-terminal mit der Gruppe -CO- verbundenen und C-terminal gegebenenfalls amidierten oder veresterten Di- oder Tripeptidrest, der aus natürlichen α-Aminosäuren und gewünschtenfalls aus Statin (abgekürzt H-Sta-OH, 4-Amino-3-hydroxy-6-methylheptansäure) besteht, darstellen.

Ein unsubstituierter oder substituierter, gesättigter oder ungesättigter, aliphatischer Kohlenwasserstoffrest, welcher die Aminogruppe oder Hydroxygruppe $R_6$ substituiert, ist beispielsweise Alkyl mit bis zu 10 C-Atomen, sowie Niederalkenyl oder Niederalkinyl mit bis einschliesslich 7 C-Atomen.

Diese Reste können wie Niederalkyl $R^a$ durch eine oder mehrere der weiter vorn genannten funktionellen Gruppen, sowie durch Sulfo, substituiertes Amino, worin die Aminogruppe Teil eines fünf- oder sechsgliedrigen Heterocyclus enthaltend ein bis zwei Stickstoffatome und gewünschtenfalls ein Sauerstoff- oder Schwefelatom ist, z.B. 1-Pyrrolidinyl, 1-Piperidinyl, 1-Pyridazinyl, 4-Morpholinyl oder 4-Thiomorpholinyl, oder durch acyliertes Amino, z.B. Niederalkoxycarbonylamino, wie tert-Butoxycarbonylamino, Arylniederalkoxycarbonylamino, wie Benzyloxycarbonylamino, oder Niederalkanoylamino, wie Acetylamino, substituiert sein.

Als Substituenten sind Hydroxy, Carboxy, verestertes Carboxy, z.B. Niederalkoxycarbonyl, wie tert-Butoxycarbonyl, oder ein physiologisch spaltbares verestertes Carboxy, z.B. 1-(Niederalkanoyloxy)-niederalkoxycarbonyl, wie Acetoxymethoxycarbonyl, Pivaloyloxymethoxycarbonyl oder 1-Propionyloxyäthoxycarbonyl, 1-(Niederalkoxycarbonyloxy)-niederalkoxycarbonyl, wie 1-(Aethoxycarbonyloxy)-äthoxycarbonyl, oder α-Aminoniederalkanoyloxymethoxycarbonyl, wie α-Aminoacetoxymethoxycarbonyl oder (S)-α-Amino-ß-methylbutyryloxymethoxycarbonyl, Amino, Diniederalkylamino, z.B. Dimethylamino, über ein Stickstoff-Atom gebundenes fünf- oder sechsgliedriges Heterocyclyl, z.B. 1-Piperidyl oder 4-Morpholinyl, und acyliertes Amino, z.B. Niederalkoxycarbonylamino, wie tert-Butoxycarbonylamino, bevorzugt.

Alkyl in einer Gruppe $R_6$ mit 1-10 C-Atomen ist z.B. n-Octyl, n-Nonyl, n-Decyl oder Niederalkyl mit den unter $R^a$ genannten Bedeutungen, z.B. Methyl, n-Pentyl, Neopentyl, n-Hexyl oder n-Heptyl.

Substituiertes Alkyl in einer Gruppe $R_6$ ist z.B. Hydroxyniederalkyl, z.B. 2-Hydroxyäthyl, Niederalkoxyniederalkyl, z.B. Niederalkoxymethyl oder Niederalkoxyäthyl, wie Methoxymethyl oder 2-Methoxyäthyl, Niederalkanoyloxyniederalkyl, z.B. Niederalkanoyloxymethyl oder Niederalkanoyloxyäthyl, wie Acetoxymethyl oder 2-Acetoxyäthyl, Halogenniederalkyl, z.B. Halogenmethyl oder Halogenäthyl, wie Trifluormethyl, Chlor- oder Brommethyl, 2-Chlor-, 2-Brom- oder 2,2,2-Trichloräthyl, Hydroxysulfonyloxyniederalkyl, z.B. Hydroxysulfonyloxymethyl oder 2-Hydroxysulfonyloxyäthyl, Carboxyalkyl, z.B. 4-Carboxy-n-butyl, 5-Carboxy-n-pentyl, 6-Carboxy-n-hexyl, 7-Carboxy-n-heptyl oder 8-Carboxy-n-octyl, ferner Carboxymethyl, 2-Carboxyäthyl, 3-Carboxy-n-propyl oder 2-Carboxyprop-2-yl, verestertes Carboxyalkyl, z.B. Niederalkoxycarbonylalkyl, wie 4-tert-Butoxycarbonyl-n-butyl, 7-tert-Butoxycarbonyl-n-heptyl oder 8-tert-Butoxycarbonyl-n-octyl, ferner tert-Butoxycarbonylmethyl oder 2-tert-Butoxycarbonyläthyl, oder physiologisch spaltbares verestertes Carboxyalkyl, z.B. 7-Pivaloyloxymethoxycarbonyl-n-heptyl, 7-(1-Aethoxycarbonyloxy-äthoxycarbonyl)-n-heptyl oder 4-Pivaloyloxymethoxycarbonyl-n-butyl, Carbamoylniederalkyl, z.B. Carbamoylmethyl oder 2-Carbamoyläthyl, Niederalkylcarbamoylniederalkyl, z.B. Methylcarbamoylmethyl, Diniederalkylcarbamoylniederalkyl, z.B. Dimethylcarbamoylmethyl, Sulfoniederalkyl, z.B. Sulfomethyl oder 2-Sulfoäthyl, Aminoalkyl, z.B. 4-Amino-n-butyl, 5-Amino-n-pentyl, 6-Amino-n-hexyl, 7-Amino-n-heptyl oder 8-Amino-n-octyl, ferner 2-Aminoäthyl oder 3-Amino-n-propyl, Niederalkylaminoniederalkyl, z.B. 2-Methylaminoäthyl oder 3-Methylamino-n-propyl, Diniederalkylaminoniederalkyl, z.B. 2-Dimethylaminoäthyl oder 3-Dimethylamino-n-propyl, über ein Stickstoffatom gebundenes fünf- oder sechsgliedriges Heterocyclylniederalkyl, z.B. 3-(1-Piperidinyl)-propyl, 3-(4-Morpholinyl)-propyl oder 2-(4-Morpholinyl)-äthyl, oder Acylaminoalkyl, z.B. Niederalkanoylaminoalkyl, z.B. Pivaloylaminoalkyl oder Acetylaminoalkyl, wie 4-Pivaloylamino-n-butyl oder 4-Acetylamino-n-butyl, oder Niederalkoxycarbonylaminoalkyl, z.B. tert-Butoxycarbonylaminoalkyl, wie 4-tert-Butoxycarbonylamino-n-butyl, 5-tert-Butoxycarbonylamino-n-pentyl, 6-tert-Butoxycarbonylamino-n-hexyl oder 7-tert-Butoxycarbonylamino-n-heptyl, ferner 2-tert-Butoxycarbonylaminoäthyl.

Niederalkenyl und Niederalkinyl in einer Gruppe $R_6$ sind wie die unter $R^a$ und $R^b$ genannten Reste definiert und können durch die gleichen Substituenten wie Niederalkyl $R^a$ substituiert sein.

Ein aromatischer oder aromatisch-aliphatischer Kohlenwasserstoffrest in einer Gruppe $R_6$ hat die gleichen wie unter $R^a$ oder $R^b$ genannten Bedeutungen und ist vorzugsweise Phenyl oder Benzyl. Diese Reste können im Aromaten z.B. durch Niederalkyl, wie Methyl oder Aethyl, Hydroxy, veräthertes Hydroxy, z.B. Niederalkoxy, wie Methoxy oder tert-Butoxy, verestertes Hydroxy, z.B. Niederalkanoyloxy, wie Acetoxy, oder Halogen, z.B. Fluor oder Chlor, Carboxy, verestertes Carboxy, z.B. Niederalkoxycarbonyl, wie Methoxycarbonyl oder

tert-Butoxycarbonyl, Carbamoyl, Amino, Niederalkylamino, z.B. Methylamino, Diniederalkylamino, z.B. Dimethylamino, acyliertes Amino, z.B. Niederalkoxycarbonylamino, wie tert-Butoxycarbonylamino, sowie durch Nitro substituiert sein.

Substituiertes Phenyl in einem Rest $R_6$ ist beispielsweise 2-, 3- oder 4-Niederalkylphenyl, z.B. 2-, 3- oder 4-Methylphenyl, 2-, 3- oder 4-Hydroxyphenyl, 2-, 3-oder 4-Niederalkoxyphenyl, z.B 2-, 3- oder 4-Methoxyphenyl, 2,3-, 2,4- oder 2,5-Dimethoxyphenyl oder 2-, 3- oder 4-tert-Butoxyphenyl, 2-, 3- oder 4-Halogenphenyl, z.B. 2-, 3- oder 4-Chlorphenyl, 2,3-, 3,4- oder 2,5-Dihalogenphenyl, z.B. 2,3-, 3,4- oder 2,5-Dichlorphenyl, 2-, 3- oder 4-Carboxyphenyl, 2-, 3- oder 4-Niederalkoxycarbonylphenyl, z.B. 2-, 3- oder 4-Methoxy- oder -tert-Butoxycarbonylphenyl, 2-, 3- oder 4-Carbamoylphenyl, 2-, 3- oder 4-Aminophenyl, 2-, 3- oder 4-Niederalkoxycarbonylaminophenyl, z.B. 2-, 3- oder 4-tert-Butoxycarbonylaminophenyl, oder 2-, 3- oder 4-Nitrophenyl.

Substituiertes Benzyl in einem Rest $R_6$ ist beispielsweise 2-, 3- oder 4-Carboxybenzyl, 2-, 3- oder 4-Niederalkoxycarbonylbenzyl, z.B. 2-, 3- oder 4-Methoxy- oder tert-Butoxycarbonylbenzyl, oder 2-, 3- oder 4-Carbamoylbenzyl.

Ein heteroaromatischer oder heteroaromatisch-aliphatischer Kohlenwasserstoffrest in einer Gruppe $R_6$ hat die gleichen wie unter $R^a$ und $R^b$ genannten Bedeutungen und ist vorzugsweise Pyridylniederalkyl, z.B. 2-(2-Pyridyl)-äthyl oder 3-(2-Pyridyl)-propyl, oder Imidazolylniederalkyl, z.B. 2-(4-Imidazolyl)-äthyl oder 3-(4-Imidazolyl)-propyl.

Die Amino- oder Hydroxygruppe $R_6$ kann auch durch einen gesättigten, fünf- oder sechsgliedrigen Heterocyclus substituiert sein, der die unter $R^a$ oder $R^b$ genannten Bedeutungen hat. Ein solcher Heterocyclus ist vorzugsweise ein 1-Benzylpiperidin-2-, -3- oder -4-ylrest.

Substituiertes Amino $R_6$ ist vorzugsweise Alkylamino, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino, Hydroxyniederalkylamino, z.B. 2-Hydroxyäthylamino, Niederalkoxyniederalkylamino, z.B. 2-Methoxyäthylamino, Carboxyalkylamino, z.B. 4-Carboxy-n-butylamino, 5-Carboxy-n-pentylamino, 6-Carboxy-n-hexylamino, 7-Carboxy-n-heptylamino oder 8-Carboxy-n-octylamino, Niederalkoxycarbonylalkylamino, z.B. 4-tert-Butoxycarbonyl-n-butylamino, 7-tert-Butoxycarbonyl-n-heptylamino oder 8-tert-Butoxycarbonyl-n-octylamino, physiologisch spaltbares verestertes Carboxyalkylamino, z.B. 7-Pivaloyloxymethoxycarbonyl-n-heptylamino, 7-(1-Aethoxycarbonyloxy-äthoxycarbonyl)-n-heptylamino oder 4-Pivaloyloxymethoxycarbonyl-n-butylamino, Aminoalkylamino, z.B. 4-Amino-n-butylamino, 5-Amino-n-pentylamino, 6-Amino-n-hexylamino, 7-Amino-n-heptylamino oder 8-Amino-n-octylamino, Diniederalkylaminoniederalkylamino, z.B. 2-Dimethylaminoäthylamino, Acylaminoniederalkylamino, z.B. Niederalkanoylaminoniederalkylamino, wie 4-Pivaloylamino-n-butylamino, oder Niederalkoxycarbonylamino-niederalkylamino, wie 4-tert-Butoxycarbonylamino-n-butylamino, 5-tert-Butoxycarbonylamino-n-pentylamino, 6-tert-Butoxycarbonylamino-n-hexylamino oder 7-tert-Butoxycarbonylamino-n-octylamino, Carboxybenzylamino, z.B. 3-Carboxybenzylamino, Niederalkoxycarbonylbenzylamino, z.B. 3-tert-Butoxycarbonylbenzylamino, sowie Carbamoylbenzylamino, z.B. 3-Carbamoylbenzylamino.

Substituiertes Hydroxy $R_6$ ist vorzugsweise Niederalkoxy, z.B. Methoxy, Aethoxy, n-Propoxy, Isopropoxy, Butoxy, Isobutoxy, tert-Butoxy oder tert-Pentoxy, oder physiologisch spaltbares Alkoxy, z.B. Pivaloyloxymethoxy.

Ein N-terminal mit der -CO-Gruppe verbundener und C-terminal gegebenenfalls amidierter oder veresterter natürlicher Aminosäurerest $R_6$ besteht vorzugsweise aus einer der 20 weiter vorn genannten α-Aminosäuren, die besonders häufig in Proteinen vorkommen, z.B. -His-,

-Phe-, -Ala- und besonders -Ile und ist gegebenenfalls C-terminal durch Amino, substituiertes Amino oder substituiertes Hydroxy substituiert. Solches substituiertes Amino oder substituiertes Hydroxy hat die weiter vorn für $R_6$ genannten Bedeutungen und ist beispielsweise Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino, Carboxyalkylamino, z.B. 4-Carboxy-n-butylamino oder 2-Carboxy-prop-2-ylamino, Niederalkoxycarbonylalkylamino, z.B. 4-tert-Butoxycarbonyl-n-butylamino oder 7-tert-Butoxycarbonyl-n-heptylamino, physiologisch spaltbares, verestertes Carboxyalkylamino, z.B. 4-Pivaloyloxymethoxycarbonyl-n-butylamino, Aminoalkylamino, z.B. 4-Amino-n-butylamino, 5-Amino-n-pentylamino oder 8-Amino-n-octylamino, Diniederalkylaminoniederalkylamino, z.B. 2-Dimethylaminoäthylamino oder 3-Dimethylamino-n-propylamino, über ein Stickstoff-Atom gebundenes gesättigtes, fünf- oder sechsgliedriges Heterocyclylniederalkylamino, z.B. Morpholin-4-ylniederalkylamino, wie 3-(4-Morpholinyl)-propylamino oder 2-(4-Morpholinyl)-äthylamino, Acylaminoniederalkylamino, z.B. Niederalkoxycarbonylaminoniederalkylamino, wie 4-tert-Butoxycarbonylamino-n-butylamino, Phenylniederalkylamino, z.B. Benzylamino, Carboxybenzylamino, z.B. 3-Methoxy- oder 3-tert-Butoxycarbonylbenzylamino, Carbamoylbenzylamino, z.B. 3-Carbamoylbenzylamino, Pyridylniederalkylamino, z.B. 2-(2-, 3- oder 4-Pyridyl)-äthylamino, Imidazolylniederalkylamino, z.B. 2-(4-Imidazolyl)-äthylamino, Piperidinylamino, z.B. 1-Benzylpiperidin-2-, -3- oder -4-ylamino, ferner Niederalkoxy, z.B. Methoxy, Aethoxy oder tert-Butoxy, oder physiologisch spaltbares substituiertes Alkoxy, z.B. Pivaloyloxymethoxy.

In einem N-terminal mit der -CO-Gruppe verundenen und C-terminal gegebenenfalls amidierten oder veresterten Di- oder Tripeptidrest $R_6$ sind die Aminosäurebestandteile vorzugsweise von den 20 weiter vorn genannten α-Aminosäuren, die besonders häufig in Proteinen

vorkommen, sowie vom Statin abgeleitet. Ein solcher Di- oder Tripeptidrest ist beispielsweise -Ile-His-, Ile-Phe-, -Ala-His-, -Gly-Gly-, -Gly-His-, -Gly-Ala-, -Ala-Sta-, -Ile-Sta-, -Gly-Gly-Gly-, -Gly-His-Lys-, -Gly-Ala-Gly- oder Ile-His-Lys.

Die Aminosäurereste können N-terminal zur Erhöhung der Stabilität der Verbindung der Formel I gegen enzymatischen Abbau durch Niederalkyl, z.B. Methyl oder Aethyl, substituiert sein. Die Carboxy-Funktion in der Seitenkette von -Glu- und -Asp- ist gewünschtenfalls mit einem Niederalkanol, z.B. mit Methanol oder tert-Butanol verestert. Die Amino-Funktion in der Seitenkette von -Lys- ist gewünschtenfalls durch Niederalkanoyl, z.B. Pivaloyl, durch Niederalkoxycarbonyl, z.B. tert-Butoxycarbonyl oder durch Arylniederalkoxycarbonyl, z.B. Benzyloxycarbonyl, acyliert.

Ein Di- oder Tripeptidrest $R_6$ ist C-terminal gegebenenfalls durch Amino, substituiertes Amino oder substituiertes Hydroxy substituiert. Solches substituiertes Amino oder substituiertes Hydroxy hat die weiter vorn für $R_6$ genannten Bedeutungen und ist beispielsweise Alkylamino, z.B. Methyl-, Aethyl-, n-Propyl-, n-Butyl- oder n-Hexylamino, Diniederalkylamino, z.B. Dimethylamino, Hydroxyniederalkylamino, z.B. 2-Hydroxyäthylamino, Aminoalkylamino, z.B. 4-Amino-n-butylamino oder 6-Amino-n-hexylamino, Acylaminoalkylamino, z.B. Niederalkoxycarbonylaminoalkylamino, wie 6-tert-Butoxycarbonylamino-n-hexylamino, oder Arylniederalkoxycarbonyl-aminoalkylamino, wie 6-Benzyloxycarbonylamino-n-hexylamino, ferner Niederalkoxy, z.B. Methoxy, Aethoxy oder tert-Butoxy, oder physiologisch spaltbares substituiertes Alkoxy, z.B. Pivaloyloxymethoxy.

Salze sind in erster Linie die pharmazeutisch verwendbaren oder nicht-toxischen Salze von Verbindungen der Formel I.

Solche Salze werden beispielsweise von Verbindungen der Formel I mit einer sauren Gruppe, z.B. einer Carboxygruppe, gebildet und sind in erster Linie geeignete Alkalimetall-, wie Natrium- oder Kalium-,

oder Erdalkalimetallsalze, z.B. Magnesium- oder Calciumsalze, ferner Zinksalze, oder Ammoniumsalze, inkl. solche Salze, welche mit organischen Aminen, wie gegebenenfalls durch Hydroxy substituierten Mono-, Di- oder Trialkylaminen gebildet werden, z.B. Diäthylamin, Di-(2-hydroxyäthyl)-amin, Triäthylamin, N,N-Dimethyl-N-(2-hydroxyäthyl)-amin, Tri-(2-hydroxy-äthyl)-amin oder N-Methyl-D-glucamin. Die Verbindungen der Formel I mit einer basischen Gruppe, z.B. einer Aminogruppe, können Säureadditionssalze bilden, z.B. mit anorganischen Säuren, z.B. Salzsäure, Schwefelsäure oder Phosphorsäure, oder mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, wie z.B. den weiter vorn genannten α-Aminosäuren, sowie Methansulfonsäure, Aethansulfonsäure, 2-Hydroxyäthansulfonsäure, Aethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Verbindungen der vorliegenden Erfindung weisen Enzym-hemmende Wirkungen auf; insbesondere hemmen sie die Wirkung des natürlichen Enzyms Renin. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Letzteres erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist.

Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I. Als Folge davon entsteht eine geringere Menge Angiotensin II. Die verminderte Konzentration dieses aktiven Peptidhormons ist die unmittelbare Ursache für die blutdrucksenkende Wirkung von Renin-Hemmern.

Die Wirkung von Renin-Hemmern wird unter anderem experimentell mittels in vitro-Tests nachgewiesen, wobei die Verminderung der Bildung von Angiotensin I in verschiedenen Systemen (Humanplasma, gereinigtes humanes Renin zusammen mit synthetischem oder natürlichem Reninsubstrat) gemessen wird. Unter anderem wird der folgende in vitro Test verwendet: Ein Extrakt von menschlichem Renin aus der Niere (0,5 mGU [Milli-Goldblatt-Einheiten]/ml) wird eine Stunde lang bei 37°C und pH 7,2 in 1-molarer wässriger 2-N-(Tris-hydroxymethyl-methyl)-amino-äthansulfonsäure-Pufferlösung mit 23 µg/ml synthetischem Renin-Substrat, dem Tetradecapeptid H-Asp-Arg-Val-Tyr-Ile-His-Pro-Phe-His-Leu-Leu-Val-Tyr-Ser-OH, inkubiert. Die Menge des gebildeten Angiotensins I wird in einem Radioimmunoassay ermittelt. Die erfindungsgemässen Hemmstoffe werden dem Inkubationsgemisch jeweils in verschiedenen Konzentrationen zugefügt. Als $IC_{50}$ wird diejenige Konzentration des jeweiligen Hemmstoffes bezeichnet, die die Bildung von Angiotensin I um 50% reduziert. Die Verbindungen der vorliegenden Erfindung zeigen in den in vitro-Systemen Hemmwirkungen bei minimalen Konzentrationen von etwa $10^{-5}$ bis etwa $10^{-8}$ Mol/l.

An salzverarmten Tieren bewirken Renin-Hemmer einen Blutdruckabfall. Das menschliche Renin unterscheidet sich von Renin anderer Spezies. Zur Prüfung von Hemmern des humanen Renins werden Primaten (Marmosets, Callithrix jacchus) verwendet, weil humanes Renin und Primaten-Renin im enzymatisch aktiven Bereich weitgehend homolog sind. Unter anderem wird der folgende in vivo Test benutzt: Die Testverbindungen werden an normotensiven Marmosets beider Geschlechter mit einem Körpergewicht von etwa 300 g, die bei Bewusstsein sind,

geprüft. Blutdruck und Herzfrequenz werden mit einem Katheter in der Oberschenkelarterie gemessen. Die endogene Freisetzung von Renin wird durch die intravenöse Injektion von Furosemid (5 mg/kg) angeregt. 30 Minuten nach der Injektion von Furosemid werden die Testsubstanzen über einen Katheter in der lateralen Schwanzvene entweder durch einmalige Injektion oder durch kontinuierliche Infusion verabreicht und ihre Wirkung auf Blutdruck und Herzfrequenz ausgewertet. Die Verbindungen der vorliegenden Erfindung sind in dem beschriebenen in vivo Test bei Dosen von etwa 0,1 bis etwa 1,0 mg/kg i.v. wirksam.

Die Verbindungen der vorliegenden Erfindung können als Antihypertensiva, ferner zur Behandlung von Herzinsuffizienz verwendet werden.

Die Erfindung betrifft insbesondere Verbindungen der Formel I, worin $R_1$ und $R_4$ unabhängig voneinander Wasserstoff oder Acyl mit den Teilformeln $R^b$-CO-, $R^a$-O-CO- oder $(R^b)(R^b)$N-CO-, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18, bevorzugt bis einschliesslich 10, C-Atomen oder einen unsubstituierten oder substituierten, aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen oder einen unsubstituierten oder substituierten, gesättigten, fünf- oder sechsgliedrigen Heterocyclus darstellt und $R^b$ Wasserstoff bedeutet oder die Bedeutungen von $R^a$ hat, $X_1$ einen natürlichen Aminosäurerest, der N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2$-verbunden ist, $R_2$ Wasserstoff, $R_3$ und $R_5$ unabhängig voneinander Wasserstoff, unsubstituiertes oder substituiertes Niederalkyl, unsubstituiertes oder substituiertes Arylniederalkyl oder Aryl und $R_6$ eine Amino-oder Hydroxygruppe, welche durch einen oder gegebenenfalls zwei unsubstituierte oder substituierte, gesättigte oder

ungesättigte, aliphatische Kohlenwasserstoffreste bis einschliesslich 18, bevorzugt bis einschliesslich 10, C-Atomen oder einen aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen substituiert ist, oder einen N-terminal mit der Gruppe -CO- verbundenen und C-terminal gegebenenfalls amidierten oder veresterten Rest einer natürlichen α-Aminosäure oder einen N-terminal mit der Gruppe -CO- verbundenen und C-terminal gegebenenfalls amidierten oder veresterten Di- oder Tripeptidrest, der aus natürlichen α-Aminosäuren und gewünschtenfalls aus Statin besteht, darstellen, mit Ausnahme der Verbindungen, worin $R_1$ einen gegebenenfalls N-acylierten Rest der Aminosäure L-Prolin oder worin $X_1$ und $X_2$ zusammen -Arg-Pro-, -Tyr-Gly- oder -Gly-Gly- bedeuten, ferner pharmazeutisch annehmbare Salze von Verbindungen der Formel I mit salzbildenden Gruppen.

Die Erfindung betrifft ebenfalls Verbindungen der Formel I, worin $R_1$ und $R_4$ unabhängig voneinander Wasserstoff oder Acyl mit den Teilformeln $R^b$-CO-, $R^a$-O-CO-, $(R^b)(R^b)$N-CO-, $(R^b)(R^b)$-N-CS-, $R^b$-$SO_2$-oder $(R^b)(R^b)$N-$SO_2$-, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18, bevorzugt bis einschliesslich 10, C-Atomen oder einen unsubstituierten oder substituierten, aromatischen oder aromatischh-aliphatischen Kohlenwasserstoffrest bis einschliesslich 18, bevorzugt bis einschliesslich 10, C-Atomen oder einen unsubstituierten oder substituierten, fünf- oder sechsgliedrigen Heterocyclus darstellt und $R^b$ Wasserstoff bedeutet oder die Bedeutungen von $R^a$ hat, $X_1$ einen natürlichen Aminosäurerest, der N-terminal mit $R_1$ und C-terminal mit $X_2$ verbunden ist, $X_2$ einen natürlichen Aminosäurerest, der N-terminal mit $X_1$ und C-terminal mit der Gruppe -$NR_2$-verbunden ist, $R_2$ Wasserstoff, $R_3$ und $R_5$ unabhängig voneinander Wasserstoff, Niederalkyl, Arylniederalkyl oder Aryl und $R_6$ eine Amino- oder Hydroxygruppe, welche durch einen unsubstituierten oder substituierten, gesättigten oder ungesättigten, aliphatischen

Kohlenwasserstoffrest bis einschliesslich 18, bevorzugt bis einschliesslich 10, C-Atomen oder einen aromatischen, aromatisch-aliphatischen Kohlenwasserstoffrest bis einschliesslich 18, bevorzugt bis einschliesslich 10, C-Atomen substituiert ist, oder einen N-terminal mit der Gruppe -CO- verbundenen und C-terminal gegebenenfalls amidierten oder veresterten Rest einer Aminosäure oder einen N-terminal mit der Gruppe -CO- verbundenen und C-terminal gegebenenfalls amidierten oder veresterten Di-oder Tripeptidrest, darstellen, mit Ausnahme der Verbindungen, worin $R_1$ einen gegebenenfalls N-acylierten Rest der Aminosäure L-Prolin oder worin $X_1$ und $X_2$ zusammen -Arg-Pro-, -Tyr-Gly- oder -Gly-Gly- bedeuten, ferner pharmazeutisch annehmbare Salze von Verbindungen der Formel I mit salzbildenden Gruppen.

Die Erfindung betrifft hauptsächlich Verbindungen der Formel I, worin $R_1$ Wasserstoff, Alkanoyl, z.B. n-Decanoyl oder Niederalkanoyl, z.B. Formyl, Acetyl oder Pivaloyl, Niederalkoxyniederalkanoyl, z.B. Methoxyacetyl, Niederalkoxyniederalkoxyniederalkoxyniederalkanoyl, z.B. 2-(2-Methoxyäthoxy)-äthoxyacetyl, Halogenniederalkanoyl, z.B. 2-Halogenacetyl, wie 2-Chlor-, 2-Brom-, 2-Jod- oder 2,2,2-Trichloracetyl, Carboxyniederalkanoyl, z.B. Carboxyacetyl, Niederalkoxycarbonylniederalkanoyl, z.B. Methoxycarbonylacetyl, Diniederalkylaminoniederalkanoyl, z.B. Dimethylaminoacetyl, Niederalkenoyl, z.B. Acryloyl oder α-Carboxymethyl-β,β-dimethyl-acryloyl, Niederalkinoyl, z.B. Propiolyl, Cycloalkylcarbonyl, z.B. Cyclopropyl-, Cyclopentyl- oder Cyclohexylcarbonyl, Benzoyl oder durch Halogen, z.B. Chlor, Niederalkoxy, z.B. Methoxy, und/oder Nitro substituiertes Benzoyl, z.B. 4-Chlor-, 4-Methoxy- oder 4-Nitrobenzoyl, Pyrrolyl-2-carbonyl, 5-Oxopyrrolidinyl-2-carbonyl, Niederalkoxycarbonyl, z.B. Methoxycarbonyl oder tert-Butoxycarbonyl, 2-Halogenniederalkoxycarbonyl, z.B. 2-Chlor-, 2-Brom-, 2-Jod- oder 2,2,2-Trichloräthoxycarbonyl oder 1,1-Dimethyl-2,2,2-trichloräthoxycarbonyl, 1-Adamantyloxycarbonyl, Arylniederalkoxycarbonyl mit einem oder zwei Arylresten, worin Aryl gegebenenfalls z.B. durch Niederalkyl, wie Methyl oder

tert-Butyl, Niederalkoxy, wie Methoxy, Hydroxy, Halogen, wie Chlor und/oder Nitro mono-, di- oder trisubstituiertes Phenyl ist, z.B. Benzyloxycarbonyl, 4-Nitrobenzyloxycarbonyl, Diphenylmethoxycarbonyl oder Di-(4-methoxyphenyl)-methoxycarbonyl, ferner Niederalkylaminocarbonyl, z.B. tert-Butylaminocarbonyl,

$X_1$ Phenylalanin, welches N-terminal mit $R_1$ verbunden ist, $X_2$ Histidin, Glycin, Alanin oder Phenylalanin, welche am N-Atom durch Niederalkyl substituiert sein können und N-terminal mit $X_1$ und C-terminal mit der Gruppe $-NR_2-$verbunden sind, $R_2$ Wasserstoff oder Niederalkyl, z.B. Methyl, $R_3$ und $R_5$ unabhängig voneinander Niederalkyl, z.B. Methyl, Isopropyl, Isobutyl oder 2-Butyl, Hydroxyniederalkyl, z.B. Hydroxymethyl oder 2-Hydroxyäthyl, veräthertes Hydroxyniederalkyl, z.B. Niederalkoxyniederalkyl, wie Methoxymethyl, oder Arylniederalkoxyniederalkyl, wie Benzyloxymethyl oder 2-Benzyloxyäthyl, verestertes Hydroxyniederalkyl, z.B. Niederalkanoyloxyniederalkyl, wie Acetoxymethyl, Arylniederalkyl, z.B. Benzyl, Aryl-hydroxy-niederalkyl, z.B. α-Hydroxybenzyl, oder Phenyl, $R_4$ Wasserstoff und

$R_6$ Alkylamino, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino, Hydroxyniederalkylamino, z.B. 2-Hydroxyäthylamino, Niederalkoxyniederalkylamino, z.B. 2-Methoxyäthylamino, Carboxyalkylamino, z.B. 4-Carboxy-n-butylamino, 5-Carboxy-n-pentylamino, 6-Carboxy-n-hexylamino, 7-Carboxy-n-heptylamino oder 8-Carboxy-n-octylamino, Niederalkoxycarbonylalkylamino, z.B. 4-tert-Butoxycarbonyl-n-butylamino, 7-tert-Butoxycarbonyl-n-heptylamino oder 8-tert-Butoxycarbonyl-n-octylamino, physiologisch spaltbares verestertes Carboxyalkylamino, z.B. 7-Pivaloyloxymethoxycarbonyl-n-heptylamino, 7-(1-Aethoxycarbonyloxy-äthoxycarbonyl)-n-heptylamino oder 4-Pivaloyloxymethoxycarbonyl-n-butylamino, Aminoalkylamino, z.B. 4-Amino-n-butylamino, 5-Amino-n-pentylamino, 6-Amino-n-hexylamino, 7-Amino-n-heptylamino oder 8-Amino-n-octylamino, Diniederalkylaminoniederalkylamino, z.B. 2-Dimethylaminoäthylamino, Acylaminoniederalkylamino, z.B. Niederalkanoylaminoniederalkylamino, wie 4-Pivaloylamino-n-butylamino, oder Niederalkoxycarbonylamino-niederalkylamino, wie 4-tert-Butoxycarbonylamino-n-butylamino, 5-tert-Butoxycarbonylamino-n-pentylamino, 6-tert-Butoxycarbonylamino-n-hexylamino oder 8-tert-Butoxycarbonylamino-n-octylamino, Carboxybenzylamino, z.B. 3-Carboxybenzylamino, Niederalkoxycarbonylbenzylamino, z.B. 3-tert-Butoxycarbonylbenzylamino, sowie Carbamoylbenzylamino, z.B. 3-Carbamoylbenzylamino, Niederalkoxy, z.B. Methoxy, Aethoxy, n-Propoxy, Isopropoxy, Butoxy, Isobutoxy, tert-Butoxy oder tert-Pentoxy, oder physiologisch spaltbares Alkoxy, z.B. Pivaloyloxymethoxy, ferner Histidin, Phenylalanin, Alanin oder Isoleucin, C-terminal gewünschtenfalls substituiert durch Amino, Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino, Carboxyalkylamino, z.B. 4-Carboxy-n-butylamino oder 2-Carboxy-prop-2-ylamino, Niederalkoxycarbonylalkylamino, z.B. 4-tert-Butoxycarbonyl-n-butylamino oder 7-tert-Butoxycarbonyl-n-heptylamino, physiologisch spaltbares, verestertes Carboxyalkylamino, z.B. 4-Pivaloyloxymethoxycarbonyl-n-butylamino, Aminoalkylamino, z.B. 4-Amino-n-butylamino, 5-Amino-n-pentylamino oder 8-Amino-n-octylamino, Diniederalkylaminoniederalkylamino, z.B. 2-Dimethylaminoäthylamino oder 3-Dimethylamino-n-propylamino, über ein Stickstoff-Atom gebundenes gesättigtes, fünf- oder sechsgliedriges Heterocyclylniederalkylamino, z.B. Morpholin-4-ylniederalkylamino, wie 3-(4-Morpholinyl)-propylamino oder 2-(4-Morpholinyl)-äthylamino, Acylaminoniederalkylamino, z.B. Niederalkoxycarbonylaminoniederalkylamino, wie 4-tert-Butoxycarbonylamino-n-butylamino, Phenylniederalkylamino, z.B. Benzylamino, Carboxybenzylamino, z.B. 3-Methoxy-oder 3-tert-Butoxycarbonylbenzylamino, Carbamoylbenzylamino, z.B. 3-Carbamoylbenzylamino, Pyridylniederalkylamino, z.B. 2-(2-, 3- oder 4-Pyridyl)-äthylamino, Imidazolylniederalkylamino, z.B. 2-(4-Imidazolyl)-äthylamino, Piperidinylamino, z.B. 1-Benzylpiperidin-2-, -3- oder -4-ylamino, ferner Niederalkoxy, z.B.

Methoxy, Aethoxy oder tert-Butoxy, oder physiologisch spaltbares substituiertes Alkoxy, z.B. Pivaloyloxymethoxy, ferner -Ile-His-, Ile-Phe-, -Ala-His-, -Gly-Gly-, -Gly-His-, -Gly-Ala-, -Ala-Sta-, -Ile-Sta-, -Gly-Gly-Gly-, -Gly-His-Lys-, -Gly-Ala-Gly- oder Ile-His-Lys, worin die Aminosäurereste N-terminal durch Niederalkyl, z.B. Methyl oder Aethyl, substituiert sein können und worin die Aminofunktion in der Seitenkette von -Lys- gewünschtenfalls durch Niederalkanoyl, z.B. Pivaloyl, durch Niederalkoxycarbonyl, z.B. tert-Butoxycarbonyl, oder durch Arylniederalkoxycarbonyl, z.B. Benzyloxycarbonyl, acyliert ist, C-terminal gewünschtenfalls substituiert durch Amino, Alkylamino, z.B. Methyl-, Aethyl-, n-Propyl-, n-Butyl- oder n-Hexylamino, Diniederalkylamino, z.B. Dimethylamino, Hydroxyniederalkylamino, z.B. 2-Hydroxyäthylamino, Aminoalkylamino, z.B. 4-Amino-n-butylamino oder 6-Amino-n-hexylamino, Acylaminoalkylamino, z.B. Niederalkoxycarbonylaminoalkylamino, wie 6-tert-Butoxycarbonylamino-n-hexylamino, oder Arylniederalkoxycarbonyl-aminoalkylamino, wie 6-Benzyloxycarbonylamino-n-hexylamino, ferner Niederalkoxy, z.B. Methoxy, Aethoxy oder tert-Butoxy, oder physiologisch spaltbares substituiertes Alkoxy, z.B. Pivaloyloxymethoxy,

darstellt, sowie pharmazeutisch annehmbare Salze von diesen Verbindungen mit salzbildenden Gruppen.

Die Erfindung betrifft bevorzugt Verbindungen der Formel I, worin $R_1$ Wasserstoff, Niederalkanoyl, z.B. Formyl, Acetyl oder Pivaloyl, 2-(2-Methoxyäthoxy)-äthoxyacetyl, Carboxyniederalkanoyl, z.B. Carboxyacetyl, Niederalkoxycarbonylniederalkanoyl, z.B. Methoxycarbonylacetyl, Diniederalkylaminoniederalkanoyl, z.B. Dimethylaminoacetyl, α-Carboxymethyl-ß,ß-dimethyl-acryloyl, Cycloalkylcarbonyl, z.B. Cyclopropyl-, Cyclopentyl- oder Cyclohexylcarbonyl, Benzoyl, Pyrrolyl-2-carbonyl, 5-Oxopyrrolidinyl-2-carbonyl, Niederalkoxycarbonyl, z.B. Methoxycarbonyl oder tert-Butoxycarbonyl, 2-Halogenniederalkoxycarbonyl, z.B. 2-Chlor-, 2-Brom-, 2-Jod- oder 2,2,2-Trichloräthoxycarbonyl oder 1,1-Dimethyl-2,2,2-trichloräthoxycarbonyl, 1-Adamantyloxycarbonyl, Arylniederalkoxycarbonyl, z.B. Benzyloxycarbonyl, oder Niederalkylaminocarbonyl, z.B. tert-Butylaminocarbonyl,

$X_1$ und $X_2$ zusammen -Phe-His-, -Phe-(N-methyl-His)- oder Phe-Gly, welche N-terminal mit $R_1$ und C-terminal mit der Gruppe $-NR_2-$ verbunden sind, $R_2$ Wasserstoff oder Niederalkyl, z.B. Methyl, $R_3$ Isobutyl, Benzyl oder α-Hydroxybenzyl, $R_4$ Wasserstoff, $R_5$ Methyl, Isopropyl, Hydroxymethyl, 2-Hydroxyäthyl, 2-Benzyloxyäthyl, Benzyl oder Phenyl, und

$R_6$ Alkylamino, z.B. Methyl-, Aethyl-, n-Butyl- oder n-Hexylamino, Hydroxyniederalkylamino, z.B. 2-Hydroxyäthylamino, Carboxyalkylamino, z.B. 4-Carboxy-n-butylamino, 5-Carboxy-n-pentylamino, 6-Carboxy-n-hexylamino, 7-Carboxy-n-heptylamino oder 8-Carboxy-n-octylamino, Niederalkoxycarbonylalkylamino, z.B. 4-tert-Butoxycarbonyl-n-butylamino, 7-tert-Butoxycarbonyl-n-heptylamino oder 8-tert-Butoxycarbonyl-n-octylamino, Carboxybenzylamino, z.B. 3-Carboxybenzylamino, Niederalkoxycarbonylbenzylamino, z.B. 3-tert-Butoxycarbonylbenzylamino, ferner Isoleucin, C-terminal substituiert durch Amino, Alkylamino, z.B. Methyl-, Aethyl-, n-Butyl- oder n-Hexylamino, Hydroxyniederalkylamino, z.B. 2-Hydroxyäthylamino, Aminoalkylamino, z.B. 4-Amino-n-butylamino, Diniederalkylaminoniederalkylamino, z.B. 2-Dimethylaminoäthylamino oder 3-Dimethylamino-n-propylamino, Morpholin-4-yl-niederalkylamino, z.B. 3-(4-Morpholinyl)-propylamino oder 2-(4-Morpholinyl)-äthylamino, Benzylamino, Carboxybenzylamino, z.B. 3-Carboxybenzylamino, Niederalkoxycarbonylbenzylamino, z.B. 3-Methoxy- oder 3-tert-Butoxycarbonylbenzylamino, Carbamoylbenzylamino, z.B. 3-Carbamoylbenzylamino, Imidazolylniederalkylamino, z.B. 2-(4-Imidazolyl)-äthylamino, Piperidinylamino, z.B. 1-Benzylpiperidin-2-, -3- oder -4-ylamino, ferner -Ile-His-, -Ile-Phe-, -Gly-Gly-, -Gly-(N-methyl-Gly)-, -Ala-Sta-, -Ile-Sta-,

-Gly-Gly-Gly- oder -Gly-His-Lys-, worin die Amino-Funktion in der Seitenkette von -Lys- gewünschtenfalls durch tert-Butoxycarbonyl oder Benzyloxycarbonyl acyliert ist, C-terminal substituiert durch Amino, Alkylamino, z.B. Methyl-, Aethyl-, n-Butyl oder n-Hexylamino, Hydroxyniederalkylamino, z.B. 2-Hydroxyäthylamino, Aminoalkylamino, z.B. 6-Amino-n-hexylamino, tert-Butoxycarbonylaminoalkylamino, z.B. 6-tert-Butoxycarbonylamino-n-hexylamino, Benzyloxycarbonylamino-alkylamino, z.B. 6-Benzyloxycarbonylamino-n-hexylamino, oder Niederalkoxy, z.B. Methoxy, Aethoxy oder tert-Butoxy,

darstellt, sowie pharmazeutisch annehmbare Salze von diesen Verbindungen mit salzbildenden Gruppen.

Die Erfindung betrifft vor allem Verbindungen der Formel I, worin $R_1$ Wasserstoff, Niederalkanoyl, z.B. Formyl, Acetyl oder Pivaloyl, 2-(2-Methoxyäthoxy)-äthoxyacetyl, Diniederalkylaminoniederalkanoyl, z.B. Dimethylaminoacetyl, α-Carboxymethyl-ß,ß-dimethyl-acryloyl, Cycloalkylcarbonyl, z.B. Cyclopropyl-, Cyclopentyl- oder Cyclohexylcarbonyl, Benzoyl, Pyrrolyl-2-carbonyl, 5-Oxopyrrolidinyl-2-carbonyl, Niederalkoxycarbonyl, z.B. Methoxycarbonyl oder tert-Butoxycarbonyl, 1,1-Dimethyl-2,2,2-trichloräthoxycarbonyl, 1-Adamantyloxycarbonyl, Benzyloxycarbonyl, oder Niederalkylaminocarbonyl, z.B. tert-Butylaminocarbonyl, $X_1$ und $X_2$ zusammen -Phe-His- oder -Phe-(N-methyl-His)-, welche N-terminal mit $R_1$ und C-terminal mit der Gruppe $-NR_2-$ verbunden sind, $R_2$ Wasserstoff oder Niederalkyl, z.B. Methyl, $R_3$ Isobutyl, Benzyl oder α-Hydroxybenzyl, $R_4$ Wasserstoff, $R_5$ Methyl, Isopropyl, Hydroxymethyl, 2-Hydroxyäthyl, 2-Benzyloxyäthyl, Benzyl oder Phenyl, und $R_6$ Carboxyalkylamino, z.B. 4-Carboxy-n-butylamino, 5-Carboxy-n-pentylamino, 6-Carboxy-n-hexylamino, 7-Carboxy-n-heptylamino oder 8-Carboxy-n-octylamino, Niederalkoxycarbonylalkylamino, z.B. 4-tert-Butoxycarbonyl-n-butylamino, 7-tert-Butoxycarbonyl-n-heptylamino oder 8-tert-Butoxycarbonyl-n-octylamino, Carboxybenzylamino, z.B. 3-Carboxybenzylamino, Niederalkoxycarbonylbenzylamino, z.B. 3-tert-Butoxycarbonylbenzylamino, ferner Isoleucin, C-terminal substituiert durch Amino, Alkylamino,

z.B. Methyl-, Aethyl-, n-Butyl- oder n-Hexylamino, Hydroxyniederalkylamino, z.B. 2-Hydroxyäthylamino, Diniederalkylaminoniederalkylamino, z.B. 2-Dimethylaminoäthylamino oder 3-Dimethylamino-n-propylamino, Morpholin-4-yl-niederalkylamino, z.B. 3-(4-Morpholinyl)-propylamino oder 2-(4-Morpholinyl)-äthylamino, Carbamoylbenzylamino, z.B. 3-Carbamoylbenzylamino, ferner -Ile-His-, -Ile-Phe-, -Gly-Gly-, -Gly-(N-methyl-Gly)-, -Ile-Sta-, C-terminal substituiert durch Amino, Alkylamino, z.B. Methyl-, Aethyl-, n-Butyl- oder n-Hexylamino, oder Hydroxyniederalkylamino, z.B. 2-Hydroxyäthylamino, oder -Ala-Sta-, C-terminal substituiert durch Niederalkoxy, z.B. Methoxy, Aethoxy oder tert-Butoxy, darstellt, sowie pharmazeutisch annehmbare Salze von diesen Verbindungen mit salzbildenden Gruppen.

Die Erfindung betrifft in erster Linie Verbindungen der Formel I, worin $R_1$ Wasserstoff, Niederalkanoyl, z.B. Formyl, Acetyl oder Pivaloyl, 2-(2-Methoxyäthoxy)-äthoxyacetyl, Diniederalkylaminoniederalkanoyl, z.B. Dimethylaminoacetyl, Cycloalkylcarbonyl, z.B. Cyclopropyl-, Cyclopentyl- oder Cyclohexylcarbonyl, Benzoyl, 5-Oxopyrrolidinyl-2-carbonyl, Niederalkoxycarbonyl, z.B. Methoxycarbonyl oder tert-Butoxycarbonyl, 1-Adamantyloxycarbonyl, Benzyloxycarbonyl, oder Niederalkylaminocarbonyl, z.B. tert-Butylaminocarbonyl, $X_1$ und $X_2$ zusammen -Phe-His-, welche N-terminal mit $R_1$ und C-terminal mit der Gruppe $-NR_2-$ verbunden sind, $R_2$ und $R_4$ Wasserstoff, $R_3$ Isobutyl, $R_5$ Isopropyl, $R_6$ Isoleucin, C-terminal substituiert durch Morpholin-4-yl-niederalkylamino, z.B. 3-(4-Morpholinyl)-propylamino oder 2-(4-Morpholinyl)-äthylamino, oder Carbamoylbenzylamino, z.B. 3-Carbamoylbenzylamino, oder -Ile-His-, -Ile-Phe-, oder -Ile-Sta-, C-terminal substituiert durch Amino oder Hydroxyniederalkylamino, z.B. 2-Hydroxyäthylamino, oder -Ala-Sta-, C-terminal substituiert durch Niederalkoxy, z.B. Methoxy, Aethoxy oder tert-Butoxy, darstellt, sowie pharmazeutisch annehmbare Salze von diesen Verbindungen mit salzbildenden Gruppen.

Die Erfindung betrifft zu allererst die in den Beispielen erwähnten Verbindungen und deren pharmazeutisch annehmbare Salze.

Die Erfindung betrifft vor allem folgende in den Beispielen beschriebene Verbindungen:

Benzyloxycarbonyl-Phe-His-(2(S)-amino-4-methyl-pentyl)-Val-Ile-His-$NH_2$,

tert-Butylaminocarbonyl-Phe-His-(2(S)-amino-4-methyl-pentyl)-Val-Ile-His-$NH_2$,

Benzyloxycarbonyl-Phe-His-(2(S)-amino-4-methyl-pentyl)-Val-Ile-3-carbamoylbenzylamid,

Benzyloxycarbonyl-Phe-His-(2(S)-amino-4-methyl-pentyl)-Val-Ala-Sta-$OCH_3$,

tert-Butoxycarbonyl-Phe-His-(2(S)-amino-4-methyl-pentyl)-Val-Ile-Sta-$NH_2$,

Pivaloyl-Phe-His-(2(S)-amino-4-methyl-pentyl)-Val-Ile-His-$NH_2$,

tert-Butoxycarbonyl-Phe-His-(2(S)-amino-4-methyl-pentyl)-Ser-Ile-His-$NH_2$.

Verfahren:

Die erfindungsgemässen Verbindungen der Formel I und Salze von solchen Verbindungen mit mindestens einer salzbildenden Gruppe werden nach an sich bekannten Verfahren erhalten, z.B. indem man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung einer Verbindung der Formel I, worin $R_6$ substituiertes Amino darstellt, in einer Verbindung der Formel

$$R_1 - X_1 - X_2 - \underset{}{\overset{R_2}{\overset{|}{N}}} - \underset{\underset{R^3}{|}}{CH} - CH_2 - \overset{R_4}{\overset{|}{N}} - \overset{R_5}{\overset{|}{CH}} - CN \quad (III),$$

bzw. in einer Verbindung der Formel

$$R_1 - X_1 - X_2 - \overset{R_2}{\overset{|}{N}} - \underset{\underset{R_3}{|}}{CH} - CH_2 - \overset{R_4}{\overset{|}{N}} - \overset{R_5}{\overset{|}{CH}} - \overset{O}{\overset{\|}{C}} - R_6' - CN \quad (IV),$$

worin die Gruppe $R_6'$-CN den Rest einer natürlichen α-Aminosäure, von Statin, eines Dipeptids oder eines Tripeptids, worin jeweils die terminale -COOH-Gruppe durch -CN ersetzt ist, bedeutet, die übrigen Substituenten die genannten Bedeutungen haben und vorhandene funktionelle Gruppen gegebenenfalls in geschützter Form vorliegen, die Cyanogruppe in eine N-substituierte oder gegebenenfalls unsubstituierte Carboxamidogruppe überführt, oder

c) eine Verbindung der Formel

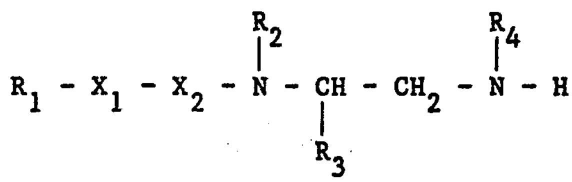

(V)

mit einem α-substituierten Ccarbonsäurederivat der Formel

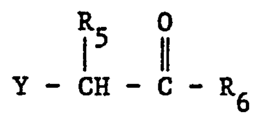

(VI),

worin die Substituenten die angegebenen Bedeutungen haben, Y eine Abgangsgruppe, z.B. Chlor, Brom oder Jod, bedeutet und freie funktionelle Gruppen gegebenenfalls in geschützter Form vorliegen, umsetzt, oder

d) eine Verbindung der Formel

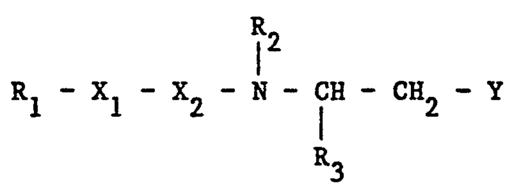

(VII)

mit einem α-Aminocarbonsäurederivat der Formel

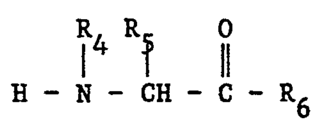

(VIII),

worin die Substituenten die genannten Bedeutungen haben, Y eine Abgangsgruppe, z.B. Chlor, Brom, Jod oder Sulfonyloxy, bedeutet und freie funktionelle Gruppen gegebenenfalls in geschützter Form vorliegen, umsetzt, oder

e) eine Verbindung der Formel V mit einem α-Oxocarbonsäurederivat der Formel

$$O = \overset{\overset{R_5}{|}}{C} - \overset{\overset{O}{\|}}{C} - R_6 \qquad (IX),$$

worin die Substituenten die genannten Bedeutungen haben und freie funktionelle Gruppen gegebenenfalls in geschützter Form vorliegen, kondensiert und das Kondensationsprodukt mit einem Reduktionsmittel behandelt, oder

f) eine Verbindung der Formel

$$R_1 - X_1 - X_2 - \overset{\overset{R_2}{|}}{N} - \underset{\underset{R_3}{|}}{CH} - CH = O \qquad (X)$$

mit einem α-Aminocarbonsäurederivat der Formel VIII, worin die Substituenten die genannten Bedeutungen haben und freie funktionelle Gruppen gegebenenfalls in geschützter Form vorliegen, kondensiert und das Kondensationsprodukt mit einem Reduktionsmittel behandelt,

und gewünschtenfalls in einer erhältlichen Verbindung vorhandene Schutzgruppen abspaltet und/oder gewünschtenfalls nach Ausführung eines der vorstehend genannten Verfahren oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

Die Erfindung betrifft auch die nach irgendeinem der oben genannten Verfahren erhältlichen anderen Verbindungen als Verbindungen der Formel I (Nebenprodukt), sowie Verbindungen der Formel I und ihre Salze, welche nach einem anderen Verfahren als einem der weiter vorn genannten hergestellt werden.

Verfahren a) (Herstellung einer Amidbindung):

Fragmente einer Verbindung der Formel I mit einer endständigen Carboxygruppe, welche mit einem zur Verbindung der Formel I komplementären Fragment unter Bildung einer Amidbindung kondensiert werden können, sind z.B. Verbindungen der Formeln: $R_1$-OH, $R_1$-$X_1$-OH, $R_1$-$X_1$-$X_2$-OH,

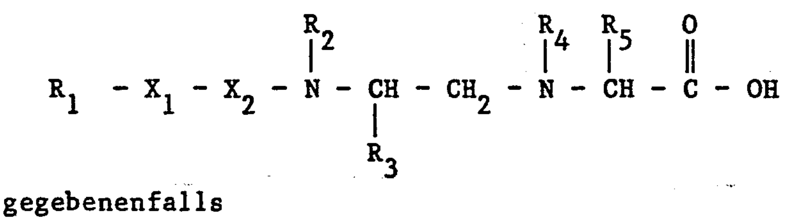
,

sowie gegebenenfalls

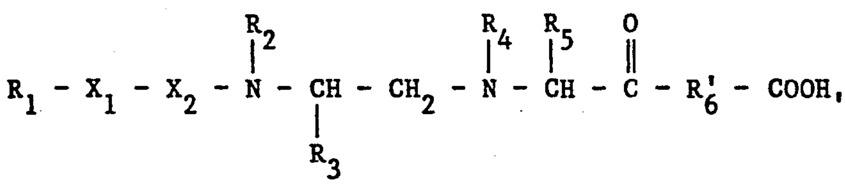
,

worin die Gruppe $R_6'$-COOH den Rest einer α-Aminosäure, von Statin, eines Dipeptids oder eines Tripeptids darstellt, wie er für $R_6$ oben definiert ist, oder die von diesen Verbindungen abgeleiteten aktivierten Ester oder reaktionsfähigen Anhydride, ferner reaktionsfähige cyclische Amide. Die reaktionsfähigen Säurederivate können auch in situ gebildet werden.

Aktivierte Ester sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters),

Carbamoylvinylester (erhältlich z.B. durch Behandeln der entsprechenden Säure mit einem Isoxazoliumreagens; 1,2-Oxazolium- oder Woodward-Methode) oder 1-Niederalkoxyvinylester (erhältlich z.B. durch Behandeln der entsprechenden Säure mit einem Niederalkoxyacetylen; Aethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode), oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten geeignet substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimid-Methode; Methode der aktivierten Thiolester), oder insbesondere Amino- oder Amidoester (erhältlich z.B. durch Behandeln der entsprechenden Säure mit einer N-Hydroxyamino- bzw. N-Hydroxyamido-Verbindung, z.B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benzotriazin-4-on, z.B. nach der Anhydrid- oder Carbodiimid-Methode; Methode der aktivierten N-Hydroxyester).

Anhydride von Säuren können symmetrische oder vorzugsweise gemischte Anhydride dieser Säuren sein, z.B. Anhydride mit anorganischen Säuren, wie Säurehalogenide, insbesondere Säurechloride (erhältlich

z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy-1,2-dihydrochinolin, z.B. 1-Niederalkoxycarbonyl-2-äthoxy-1,2-dihydrochinolin; Methode der gemischten O-Alkylkohlensäureanhydride), oder Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimids oder von 1-Diäthylaminopropin; Methode der symmetrischen Anhyride).

Geeignete cyclische Amide sind insbesondere Amide mit fünfgliedrigen Diazacyclen aromatischen Charakters, wie Amide mit Imidazolen, z.B. Imidazol (erhältlich z.B. durch Behandeln der entsprechenden Säure mit N,N'-Carbonyldiimidazol; Imidazol-Methode), oder Pyrazol, z.B. 3,5-Dimethylpyrazol (erhältlich z.B. über das Säurehydrazid durch Behandeln mit Acetylaceton; Pyrazolid-Methode).

Zur Verbindung der Formel I komplementäre Fragmente mit einer freien Aminogruppe sind je nach Bedeutung von $R_6$ Ammoniak, ein primäres oder sekundäres Amin, eine α-Aminosäure, Statin, ein Dipeptid oder ein Tripeptid mit einer freien α-Aminogruppe, ferner Verbindungen der Formeln:

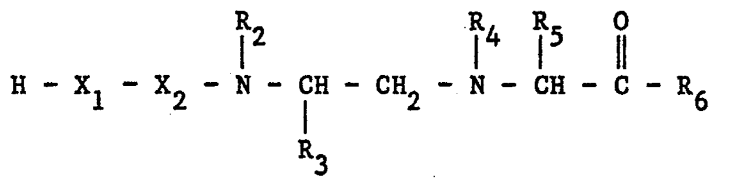

,

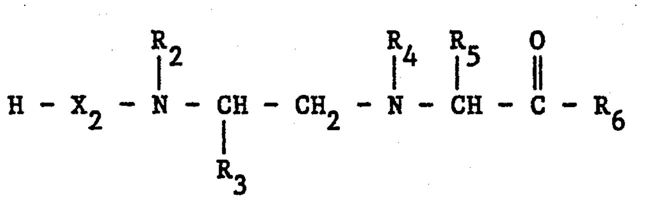

,

oder 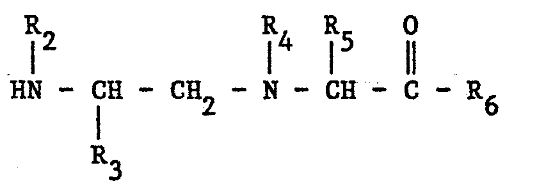 .

Die an der Reaktion teilnehmende Aminogruppe in einem zu einer Verbindung der Formel I komplementären Fragment liegt bevorzugt in freier Form vor, insbesondere wenn die damit reagierende Carboxygruppe in reaktionsfähiger Form vorliegt, sie kann aber auch selbst derivatisiert sein, z.B. durch Reaktion mit einem Phosphit, wie Diäthylchlorphosphit, 1,2-Phenylenchlorphosphit, Aethyldichlorphosphit, Aethylenchlorphosphit oder Tetraäthylpyrophosphit. Ein Derivat eines solchen komplementären Bruchstücks mit einer Aminogruppe ist z.B. auch ein Carbaminsäurehalogenid oder ein Isocyanat, wobei die an der Reaktion teilnehmende Aminogruppe durch Halogencarbonyl, z.B. Chlorcarbonyl, substituiert bzw. als Isocyanatgruppe abgewandelt ist, wobei im letzteren Falle nur Verbindungen der Formel I zugänglich sind, die am Stickstoffatom der durch die Reaktion gebildeten Amidgruppe ein Wasserstoffatom tragen.

Ist das komplementäre Fragment mit einer Aminogruppe Ammoniak selbst oder ein durch Niederalkyl oder Arylniederalkyl mono- oder disubstituiertes Amin, so stellt auch eine entsprechende Harnstoff-Verbindung ein reaktionsfähiges Derivat dar. Beispielsweise erhält man beim Erhitzen äquimolarer Mengen dieser Harnstoffverbindung und der Komponente mit freier Carboxygruppe entsprechende Verbindungen der Formel I mit zum Teil guter Ausbeute.

Ist das komplementäre Fragment Dimethylamin, so ist auch Dimethylformamid ein reaktionsfähiges Derivat.

Funktionelle Gruppen in Ausgangsmaterialien, deren Umsetzung vermieden werden soll, insbesondere Carboxy-, Amino-, Hydroxy- und Mercaptogruppen, können durch geeignete Schutzgruppen (conventional protecting groups) geschützt sein, die üblicherweise bei der Synthese von Peptid-Verbindungen, aber auch von Cephalosporinen und Penicillinen verwendet werden. Diese Schutzgruppen können bereits in den Vorstufen vorhanden sein und sollen die betreffenden funktionellen Gruppen gegen unerwünschte Nebenreaktionen wie Acylierungen, Verätherungen, Veresterungen, Oxydationen, Solvolyse etc. schützen. Schutzgruppen können aber auch in den Endstoffen vorhanden sein. Verbindungen der Formel I mit geschützten funktionellen Gruppen können eine höhere metabolische Stabilität aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungsreaktionen, sind beispielsweise in Standardwerken wie in J.F.W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (Herausg. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben.

Eine Carboxygruppe ist z.B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z.B. tert-Butoxycarbonyl, Arylmethoxycarbonyl mit einem oder zwei Arylresten, worin Aryl unsubstituiertes oder z.B. durch Niederalkyl, z.B. tert-Niederalkyl, wie tert-Butyl, Niederalkoxy, z.B. Methoxy, Hydroxy. Halogen, z.B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise 1-Niederalkoxyniederalkoxycarbonyl, z.B. Methoxymethoxycarbonyl, 1-Methoxyäthoxycarbonyl oder 1-Aethoxyäthoxycarbonyl, 1-Niederalkylthioniederalkoxycarbonyl, z.B. 1-Methylthiomethoxycarbonyl oder 1-Aethylthioäthoxycarbonyl, Aroylmethoxycarbonyl, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, sowie 2-Triniederalkylsilylniederalkoxycarbonyl, z.B. 2-Trimethylsilyläthoxycarbonyl.

Eine Carboxygruppe kann auch als organische Silyloxycarbonylgruppe geschützt sein. Eine organische Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch durch zwei Niederalkylgruppen, z.B. Methylgruppen, und die Aminogruppe oder die Carboxygruppe eines zweiten Moleküls der Formel I substituiert sein. Verbindungen mit solchen Schutzgruppen lassen sich z.B. mit Dimethylchlorsilan als Silylierungsmittel herstellen.

Eine geschützte Carboxygruppe ist bevorzugt tert-Niederalkoxycarbonyl, z.B. tert-Butoxycarbonyl, Benzyloxycarbonyl, 4-Nitrobenzyloxycarbonyl oder Diphenylmethoxycarbonyl.

Eine Aminogruppe kann z.B. in Form einer Acylamino-, Arylmethylamino-, verätherten Mercaptoamino- oder Silylaminogruppe oder als Azidogruppe geschützt sein.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Niederalkancarbonsäure oder gegebenenfalls, z.B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Jod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem oder zwei Arylresten, die gegebenenfalls, z.B. durch Niederalkyl, z.B. tert-Niederalkyl, wie tert-Butyl, Niederalkoxy, wie Methoxy, Hydroxy, Halogen, wie Chlor, und/oder Nitro, mono- oder polysubstituiertes Phenyl darstellen, z.B. Benzyloxycarbonyl, 4-Nitrobenzyloxycarbonyl, Diphenylmethoxycarbonyl oder Di-(4-methoxyphenyl)-methoxycarbonyl, Aroylmethoxycarbonyl, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, 2-Triniederalkylsilylniederalkoxycarbonyl, z.B. 2-Trimethlysilyläthoxycarbonyl, oder 2-Triarylsilylniederalkoxycarbonyl, z.B. 2-Triphenylsilyläthoxycarbonyl.

Eine Arylmethylaminogruppe ist z.B. Mono-, Di- oder insbesondere Triphenylmethylamino, z.B. Benzyl-, Diphenylmethyl- oder Tritylamino.

In einer verätherten Mercaptoaminogruppe ist die verätherte Mercaptogruppe in erster Linie substituiertes Arylthio, z.B. 4-Nitrophenylthio.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. mit Dimethylchlorsilan als Silylierungsmittel herstellen.

Bevorzugte Aminoschutzgruppen sind Acylreste von Kohlensäurehalbestern, insbesondere tert-Butoxycarbonyl, gegebenenfalls substituiertes Benzyloxycarbonyl, z.B. 4-Nitrobenzyloxycarbonyl, Diphenylmethoxycarbonyl, 2-Halogen-niederalkoxycarbonyl, z.B. 2,2,2-Trichloräthoxycarbonyl, ferner Trityl oder Formyl.

Eine Hydroxygruppe kann beispielsweise durch eine Acylgruppe, z.B. durch Halogen, z.B. Chlor, substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise

2,2,2-Trichloräthoxycarbonyl, 4-Nitrobenzyloxycarbonyl, Diphenylmethoxycarbonyl oder Trityl. Eine Hydroxygruppe kann ferner durch Triniederalkylsilyl, z.B. Trimethylsilyl oder Dimethyl-tert-butylsilyl, eine leicht abspaltbare Alkylgruppe, wie tert-Niederalkyl, z.B. tert-Butyl, einen oxa- oder einen thiaaliphatischen oder -cycloaliphatischen Kohlenwasserstoffrest, beispielsweise 1-Niederalkoxyniederalkyl oder 1-Niederalkylthioniederalkyl, z.B. Methoxymethyl, 1-Methoxyäthyl, 1-Aethoxyäthyl, Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl, oder ein entsprechendes Thiaanaloges, sowie durch 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt sein.

Zwei benachbarte Hydroxylgruppen können beispielsweise durch eine vorzugsweise substituierte Methylengruppe geschützt sein, z.B. durch Niederalkyliden, z.B. Isopropyliden, Cycloalkyliden, z.B. Cyclohexyliden, oder Benzyliden.

Eine Mercaptogruppe, wie z.B. in Cystein, kann insbesondere durch S-Alkylierung mit gegebenenfalls substituierten Alkylresten, Thioacetalbildung, S-Acylierung oder durch die Bildung asymmetrischer Disulfid-Gruppierungen geschützt sein. Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls am Phenylrest, z.B. durch Methoxy, substituiertes Diphenylmethyl, wie 4,4'-Dimethoxydiphenylmethyl, Triphenylmethyl, Trimethylsilyl, Benzylthiomethyl, Tetrahydropyranyl, Acylaminomethyl, Benzoyl, Benzyloxycarbonyl oder Niederalkylaminocarbonyl, wie Aethylaminocarbonyl.

Die Kondensation zur Herstellung der Amidbindung kann in an sich bekannter Weise durchgeführt werden, beispielsweise wie in Standardwerken, wie "Houben-Weyl, Methoden der organischen Chemie",

4. Auflage, Band 15/II, Georg Thieme Verlag, Stuttgart 1974, "The Peptides" (Herausg. E. Gross und J. Meienhofer), Band 1 und 2, Academic Press, London und New York, 1979/1980, oder M. Bodanszky, "Priciples of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diäthyl-, Dipropyl-, N-Aethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Aethyl-5-phenyl-1,2-oxazolium-3'-sulfonat und 2-tert-Butyl-5-methylisoxazoliumperchlorat, oder eine geeignete Acylaminoverbindung, z.B. 2-Aethoxy-1-äthoxycarbonyl-1,2-dihydrochinolin, ferner aktivierte Phosphate, z.B. Diphenylphosphorylazid, Diäthylphosphorylcyanid oder Phenyl-N-phenylphosphoramidochloridat.

Gewünschtenfalls wird eine organische Base zugegeben, z.B. ein Triniederalkylamin mit voluminösen Resten, z.B. Aethyldiisopropylamin, oder eine heterocyclischen Base, z.B. Pyridin, 4-Dimethylaminopyridin oder bevorzugt N-Methylmorpholin.

Die Kondensation von Säureanhydriden mit Aminen kann z.B. in Gegenwart von anorganischen Carbonaten, z.B. Alkalimetallcarbonaten oder -hydrogencarbonaten, wie Natrium- oder Kaliumcarbonat oder -hydrogencarbonat, üblicherweise zusammen mit einem Sulfat, erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Reaktionsfähige Säurederivate können auch in situ gebildet werden. So kann man z.B. N,N'-disubstituierte Amidinoester in situ bilden, indem man das Gemisch des Fragments mit freier Carboxygruppe und des komplementären Fragments mit einer Aminogruppe in Gegenwart eines geeigneten disubstituierten Carbodiimids, z.B. Dicyclohexylcarbodiimid, umsetzt. Ferner kann man Amino- oder Amidoester von solchen Säuren in Gegenwart der zu acylierenden Aminokomponente bilden, indem man das Gemisch der entsprechenden Säure- und Amino-Ausgangsstoffe in Gegenwart eines disubstituierten Carbodiimids, z.B. Dicyclohexylcarbodiimid, und eines N-Hydroxylamins oder N-Hydroxyamids, z.B. N-Hydroxybenztriazol, N-Hydroxysuccinimid oder N-Hydroxy-5-norbornen-2,3-dicarbonsäureimid, gegebenenfalls in Anwesenheit einer geeigneten Base, z.B. 4-Dimethylaminopyridin, N-Methylmorpholin oder Aethyldiisopropylamin, umsetzt.

Verfahren b) (Ueberführung einer Cyanogruppe in eine Amidgruppe)

In einem Ausgangsmaterial der Formel III oder IV sind funktionelle Gruppen durch die unter a) genannten Schutzgruppen gegebenenfalls geschützt.

Die Ueberführung einer Verbindung der Formel III oder IV in eine Verbindung der Formel I kann durch partielle Hydrolyse, durch eine Ritter-Reaktion oder über Carbonsäureesterimid-Salze erfolgen.

Die Bedingungen der Hydrolyse einer Verbindung der Formel IV können so gewählt werden, dass die Reaktion auf der Stufe des Amids stehen bleibt. Zu diesem Zweck wird das Nitril der Formel IV mit Säure

hydrolysiert, wobei man je nach thermischer Empfindlichkeit der in einer Verbindung der Formel IV vorhandenen Substituenten insbesondere zwischen 80%iger Schwefelsäure (unter Erwärmen), Polyphosphorsäure (bei 110-150°), Bromwasserstoff/Eisessig oder Ameisensäure (Raumtemperatur), Chlorwasserstoffgas in ätherischer Lösung gefolgt von Wasser oder wässriger Salzsäure, oder Borhalogeniden z.B. Bortrifluorid-Essigsäure-Komplex, gefolgt von Wasser, wählen kann. In einigen Fällen gelingt auch die alkalische Hydrolyse, insbesondere nach der Methode von Radziszewski, mit Wasserstoffperoxid in Gegenwart von Alkalimetallhydroxiden bei mässiger Temperatur, z.B. Raumtemperatur.

Mit Hilfe der Ritter-Reaktion gelingt die Herstellung N-substituierter Amide aus Nitrilen der Formel III oder IV. Hierzu setzt man die Nitrile in Gegenwart einer starken Säure, beispielsweise 85-90%iger Schwefelsäure, oder auch Polyphosphorsäure, Fluorwasserstoff, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Benzylalkohol, meist ohne Lösungsmittel oder beispielsweise in Eisessig.

In einer Variante der Ritter-Reaktion wird ein Nitril der Formel III oder IV mit einem Olefin und Quecksilber(II)-nitrat umgesetzt und die Organoquecksilberverbindung anschliessend mit Natriumborhydrid zu einer Verbindung der Formel I reduziert.

Die Carbonsäureesterimide erhält man z.B. durch säurekatalysierte Anlagerung von Alkoholen an die Nitrile, bevorzugt in Gegenwart von Chlorwasserstoff. Aus den so gebildeten Esterimid-Salzen erhält man die Amide durch thermische Umlagerung bei Temperaturen oberhalb von etwa 80°C.

Verfahren c) und d) (Alkylierung eines Amins mit einem Halogenid, Sulfonat oder dergleichen)

Die Umsetzung eines Aethylendiamin-Derivats der Formel V mit einem α-substituierten Carbonsäurederivat der Formel VI (Verfahren c)) oder eines ß-substituierten Aethylamin-Derivats der Formel VII mit einem α-Aminocarbonsäurederivat der Formel VIII (Verfahren d)) wird nach an sich bekannten Methoden der Alkylierung von Aminen durchgeführt, wie sie beispielsweise in "Houben-Weyl, Methoden der organischen Chemie", Band 11/1, S. 24, Thieme-Verlag, Stuttgart 1957, beschrieben sind.

Geeignete Carbonsäurederivate der Formel VI sind beispielsweise α-Halogencarbonsäurederivate, worin Halogen Chlor, Brom oder Jod ist, aber auch α-Hydroxycarbonsäurederivate mit veresterter, durch Nukleophile leicht substituierbarer Hydroxygruppe, z.B. α-4-Nitrobenzoyloxycarbonsäurederivate. In einem ß-substituierten Aethylamin-Derivat der Formel VII kann der Substituent Y Halogen, z.B. Chlor, Brom oder Jod, Sulfonyloxy, z.B. Niederalkansulfonyloxy, wie Methansulfonyloxy, auch Trifluormethansulfonyloxy, oder Arylsulfonyloxy, wie Benzolsulfonyloxy, p-Toluolsulfonyloxy, p-Brombenzolsulfonyloxy, oder p-Nitrosulfonyloxy, Arylcarbonyloxy, z.B. 2,4-Dinitrobenzoyloxy, Aryloxy, z.B. 2,4,6-Trinitrophenoxy, oder dergleichen sein, wobei dieser Substituent durch Nukleophile leicht austauschbar sein muss.

In den Reaktionskomponenten sind leicht alkylierbare funktionelle Gruppen, beispielsweise Amino- oder Mercapto-Gruppen, gewünschtenfalls aber auch Hydroxy- und Carboxy-Gruppen, durch eine der unter Verfahren a) beschriebenen Schutzgruppen geschützt.

Die Umsetzung wird vorteilhafterweise in einem polaren Lösungsmittel in Gegenwart einer organischen oder anorganischen Base bei Temperaturen zwischen ca. -20°C bis ca. +100°C, beispielsweise zwischen 0°C und +50°C, durchgeführt.

Geeignete polare Lösungsmittel sind beispielsweise ein Alkohol, z.B. Aethanol, Isopropanol oder tert-Butanol, Diole, z.B. Aethylenglykol oder Diäthylenglykol, auch in mono-alkylierter Form, wie in 2-Methoxyäthanol oder 2-(2-Methoxy)-äthoxyäthanol, ferner polare aprotische Lösungsmittel, z.B. Aceton, Acetonitril, Tetrahydrofuran, Dioxan, Dimethylformamid, Dimethylacetamid, N-Methylpyrrolidon, Dimethylsulfoxid, Hexamethylphosphorsäuretriamid oder dergleichen.

Es ist möglich, die Reaktion ohne Zusatz einer Base durchzuführen, doch wird in solchen Fällen vorteilhafterweise ein Ueberschuss der Amin-Komponente eingesetzt, um den bei der Umsetzung freigesetzten Halogenwasserstoff bzw. die Sulfonsäure oder Carbonsäure zu binden. Die Reaktion verläuft aber glatt mit äquimolaren Mengen der Reaktionspartner, wenn eine nicht-nukleophile Base zugesetzt wird, beispielsweise ein anorganisches Carbonat, z.B. ein Alkalimetall- oder Erdalkalimetallcarbonat, wie Natrium-, Kalium-, Calcium- oder Magnesiumcarbonat, oder ein Alkalimetallhydrogencarbonat, wie Natrium- oder Kaliumhydrogencarbonat, ferner sterisch gehinderte Triniederalkylamine, z.B. Aethyldiisopropylamin, oder Amidinbasen, z.B. 1,5-Diazabicyclo[4.3.0]nonen-5 (DBN) oder 1,8-Diazabicyclo[5.4.0]undecen-7 (DBU).

Dem Reaktionsgemisch kann gewünschtenfalls eine katalytische oder äquimolare Menge eines anorganischen Jodids, z.B. eines Alkalimetalljodids, wie Natriumiodid oder Kaliumiodid, zugesetzt werden, um wenig reaktive Abgangsgruppen Y, z.B. Chlor, Brom oder ein Sulfonat, in situ in das reaktive Jod umzuwandeln.

Verfahren e) und f) (reduktive Aminierung einer Carbonylverbindung)
Die Kondensation eines Aethylendiamin-Derivats der Formel V mit einem α-Oxocarbonsäurederivat der Formel IX mit anschliessender Reduktion (Verfahren e)) bzw. die Kondensation eines α-Aminocarbonsäurederivats der Formel VIII mit einem substituierten α-Aminoaldehyden der Formel X mit anschliessender Reduktion (Verfahren f)) zu

einer Verbindung der Formel I wird nach an sich bekannten Methoden der reduktiven Aminierung durchgeführt, wie sie beispielsweise in "Houben-Weyl, Methoden der organischen Chemie", Band 11/1, S. 602, Thieme-Verlag, Stuttgart 1957, beschrieben sind.

In den Reaktionskomponenten sind reaktive funktionelle Gruppen, beispielsweise nicht an der Reaktion teilnehmende Aminogruppen, durch eine der unter Verfahren a) beschriebenen Schutzgruppen geschützt.

Die Reaktion wird beispielsweise in einem Schritt durchgeführt, wobei das Kondensationsprodukt aus dem Amin der Formel V oder VIII und der Carbonylverbindung der Formel IX bzw. X in situ durch das im Reaktionsgemisch vorhandene Reduktionsmittel abgefangen und zu einem substituierten α-Aminocarbonsäurederivat der Formel I reduziert wird. Als Reduktionsmittel im Einschritt-Verfahren kommen Reagentien in Frage, die selektiv C=N-Doppelbindungen in Gegenwart von Aldehyden oder Ketonen reduzieren, beispielsweise Natriumcyanoborhydrid. Die reduktive Aminierung wird bevorzugt in einem Alkohol oder in einem Lösungsmittelgemisch, enthaltend einen Alkohol, beispielsweise in Methanol, Aethanol, Isopropanol oder Gemischen dieser Alkohole mit Tetrahydrofuran, Dioxan, Dimethoxyäthan oder einem anderen polaren Aether oder auch Dimethylformamid oder Dimethylacetamid und/oder gewünschtenfalls Wasser, in einem pH-Bereich von pH 3 bis pH 8 bei Temperaturen zwischen ca. -20°C bis ca. +100°C, beispielsweise zwischen 0°C und +50°C, durchgeführt.

Es ist auch möglich, die reduktive Aminierung in zwei Schritten durchzuführen. Dabei wird im ersten Schritt nach an sich bekannten Methoden das Amin der Formel V oder VIII mit der Carbonylverbindung der Formel IX bzw. X in einem inerten Lösungsmittel, beispielsweise in einem Kohlenwasserstoff, z.B. Toluol, Halogenkohlenwasserstoff, z.B. Methylenchlorid oder Dichloräthan, einem Nitril, z.B. Acetonitril, einem Aether, z.B. Tetrahydrofuran, Dioxan oder 1,2-Dimethoxyäthan, oder dergleichen in Gegenwart eines Wasser-bindenden

Mittels, beispielsweise Molekularsieb oder konzentrierter Schwefelsäure, oder auch mit azeotroper Entfernung des Reaktionswassers, gewünschtenfalls mit einer katalytischen Menge einer Säure, z.B. Mineralsäure, wie Halogenwasserstoff oder Schwefelsäure, einer organischen Sulfonsäure, wie p-Toluolsulfonsäure, oder einer Lewissäure, wie Bortrifluoridätherat, oder einer katalytischen Menge einer Base, z.B. eines tertiären Amins, wie Triäthylamin oder N-Methylmorpholin, bei Temperaturen zwischen ca. 0° und ca. 150°C, beispielsweise beim Siedepunkt des Lösungsmittels, kondensiert. Im zweiten Schritt wird das Kondensationsprodukt, bei dem es sich um ein Imin oder ein Enamin handelt, mit einem Reduktionsmittel umgesetzt, beispielsweise mit Natriumcyanoborhydrid oder einem anderen Borhydrid unter Bedingungen, wie sie oben für die Einschritt-Methode beschrieben sind, oder mit Wasserstoff in Gegenwart eines Hydrier-Katalysators, beispielsweise eines heterogenen Katalysators, wie Platinoxid, Nickel, Platin oder Palladium, gegebenenfalls auf einem anorganischen Träger, oder eines homogenen Katalysators, wie eines Rhodium- oder Iridiumkomplexes, gegebenenfalls in Gegenwart eines chiralen Komplexliganden, bei einem Druck von ca. 1 bar bis ca. 100 bar, bevorzugt bei Normaldruck oder leichtem Ueberdruck in einem inerten Lösungsmitel und bei Temperaturen zwischen ca. 0° und +100°C, bevorzugt um Raumtemperatur.

Nachoperationen:

In einer erhältlichen Verbindung der Formel I, worin $R_1$, $X_1$, $X_2$, $R_2$, $R_3$, $R_4$ und $R_5$ die genannten Bedeutungen haben und $R_6$ den Rest einer Aminosäure, eines Dipeptids oder eines Tripeptids mit einer endständigen Carboxamid-, Carboxy- oder Carbonsäureestergruppe darstellt, kann man die endständige Carboxamidgruppe substituieren, die in freier oder in reaktionsfähiger Form vorliegende Carboxygruppe verestern bzw. eine veresterte Carboxygruppe in eine Carboxamidgruppe überführen.

Die Substitution der terminalen Carboxamidgruppe oder einer anderen Aminogruppe erfolgt z.B. durch Alkylierung.

Geeignete Mittel zur Alkylierung der terminalen Carboxamidgruppe in einer Verbindung der Formel I sind z.B. Diazoverbindungen, z.B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z.B. in Gegenwart eines Edelmetalls in fein verteilter Form, z.B. Kupfer, oder eines Edelmetallsalzes, z.B. Kupfer(I)-chlorid oder Kupfer(II)-sulfat.

Weitere Alkylierungsmittel sind die in der Deutschen Offenlegungsschrift 2 331 133 genannte Alkylierungsmittel, z.B. Alkylhalogenide, Sulfosäureester, Meerweinsalze oder 1-substituierte 3-Aryltriazene. welche man unter den dort genannten Reaktionsbedingungen mit einer Verbindung der Formel I mit endständiger Carboxamidgruppe umsetzen kann.

Zur Veresterung einer endständigen Carboxygruppe in einer Verbindung der Formel I kann man die freie Säure verwenden oder die freie Säure in eines der unter Verfahren a) genannten reaktionsfähigen Derivate überführen und mit einem Alkohol umsetzen, oder man kann die freie Säure oder ein reaktionsfähiges Salz, z.B. das Cäsiumsalz, mit einem reaktionsfähigen Derivat eines Alkohols umsetzen. Beispielsweise kann man das Cäsiumsalz einer Carbonsäure mit dem Halogenid eines Alkohols umsetzen.

Die Veresterung einer endständigen Carboxygruppe kann mit den für die Substitution der Carboxamidgruppe oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

In einer erhältlichen Verbindung der Formel I kann man eine endständige veresterte Carboxygruppe durch Aminolyse mit Ammoniak oder einem primären oder sekundären Amin in eine gegebenenfalls substituierte Carboxamidgruppe überführen.

Die Aminolyse kann nach den im Organikum, letzte Auflage, VEB Deutscher Verlag der Wissenschaften, Berlin (Ost) für solche Umsetzungen genannten Reaktionsbedingungen erfolgen.

In einer erhältlichen Verbindung der Formel I, worin die Substituenten die genannten Bedeutungen haben und mindestens eine freie Hydroxygruppe vorhanden ist und die übrigen funktionellen Gruppen gegebenenfalls in geschützter Form vorliegen, kann man die freie Hydroxygruppe veräthern oder verestern.

Die Verätherung dieser Hydroxygruppe kann mit den oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

Die Veresterung der freien Hydroxygruppe kann mit den üblichen Acylierungsmitteln und den üblichen im Organikum angegebenen Reaktionsbedingungen erfolgen, z.B. mit Essigsäureanhydrid.

Die genannten Alkylierungsreaktionen, Verätherungen, Veresterungen etc. können statt im Endstoff auch in einem Ausgangsmaterial entsprechend durchgeführt werden.

In einer erhältlichen Verbindung der Formel I, worin eine oder mehrere funktionelle Gruppen geschützt sind, können diese Gruppen, z.B. Carboxy-, Amino-, Hydroxy- und/oder Mercaptogruppen, in an sich bekannter Weise, mittels Solvolyse, insbesondere Hydrolyse, gegebenenfalls enzymatische Hydrolyse, Alkoholyse oder Acidolyse, oder

mittels Reduktion, insbesondere Hydrogenolyse, oder chemische Reduktion, gegebenenfalls stufenweise oder gleichzeitig, freigesetzt werden. Die Abspaltung der Schutzgruppen ist in den weiter vorn im Abschnitt "Schutzgruppen" genannten Standardwerken beschrieben.

Beispielsweise kann man geschütztes Carboxy, z.B. tert-Niederalkoxycarbonyl, in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nukleophilen Verbindung, wie Phenol oder Anisol, in freies Carboxy überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch durch Reduktion, z.B. durch Behandeln mit einem Alkalimetall-, z.B. Natrium-dithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxy umwandeln. Aroylmethoxycarbonyl kann ebenfalls durch Behandeln mit einem nukleophilen, vorzugsweise salzbildenden Reagens, wie Natriumthiophenolat oder Natriumjodid, gespalten

werden. 2-Triniederalkylsilylniederalkoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy kann in üblicher Weise solvolytisch, z.B. durch Behandeln mit Wasser, einem Alkohol oder Säure, oder ausserdem einem Fluorid, wie oben beschrieben, freigesetzt werden. Verestertes Carboxy kann auch enzymatisch gespalten werden, z.B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nukleophilen, vorzugsweise salzbildenden Reagenz, wie Natriumthiophenolat, und 4-Nitrobenzyloxycarbonylamino auch durch Behandeln mit einem Alkalimetall-, z.B. Natrium-dithionit, gespalten werden. Gegebenenfalls substituiertes Diphenylmethoxycarbonylamino, tert-Niederalkoxycarbonylamino oder 2-Triniederalkylsilylniederalkoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kolenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert-Niederalkyl oder durch einen 2-oxa- oder 2-thia-aliphatischen oder -cycloaliphatischen Kohlenwasserstoffrest, durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der 2-Aethylhexansäure, oder mit anorganischen Alkali- oder Erdalkalimetallsalzen, z.B. Natriumhydrogencarbonat, oder mit Ammoniak oder einem geeigneten organische Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauscherreagenz. Innere Salze von Verbindungen der Formel I, welche z.B. eine freie Carboxygruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen übergeführt werden; Metall- und Ammoniumsalze z.B. durch Behandeln mit geeigneten Säuren, Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Pharmazeutische Präparate:

Die pharmakologisch verwendbaren Verbindungen der vorliegenden Erfindung können z.B. zur Herstellung von pharmazeutischen Präparaten verwendet werden, welche eine wirksame Menge des Wirkstoffs zusammen oder im Gemisch mit einer signifikanten Menge von anorganischen oder organischen, festen oder flüssigen, pharmazeutisch verwendbaren Trägerstoffen enthalten.

Zur enteralen, wie nasalen, rektalen oder oralen, oder parenteralen, wie intramuskulären oder intravenösen, Verabreichung an Warmblüter (Menschen und Tiere) werden pharmazeutische Präparate verwendet, welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, Alter und dem individuellen Zustand, der zu behandelnden Krankheit sowie von der Applikationsweise ab.

Die an Warmblüter, z.B. Menschen, von etwa 70 kg Körpergewicht zu verabreichenden Dosismengen liegen zwischen etwa 3 mg und etwa 3 g, vorzugsweise zwischen etwa 10 mg und etwa 1 g, z.B. bei ungefähr 300 mg pro Person und Tag, verteilt auf vorzugsweise 1 bis 3 Einzeldosen, die z.B. gleich gross sein können. Ueblicherweise erhalten Kinder die halbe Dosis von Erwachsenen.

Die neuen pharmazeutischen Präparate enthalten von etwa 1% bis etwa 95%, vorzugsweise von etwa 20% bis etwa 90%, des Wirkstoffes. Erfindungsgemässe pharmazeutische Präparate können z.B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffs, daneben auch Suspensionen, und zwar insbesondere isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Oel enthalten als ölige Komponente die für Injektionszwecke gebräuchlichen pflanzlichen, synthetischen oder halbsynthetischen Oele. Als solche sind insbesondere flüssige Fettsäureester zu nennen, die als Säurekomponente eine langkettige Fettsäure mit 8-22, insbesondere 12-20, C-Atomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren wie z.B. Oelsäure, Elaidinsäure, Erucasäure, Brasidinsäure oder Linolsäure, enthalten. Die Alkoholkomponente dieser Fettsäureester hat maximal 6 Kohlenstoffatome und ist ein ein-, zwei- oder dreiwertiger Alkohol, z.B. Methanol, Aethanol, Propanol, Butanol oder Pentanol oder deren Isomere, vor allem aber Glycol oder Glyzerin. Als Fettsäureester sind daher beispielsweise zu nennen: Aethyloleat, Isopropylmyristat, Isopropylpalmitat, "Labrafil M 2735" (Polyoxyäthylenglyzerintrioleat der Firma Gattefossé, Paris), "Myglyol 812" (Triglyzerid gesättigter Fettsäuren der Kettenlänge $C_8$ bis $C_{12}$ der Firma Chemische Werke Witten/Ruhr, Deutschland), besonders aber pflanzliche Oele wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig, nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten, Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Aethylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

<u>Ausgangsmaterialien:</u>

Neue Ausgangsmaterialien und/oder Zwischenprodukte, sowie Verfahren zu ihrer Herstellung, sind ebenfalls Gegenstand der vorliegenden Erfindung. Vorzugsweise werden solche Ausgangsstoffe verwendet und die Reaktionsbedingungen so gewählt, dass man zu den als bevorzugt aufgeführten Verbindungen gelangt.

Die Ausgangsmaterialien zur Durchführung des Verfahrens a) sind bekannt oder können, falls sie neu sind, nach an sich bekannten Verfahren hergestellt werden, z.B. aus den betreffenden Aminosäuren, Dipeptiden oder Tripeptiden durch Kondensation analog dem vorstehend beschriebenen Verfahren a).

Beispielsweise kann man eine Verbindung der Formel

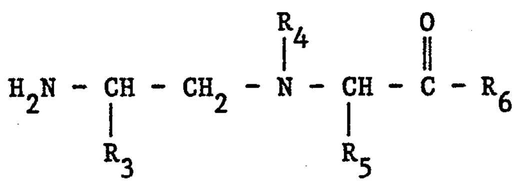
(XI),

worin $R_3$, $R_4$, $R_5$ und $R_6$ die unter Formel I genannten Bedeutungen haben, herstellen, indem man eine Verbindung der Formel

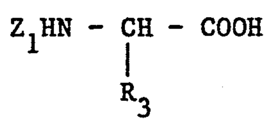
(XII),

worin $Z_1$ eine Aminoschutzgruppe ist und $R_3$ die unter Formel I genannten Bedeutungen hat, oder ein reaktionsfähiges, funktionelles Derivat davon mit einer Verbindung der Formel

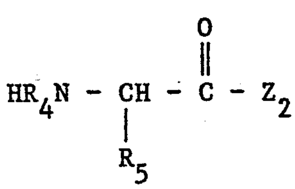
(XIII),

worin $R_4$ und $R_5$ die genannten Bedeutungen haben und $Z_2$ eine Carboxyschutzgruppe ist, unter Bildung einer Amidbindung kondensiert und in einer erhältlichen Verbindung der Formel

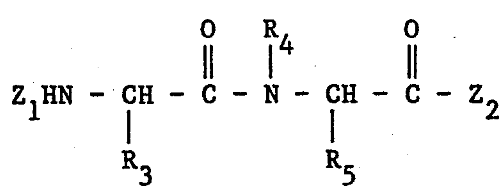
(XIV),

worin die Substituenten die genannten Bedeutungen haben, die Carbonylgruppe der Amidbindung zu einer $-CH_2-$ Gruppe reduziert, in einer erhältlichen Verbindung der Formel

$$Z_1HN - \underset{\displaystyle R_3}{\underset{|}{CH}} - CH_2 - \overset{\displaystyle R_4}{\overset{|}{N}} - \underset{\displaystyle R_5}{\underset{|}{CH}} - \overset{\displaystyle O}{\overset{\|}{C}} - Z_2 \qquad (XV)$$

in beliebiger Reihenfolge der Reaktionsschritte oder gleichzeitig die Schutzgruppen $Z_1$ und $Z_2$ abspaltet, gewünschtenfalls ein erhältliches Isomerengemisch in die einzelnen Isomere auftrennt und die erhältliche Verbindung mit einer freien Carboxygruppe je nach Bedeutung des einzuführenden Rests $R_6$ amidiert, verestert oder mit einem C-terminal gegebenenfalls amidierten oder veresterten Rest einer Aminosäure, eines Dipeptids oder eines Tripeptids substituiert.

Die Aminoschutzgruppe $Z_1$ ist eine der üblichen Schutzgruppen, wie sie weiter vorn unter Verfahren a) genannt sind, z.B. tert-Butoxycarbonyl oder Benzyloxycarbonyl.

Eine Carboxyschutzgruppe $Z_2$ ist eine der weiter vorn unter Verfahren a) genannten Carboxyschutzgruppen, z.B. verzweigtes Niederalkoxy, wie tert-Butoxy, aber auch Methoxy oder Aethoxy.

Die Umsetzung des Aminosäurederivats der Formel XII mit einem Aminosäurederivat der Formel XIII erfolgt in der unter Verfahren a) beschriebenen Weise.

Die Reduktion der Carbonylgruppe der Amidbindung in einer Verbindung der Formel XIV kann stufenweise erfolgen, beispielsweise indem man diese Carbonylgruppe in eine Thiocarbonylgruppe überführt, z.B. mit $P_2S_5$ oder nach dem von Lawesson et al. in Nouv. J. Chim. 4, 43

(1980) beschriebenen Verfahren, d.h. mit dem Lawesson-Reagens, das gebildete Thioamidderivat anschliessend, gegebenenfalls in situ, mit einem Alkylhalogenid, z.B. Methyljodid, in Gegenwart einer Base, z.B. Kalium-tert-butylat, in einen Alkylthioiminoäther überführt und diesen schliesslich mit einem schonenden Reduktionsmittel, z.B. Natriumcyanborhydrid, unter Abspaltung der Alkylmercapto-Verbindung umsetzt.

Die Abspaltung von $Z_1$ und $Z_2$ sowie die Einführung von $R_6$ erfolgen in der weiter vorn beschriebenen Weise.

Nitrile der Formel III können beispielsweise nach an sich bekannten Methoden hergestellt werden, indem man ein Aethylendiaminderivat der Formel V mit einem Aldehyden $R_5$-CHO und einem Cyanid-liefernden Reagens oder mit einem Cyanhydrin $R_5$-CH(OH)-CN umsetzt. Dabei kann beispielsweise das Amin der Formel V mit dem Aldehyden $R_5$-CHO unter den bei der Zweischritt-Methode in Verfahren e) und f) beschriebenen Bedingungen kondensiert und das gebildete Imin mit Trimethylsilylcyanid und Zinkiodid in der in Chemistry Letters 1975, 331, beschriebenen Weise umgesetzt werden. Andere geeignete Cyanidliefernde Reagentien sind beispielsweise Salze der Cyanwasserstoffsäure, z.B. Ammoniumcyanid, Alkalimetall- oder Erdalkalimetallcyanide, z.B. Natrium- oder Kaliumcyanid, oder Uebergangsmetallcyanide, z.B. Kupfercyanid.

In analoger Weise können Nitrile der Formel IV erhalten werden, indem geeignete Aminderivate mit Aldehyden und Cyanid-liefernden Reagentien oder mit Cyanhydriden in der oben beschriebenen Weise umgesetzt werden.

Aethylendiaminderivate der Formel V können beispielsweise durch Kondensation einer Carbonsäure der Formel $R_1$-$X_1$-$X_2$-OH mit einem Aethylendiamin der Formel $HR_2N$-$CHR_3$-$CH_2$-$NR_4$-$Z_1$, worin $Z_1$ eine

Aminoschutzgruppe bedeutet, nach Verfahren a) hergestellt werden. Ist $R_3$ Wasserstoff und $R_2$ und $R_4$ identisch, so kann die Kondensation auch mit einem Ueberschss an ungeschütztem Aethylendiamin der Formel $HR_2N-CH_2-CH_2-NR_4H$ durchgeführt werden.

α-Substituierte Carbonsäurederivate der Formel VI sind bekannt oder können, falls sie neu sind, nach an sich bekannten Methoden hergestellt werden, je nach Bedeutung von Y beispielsweise durch Halogenierung des entsprechenden unsubstituirten Carbonsäurederivats mit Phosphorpentahalogenid oder dergleichen oder durch Umsetzung des entsprechenden α-Hydroxycarbonsäurederivats mit einem den Rest Y einführenden Acylierungsmittel.

Verbindungen der Formel VII werden beispielsweise hergestellt, indem eine Carbonsäure der Formel $R_1-X_1-X_2-OH$ mit einem 2-Aminoäthanol-Derivat der Formel $HR_2N-CHR_3-CH_2OH$ nach Verfahren a) kondensiert und im Kondensationsprodukt die Hydroxygruppe nach an sich bekannten Methoden durch eine Abgangsgruppe Y ersetzt, je nach Bedeutung von Y beispielsweise mit Thionylchlorid, Phosphortrichlorid oder -bromid, Triphenylphosphindihalogenid, Methyltriphenylphosphoniumiodid oder acylierenden Reagentien, z.B. Sulfonsäurechloriden, wie Methansulfonsäurechlorid oder p-Toluolsulfonsäurechlorid, Carbonsäurechloriden, wie p-Nitrobenzoylchlorid, oder dergleichen.

α-Aminocarbonsäurederivate der Formel VIII sind bekannt oder können, wenn sie neu sind, nach an sich bekannten Methoden hergestellt werden, beispielsweise durch Kondensation der entsprechenden α-Aminocarbonsäure, worin die Aminogruppe in geschützter Form vorliegt, mit einem den Rest $R_6$ einführenden Amin nach Verfahren a), gefolgt von einer Abspaltung der Aminoschutzgruppe.

α-Oxocarbonsäurederivate der Formel IX sind bekannt oder können, falls sie neu sind, nach an sich bekannten Methoden hergestellt werden, beispielsweise durch Oxidation der entsprechenden α-Amino-

carbonsäurederivate der Formel VIII, z.B. mit Mangandioxid, Kaliumpermanganat oder tert-Butylhypochlorit in Gegenwart von Base, oder durch Kondensation einer α-Oxocarbonsäure der Formel $R_5$-(C=O)-COOH mit einem den Rest $R_6$ einführenden Amin nach Verfahren a).

Substituierte α-Aminoaldehyde der Formel X werden nach an sich bekannten Methoden hergestellt, beispielsweise durch Reduktion der entsprechenden nach Verfahren a) erhältlichen substituierten α-Aminocarbonsäuren oder -carbonsäureester, z.B. mit Diisobutylaluminiumhydrid, oder durch Oxidation der entsprechenden substituierten 2-Aminoäthanolderivate, die oben als Ausgangsstoffe für Verbindungen der Formel VII beschrieben sind, z.B. mit Pyridiniumchlorochromat oder mit Dimethylsulfoxid und Carbodiimid oder Oxalylchlorid in Gegenwart einer milden Base.

Die folgenden Beispiele dienen zur Illustration der Erfindung, Temperaturen werden in Celsiusgraden angegeben.

Die $R_f$-Werte werden, wenn nicht anders angegeben, auf Kieselgeldünnschichtplatten in folgenden Lösungsmittelsystemen ermittelt:

| | | |
|---|---|---|
| A | Chloroform-Methanol | 9:1 |
| B | Chloroform-Methanol-Ammoniak konz. | 40:10:1 |
| C | Chloroform-Methanol-Ammoniak konz. | 350:50:1 |
| D | Chloroform-Methanol-Eisessig-Wasser | 80:20:3:3 |
| E | Chloroform-Methanol-Eisessig-Wasser | 150:54:1:10 |
| F | Chloroform-Methanol | 95:5 |
| G | Essigester-Hexan | 1:1 |
| H | Essigester-Hexan | 1:4 |
| I | Essigester-Hexan | 1:9 |
| J | Methylenchlorid-Aether | 4:1 |
| K | Methylenchlorid-Methanol-Ammoniak konz. | 350:50:1 |
| L | Methylenchlorid-Methanol-Ammoniak konz. | 40:10:1 |
| M | Methylenchlorid-Methanol | 19:1 |
| N | Methylenchlorid-Aether | 1:1 |

| | | |
|---|---|---|
| O | Methylenchlorid-Methanol-Ammoniak konz. | 90:10:1 |
| P | Methylenchlorid-Methanol-Ammoniak konz. | 280:20:1 |
| Q | Methylenchlorid-Methanol-Ammoniak konz. | 130:20:1 |
| R | Methylenchlorid-Methanol | 10:1 |
| S | Methylenchlorid-Methanol | 5:1 |
| T | Methylenchlorid-Methanol-Ammoniak konz. | 65:10:1 |
| U | Methylenchlorid-Methanol-Ammoniak konz. | 140:10:1 |
| V | Methylenchlorid-Methanol-Ammoniak konz. | 80:10:1 |
| W | Methylenchlorid-Methanol-Ammoniak konz. | 1000:50:1 |
| X | Methylenchlorid-Methanol | 9:1 |
| Y | Essigester-Pyridin-Eisessig-Wasser | 62:21:6:11 |
| Z | Essigester-Pyridin-Wasser | 65:20:15 |
| $Z_1$ | Essigester-Pyridin-Eisessig-Wasser | 50:30:10:10 |
| $Z_2$ | n-Butanol-Pyridin-Eisessig-Wasser | 38:20:6:24 |
| $Z_3$ | Chloroform-Methanol-Eisessig-Wasser | 140:80:1:20 |
| $Z_4$ | Essigester-Methanol-Eisessig-Wasser | 67:12:10:23 |
| $Z_5$ | n-Butanol-Pyridin-Eisessig-Wasser | 38:24:8:30 |
| $Z_6$ | Chloroform-Methanol-Wasser | 70:30:5 |

Beispielsweise bedeutet die Abkürzung "$R_f$ (A)", dass der $R_f$-Wert im System A ermittelt wurde. Das Mengenverhältnis der Lösungsmittel zueinander ist in Volumenanteilen angegeben.

Die gleichen Abkürzungen werden für die Bezeichnung der Fliessmittel-Systeme bei der Flash-Chromatographie und der Mitteldruckchromatographie verwendet.

Abkürzungen für Aminosäuren und Aminosäurederivate:

| | |
|---|---|
| H-Ala-OH | L-Alanin |
| H-Gly-OH | Glycin |
| H-Gly(R)-OH | Glycin, das am α-Kohlenstoffatom durch den Rest R substituiert ist |
| H-His-OH | L-Histidin |
| H-($N^{\alpha}$-methyl-His)-OH | $N^{\alpha}$-Methyl-L-Histidin |

| | |
|---|---|
| H-Ile-OH | L-Isoleucin |
| H-Leu-OH | L-Leucin |
| H-(N-Methyl-Leu)-OH | N-Methyl-L-Leucin |
| H-Lys-OH | L-Lysin |
| H-Lys(R)-OH | L-Lysin, worin die Amino-Funktion der Seitenkette mit dem Rest R substituiert ist |
| H-Phe-OH | L-Phenylalanin |
| H-Sar-OH | N-Methyl-Glycin, Sarkosin |
| H-Sta-OH | Statin, (3S,4S)-4-Amino-3-hydroxy-6-methylheptansäure |
| H-Val-OH | L-Valin |
| $-NH_2$ statt -OH: | C-terminales (Carbonsäure)Amid |
| $-OCH_3$ statt -OH: | C-terminaler (Carbonsäure)Methylester |
| L-Thioleucin: | L-Leucin, in welchem die C=O-Gruppe der Carbonsäure durch C=S ersetzt ist. |

Das Fragment mit der Bezeichnung $-Leu\overset{red}{\text{——}}Val$ bedeutet das zweiwertige Radikal von [2(S)-Amino-4-methyl-pentyl]-2(S)-amino-3-methyl-buttersäure, d.h. von 2(S)-Amino-4-methyl-pentyl-L-Valin und hat die Formel

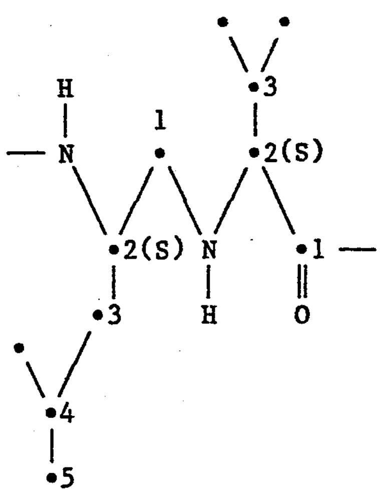

Analog bedeutet das Fragment mit der Bezeichnung

| | |
|---|---|
| -Leu$\overset{red}{\text{———}}$Ser- | [2(S)-Amino-4-methyl-pentyl]-2(S)-amino-3-hydroxy-propionyl, |
| -Phe$\overset{red}{\text{———}}$Phe- | [2(S)-Amino-3-phenyl-propyl]-2(S)-amino-3-phenyl-propionyl. |

Weitere Abkürzungen

| | |
|---|---|
| Ac | = Acetyl |
| BOC | = tert-Butoxycarbonyl |
| DC | = Dünnschichtchromatographie, wenn nicht anders angegeben auf Kieselgel |
| DCCI | = Dicyclohexylcarbodiimid |
| DCH | = Dicyclohexylharnstoff |
| DMF | = Dimethylformamid |
| DMSO | = Dimethylsulfoxid |
| HOBt | = 1-Hydroxybenzotriazol |
| Min. | = Minute(n) |
| Sdp. | = Siedepunkt |
| Smp. | = Schmelzpunkt |
| Std. | = Stunde(n) |
| THF | = Tetrahydrofuran |
| Vol. | = Volumen(anteile) |
| Z | = Benzyloxycarbonyl |

Beispiel 1:

Z-Phe-His-Leu$\overset{red}{\text{———}}$Val-7-tert-butoxycarbonyl-heptylamid

218 mg Z-Phe-His-OH (Herstellung siehe G.Lossen, G. Müller, Chem. Ber. 94, 2768 (1961)), 207 mg H-Leu$\overset{red}{\text{———}}$Val-7-tert-butoxycarbonyl-heptylamid und 77 mg HOBt (Fluka purum, enthält 11-13% Wasser) werden in 8 ml DMF gelöst und unter Rühren auf 0° abgekühlt. Es werden 134 mg DCCI zugegeben. Die Reaktionslösung wird anschliessend 1 Std. lang bei 0° und 2 Std. bei Raumtemperatur gerührt. Man

filtriert den gebildeten DCH ab, entfernt das Lösungsmittel des Filtrats im Hochvakuum und löst den Rückstand in 10 ml eines Methanol/Wasser/Eisessig-Gemisches (94:3:3). Die Lösung wird während 60 Min. bei 60° gerührt, eingedampft und der Rückstand in Essigester aufgenommen. DCH wird erneut abfiltriert, das Filtrat mit Essigester weiter verdünnt, 2 mal mit gesättigter, wässriger Natriumbicarbonatlösung extrahiert, die organischen Phasen vereinigt, über Natriumsulfat getrocknet und eingedampft. Der Rückstand wird anschliessend im System F an 140 g Kieselgel chromatographiert. Die produkthaltigen Fraktionen werden vereinigt und eingedampft. Nach Trocknen im Hochvakuum wird die Titelverbindung als farbloses Oel erhalten. $R_f(A) = 0{,}2$, $R_f(C) = 0{,}50$ und $R_f(E) = 0{,}67$.

Herstellung des Ausgangsmaterials:

a) 8-Benzyloxycarbonylaminooctansäure

Zu 7 g (44 mMol) 8-Aminooctansäure werden 25 ml 2N NaOH-Lösung gegeben. Anschliessend gibt man gleichzeitig bei ca. 0°-5° 30 Min. lang 17,1 g (50 mMol) Chlorameisensäurebenzylester (50% in Toluol) und 12,5 ml (50 mMol) 4N NaOH-Lösung zu. Man beobachtet einen voluminösen weissen Niederschlag. Das Reaktionsgemisch wird mit 150 ml Aether und 60 ml Wasser versetzt. Die wässrige Phase wird mit Aether extrahiert, mit Eis versetzt und unter Rühren langsam mit verdünnter wässriger HCl-Lösung auf pH 2 gebracht. Die entstandene Suspension wird mit Essigester zweimal extrahiert. Man vereinigt die organischen Phasen, wäscht diese mit Wasser und gesättigter, wässriger Kochsalzlösung, trocknet über Natriumsulfat und dampft im Vakuum ein. Man erhält die Titelverbindung a) mit einem Schmelzpunkt von 63°-64°. $R_f(A) = 0{,}53$.

b) 8-Benzyloxycarbonylaminooctansäure-tert-butylester

In einem Autoklaven werden 2 g 8-Benzyloxycarbonylaminooctansäure in 12 ml Dioxan in Gegenwart von 1,2 ml Schwefelsäure mit 7,5 g Isobutylen umgesetzt und 48 Std. bei Raumtemperatur stehengelassen. Die Reaktionsmischung wird mit Eis und 40 ml 1,8 N wässriger

Ammoniaklösung versetzt. Das Reaktionsgemisch wird mit Aether nochmals extrahiert. Die organische Phase wird mit Wasser und gesättigter, wässriger Kochsalzlösung gewaschen, über Natriumsulfat getrocknet und im Vakuum eingeengt. Man erhält die Titelverbindung b) als gelbes, klares Oel. $R_f(X) = 0,44$.

c) <u>8-Aminooctansäure-tert-butylester</u>

1,5 g 8-Benzyloxycarbonylaminooctansäure-tert-butylester werden in 1,5 ml Methanol gelöst und nach Zugabe von 0,1 g Pd-Kohle (10% Pd) bis zur Sättigung hydriert. Nach 4 Stunden Reaktionszeit bei 21° ist die theoretische Menge Wasserstoff aufgenommen. Das Reaktionsgemisch wird filtriert und der Rückstand mit Methanol gewaschen. Man dampft das Filtrat im Vakuum ein und erhält die Titelverbindung c) als Oel. $R_f(W) = 0,08$.

d) <u>Z-Leu$\overset{red}{—}$Val-7-tert-butoxycarbonyl-heptylamid</u>

807 mg (2,30 mMol) Z-Leu$\overset{red}{—}$Val-OH, 496 mg (2,30 mMol) 8-Amino octansäure-tert-butylester und 352 mg (2,30 mMol) HOBt werden in 25 ml DMF gelöst und unter Rühren bei 0° mit 618 mg (3,0 mMol) DCCI versetzt. Man rührt das Gemisch 2 Std. lang bei 0° und anschliessend über Nacht bei Raumtemperatur. Der gebildete DCH wird abfiltriert und das Filtrat eingedampft. Man rührt anschliessend den Rückstand in 30 ml eines Methanol/Wasser/Essigsäure-Gemisches (94:3:3), dampft ein und nimmt den Rückstand in etwas Essigester auf. Der nochmals gebildete DCH wird abgetrennt und nach erneutem Eindampfen der Rückstand durch Flash-Chromatographie an Kieselgel (Lösungsmittel: Essigester/Hexan 1:1) gereinigt. Die produkthaltigen Fraktionen werden vereinigt, eingedampft und im Hochvakuum getrocknet. Man erhält die Titelverbindung d) als gelbliches Oel. $R_f(F) = 0,57$.

e) H-Leu$\overset{red}{—}$Val-7-tert-butoxycarbonyl-heptylamid

274 mg Z-Leu$\overset{red}{—}$Val-7-tert-butoxycarbonyl-heptylamid werden in 8 ml 90%igem Methanol gelöst und nach Zugabe von 50 mg Pd-Kohle (10% Pd) unter $CO_2$-Absorption bis zur Sättigung hydriert. Der Katalysator wird abfiltriert und das Filtrat eingeengt. Der Rückstand wird mehrmals in Toluol aufgenommen, filtriert und wieder eingeengt. Nach Trocknen des Rückstands im Hochvakuum erhält man die Titelverbindung e) als farbloses Oel. $R_f(E) = 0{,}48$.

Herstellung von Z-Leu$\overset{red}{—}$Val-OH:

f) Z-Leu-Val-tert-butylester

183,8 g Benzyloxycarbonyl-L-Leucin (Z-Leu-OH) werden in 1 l absolutem Methylenchlorid gelöst, bei Raumtemperatur mit 96,1 ml Triäthylamin versetzt und anschliessend portionenweise mit 185,3 g Phenyl-N-Phenylphosphoramidochloridat (PPAC - siehe R. Mestres, C. Palomo, Synthesis 1982, 288) innerhalb von 10 Min. bei Raumtemperatur behandelt. Man rührt 15 Min. nach und tropft anschliessend ein Gemisch von 120 g L-Valin-tert-butylester und 70,1 g Triäthylamin in 200 ml Methylenchlorid unter starkem Rühren innerhalb von 30 Min. bei Raumtemperatur zu. Nach 4 Std. Rühren bei Raumtemperatur wird das Reaktionsgemisch zweimal mit je 500 ml Wasser gewaschen, die Wasserphasen einmal mit 800 ml Methylenchlorid extrahiert und die vereinigten organischen Phasen eingedampft. Man erhält ein farbloses Oel, welches an 2,5 kg Kieselgel mittels Flash-Chromatographie vorgereinigt wird. Es wird mit Hexan/Essigester 5:1 eluiert. Die produkthaltigen Fraktionen werden eingeengt und anschliessend nochmals an 3,5 kg Kieselgel chromatographiert. Die gesammelten DC-reinen Fraktionen ergeben die Titelverbindung als amorphen Festkörper. $R_f(N) = 0{,}26$.

g) Z-L-Thioleucin-Val-tert-butylester

280 g Z-Leu-Val-tert-butylester und 269,4 g Lawesson-Reagens (siehe S.O. Lawesson et al., Nouv.J.Chim. 4, 43 (1980)) werden in 2,8 l absolutem Tetrahydrofuran zum Sieden erhitzt, wobei nach 10 Min.

Sieden unter Rückfluss eine klare gelbliche Lösung entsteht. Nach weiteren 2 Std. Erwärmen wird das Reaktionsgemisch eingedampft und der Rückstand an einer kombinierten Flash-Chromatographiesäule (unten: 2 kg Kieselgel; oben: 1 kg neutrales Alox, Aktivität 4) mit Hexan/ Essigester 10:1 als Elutionsmittel vorgereinigt. Die produkthaltigen Fraktionen (je ca. 600 ml) werden eingeengt. Man erhält ein gelbliches Oel. Dieses Rohprodukt wird in drei gleichen Portionen nochmals an je 3,5 kg Kieselgel wie oben beschrieben durch Flashchromatographie gereinigt. Die DC-reinen Fraktionen (je 600 ml) werden zusammengenommen und eingedampft. Man erhält die Titelverbindung g) als gelbliches Oel. $R_f(H) = 0{,}33$.

h) Z-Leu$\overset{red}{—}$Val-tert-butylester

270 g Z-L-Thioleucin-Val-tert-butylester werden in 1,4 l absolutem Tetrahydrofuran gelöst und mit 42,8 ml Methyljodid versetzt. Die Lösung wird unter Stickstoffatmosphäre auf 0-3° gehalten. Unter starkem Rühren wird eine Lösung von 75,6 g Kalium-tert-butylat in 700 ml absolutem Tetrahydrofuran innerhalb von 15 Min. zugetropft, 30 Min. bei 0-5° nachgerührt und anschliessend das Reaktionsgemisch auf 1/4 des Volumens eingeengt. Die Suspension wird auf 1 l Eiswasser gegossen, zweimal mit je 1 l Methylenchlorid extrahiert, die organische Phase 1 mal mit 1 l gesättigter, wässriger Natriumchlorid-Lösung gewaschen und vollständig eingedampft. Nach 30 Min. Trocknen erhält man ein gelbliches Oel. Dieses Oel wird in 1,5 l Aethanol gelöst, mit 2 Tropfen Bromkresolgrün versetzt und die Lösung auf 10-15° gekühlt. Nach Zugabe von 78,5 g Natriumcyanoborhydrid ändert sich die Farbe der Lösung und wird blau. Nun wird bei 15-20° soviel (ca. 200 ml) einer 5N äthanolischen HCl-Lösung zugetropft, dass die Farbe der Reaktionslösung während mindestens einer Stunde gelb gefärbt bleibt. Anschliessend wird die Reaktionslösung vollständig eingedampft und der Rückstand zwischen 10%iger, wässriger Bicarbonatlösung und Essigester verteilt. Die organischen Phasen werden je einmal mit Wasser und gesättigter, wässriger Natriumchlorid-Lösung gewaschen, über Natriumsulfat getrocknet und vollständig eingedampft. Man erhält ein gelbliches Oel. Dieses

Rohprodukt wird durch Flash-Chromatographie an 2,5 kg Kieselgel mit Hexan/Essigester 6:1 als Elutionsmittel gereinigt. Nach Einengen der Produktfraktionen und Trocknen erhält man die Titelverbindung als hellgelbliches Oel. $R_f$(H) = 0,27.

i) Z-Leu$\overset{\text{red}}{—}$Val-OH

44 g Z-Leu$\overset{\text{red}}{—}$Val-tert-butylester werden bei Raumtemperatur in 600 ml mit HCl-Gas gesättigtem Eisessig gelöst und während 2 1/2 Stunden bei Raumtemperatur weitergerührt. Danach wird das Reaktionsgemisch vollständig eingedampft und der Rückstand mit 300 ml Wasser verrührt. Die Suspension wird mit 1N NaOH-Lösung auf pH 5,5 gebracht und 2 Std. unter Eiskühlung weitergerührt. Anschliessend wird der flockige, amorphe Niederschlag abgetrennt, dreimal mit je 100 ml Eiswasser gewaschen, getrocknet und aus 600 ml Isopropanol umkristallisiert. Nach Trocknen erhält man die Titelverbindung mit einem Schmelzpunkt von 170-171°. $R_f$(B) = 0,34.

Beispiel 2:

Z-Phe-His-Leu$\overset{\text{red}}{—}$Val-7-carboxyheptylamid

255 mg Z-Phe-His-Leu$\overset{\text{red}}{—}$Val-7-tert-butoxycarbonyl-heptylamid (Beispiel 1) werden in 10 ml mit HCl-Gas gesättigter Essigsäure gelöst. Die Lösung wird 30 Min. lang bei Raumtemperatur gerührt. Anschliessend wird das Lösungsmittel im Rotationsverdampfer entfernt und der Rückstand 4 mal in Toluol aufgenommen, wobei man das Lösungsmittel jeweils wieder im Rotationsverdampfer entfernt. Das Rohprodukt wird anschliessend an 150 g Kieselgel im System B durch Flash-Chromatographie gereinigt. Die produkthaltigen Fraktionen werden vereinigt und eingedampft. Der Rückstand wird in wenig Methanol aufgenommen, filtriert und das Filtrat erneut eingedampft. Nach Trocknen im Hochvakuum erhält man die Titelverbindung als gelblichen Schaum. $R_f$(D) = 0,28; $R_f$(E) = 0,56.

Beispiel 3:

Z-Phe-His-Leu-red-Val-3-tert-butoxycarbonyl-benzylamid

Analog Beispiel 1 wird durch Umsetzung von 218 mg Z-Phe-His-OH, 243 mg H-Leu-red-Val-3-tert-butoxycarbonyl-benzylamid und 77 mg HOBt in 8 ml DMF mit 134 mg DCCI und Aufarbeitung analog Beispiel 1 die Titelverbindung hergestellt. $R_f(A) = 0,37$; $R_f(E) = 0,71$.

Herstellung des Ausgangsmaterials:

a) 3-Benzyloxycarbonylaminomethylbenzoesäure

wird aus 17 g 3-Aminomethylbenzoesäure und 43,90 g Chlorameisensäurebenzylester analog Beispiel 1a) hergestellt und aufgearbeitet. Man erhält die Titelverbindung a) mit einem Schmelzpunkt von 135-136° und $R_f(A) = 0,24$.

b) 3-Benzyloxycarbonylaminomethylbenzoesäure-tert-butylester

wird aus 2 g 3-Benzyloxycarbonylaminomethylbenzoesäure und 8,9 g (14 ml) Isobutylen in Gegenwart von 1,4 ml Schwefelsäure in 14 ml Dioxan analog Beispiel 1b) hergestellt und aufgearbeitet. Man erhält die Titelverbindung als Oel. $R_f(X) = 0,47$.

c) 3-Aminomethylbenzoesäure-tert-butylester

wird durch Hydrierung von 1,5 g 3-Benzyloxycarbonylaminomethylbenzoesäure-tert-butylester in Gegenwart von 0,1 g Pd-Kohle (10% Pd) in 15 ml Methanol analog Beispiel 1c) erhalten. $R_f(W) = 0,43$.

d) Z-Leu-red-Val-3-tert-butoxycarbonyl-benzylamid

wird aus 1,051 g (3,0 mMol) Z-Leu-red-Val-OH, 0,746 g (3,6 mMol) 3-Aminomethylbenzoesäure-tert-butylester, 0,551 g (3,6 mMol) HOBT und 0,803 g (3,9 mMol) DCCI in 25 ml DMF analog Beispiel 1d) hergestellt und aufgearbeitet. $R_f(V) = 0,43$.

e) H-Leu$\overset{red}{—}$Val-3-tert-butoxycarbonyl-benzylamid
wird durch Hydrierung von 540 mg Z-Leu$\overset{red}{—}$Val-3-tert-butoxycarbonyl-benzylamid mit 100 mg Pd-Kohle (10% Pd) in 10 ml Methanol analog Beispiel 1e) erhalten. $R_f(E) = 0,45$.

Beispiel 4:

Z-Phe-His-Leu$\overset{red}{—}$Val-3-carboxy-benzylamid
Analog Beispiel 2 wird durch Umsetzung von 148 g Z-Phe-His-Leu$\overset{red}{—}$-Val-3-tert-butoxycarbonyl-benzylamid mit 6 ml mit HCl-Gas gesättigter Essigsäure und chromatographischer Trennung im System B die Titelverbindung erhalten. $R_f(E) = 0,59$.

Beispiel 5:

Z-Phe-His-Leu$\overset{red}{—}$Val-Gly-Sar-n-hexylamid
Analog Beispiel 1 wird durch Umsetzung von 361 mg (0,828 mMol) Z-Phe-His-OH, 360 mg (0,828 mMol) H-Leu$\overset{red}{—}$Val-Gly-Sar-n-hexylamid und 127 mg HOBt in 12 ml DMF mit 222 mg (1,076 mMol) DCCI und Aufarbeitung analog Beispiel 1 die Titelverbindung hergestellt. $R_f(A) = 0,12$; $R_f(E) = 0,63$.

Herstellung des Ausgangsmaterials:

a) Sarcosin-n-hexylamid
1,54 g Sarcosinäthylester-hydrochlorid werden mit 10 ml n-Hexylamin versetzt. Man lässt das Gemisch 8 Std. bei Raumtemperatur stehen, dampft ein und reinigt das Produkt mit Flash-Chromatographie (Laufmittel: Chloroform/Methanol/Ammoniak konz. 80:10:10). $R_f(B) = 0,67$.

b) Z-Leu$\overset{red}{\text{—}}$Val-Gly-äthylester

Analog Beispiel 1d) wird durch Umsetzung von 2,10 mg (6,0 mMol) Z-Leu$\overset{red}{\text{—}}$Val-OH, 0,92 g (6,0 mMol) Glycinäthylester-hydrochlorid, 0,92 g (6,0 mMol) HOBt, 0,67 g (6,6 mMol) N-Methylmorpholin als Base und 1,61 g (7,8 mM) DCCI und üblicher Aufarbeitung die Titelverbindung b) erhalten. $R_f(F) = 0,54$.

c) Z-Leu$\overset{red}{\text{—}}$Val-Gly-OH

1,50 g (3,44 mMol) Z-Leu$\overset{red}{\text{—}}$Val-Gly-äthylester werden in 30 ml Methanol und 30 ml Wasser mit 3,45 ml 1N NaOH-Lösung verseift. Anschliessend wird das Gemisch mit 3,45 ml 1N HCl-Lösung versetzt, filtriert, mit Wasser gewaschen, mit Aether und Essigester extrahiert und eingedampft. $R_f(E) = 0,31$.

d) Z-Leu$\overset{red}{\text{—}}$Val-Gly-Sar-n-hexylamid

Analog Beispiel 1d) wird durch Umsetzung von 408 mg (1,0 mMol) Z-Leu$\overset{red}{\text{—}}$Val-Gly-OH, 207 mg (1,2 mMol) Sarcosin-n-hexylamid und 153 mg (1,0 mMol) HOBt in 10 ml DMF mit 258 mg DCCI (1,25 mMol) und üblicher Aufarbeitung die Titelverbindung d) erhalten. $R_f(F) = 0,19$.

e) H-Leu$\overset{red}{\text{—}}$Val-Gly-Sar-n-hexylamid

Analog Beispiel 1e) wird durch Hydrierung von 465 mg Z-Leu$\overset{red}{\text{—}}$Val-Gly-Sar-n-hexylamid mit 50 mg Pd-Kohle (10% Pd) in 10 ml Methanol die Titelverbindung e) erhalten. $R_f(E) = 0,39$; $R_f(C) = 0,09$.

Beispiel 6:

Z-Phe-His-Leu$\overset{red}{\text{—}}$Val-Gly-Gly-Gly-tert-butylester

Analog Beispiel 1 wird durch Umsetzung von 236 mg (0,54 mMol) Z-Phe-His-OH, 240 mg (0,54 mMol) H-Leu$\overset{red}{\text{—}}$Val-Gly-Gly-Gly-tert-butylester und 83 mg HOBt in 10 ml DMF mit 145 mg (0,70 mMol) DCCI und üblicher Aufarbeitung die Titelverbindung hergestellt. $R_f(E) = 0,64$; $R_f(D) = 0,28$.

Herstellung des Ausgangsmaterials:

a) Z-Gly-Gly-OH

23,3 g (176,4 mMol) H-Gly-Gly-OH werden in 100 ml 2N NaOH-Lösung gelöst. Man kühlt die Lösung auf 0° ab und gibt dazu 50 ml 4N NaOH-Lösung und 68,8 g (260,4 mMol) Chlorameisensäurebenzylester in 67 ml Toluol. Man rührt 15 Minuten bei 0-5° und extrahiert das Gemisch mit Aether, versetzt die produkthaltige, wässrige Phase mit Eis und konzentrierter HCl-Lösung bis ca. pH 2. Man filtriert die erhältliche weisse Suspension und kristallisiert den Feststoff aus Wasser um. Nach Trocknen erhält man die Titelverbindung a) mit einem Schmelzpunkt von 178,5°-179,5°. $R_f(U) = 0{,}15$.

b) Z-Gly-Gly-tert-butylester

Analog Beispiel 1 b) wird durch Umsetzung von 79,7 g Z-Gly-Gly-OH mit 378,8 g (601,2 mMol) Isobutylen in Gegenwart von 58,2 ml Schwefelsäure in 500 ml Dioxan die Titelverbindung b) hergestellt. $R_f(G) = 0{,}14$.

c) H-Gly-Gly-tert-butylester

Analog Beispiel 1c) wird durch Hydrierung von 3,78 g Z-Gly-Gly-tert-butylester in Gegenwart von 0,4 g Pd-Kohle (10% Pd) in 50 ml Methanol die Titelverbindung c) erhalten. $R_f(E) = 0{,}18$.

d) Z-Leu$\overset{red}{—}$Val-Gly-Gly-Gly-tert-butylester

Analog Beispiel 1d) wird durch Umsetzung von 945 mg (2,32 mMol) Z-Leu$\overset{red}{—}$Val-Gly-OH (Herstellung siehe Beispiel 5c)), 480 mg (2,55 mMol) H-Gly-Gly-tert-butylester und 355 mg HOBt in 25 ml DMF und 621 mg DCCI (3,02 mMol) und üblicher Aufarbeitung die Titelverbindung d) erhalten. $R_f(F) = 0{,}1$.

e) H-Leu$\overset{red}{—}$Val-Gly-Gly-Gly-tert-butylester

Analog Beispiel 1 e) wird durch Hydrierung von 346 mg Z-Leu$\overset{red}{—}$Val-Gly-Gly-Gly-tert-butylester in Gegenwart von 50 mg Pd-Kohle (10% Pd) in 10 ml Methanol die Titelverbindung e) erhalten. $R_f$(B) = 0,33.

Beispiel 7:

Z-Phe-His-Leu$\overset{red}{—}$Val-Gly-Gly-6-tert-butoxycarbonylamino-hexylamid

197 mg (0,45 mMol) Z-Phe-His-OH, 240 mg (0,45 mMol) H-Leu$\overset{red}{—}$Val-Gly-Gly-6-tert-butoxycarboylamino-hexylamid und 69 mg (0,45 mMol ) HOBt in 8 ml DMF werden analog Beispiel 1 mit 124 mg (0,60 mMol) DCCI umgesetzt. Nach üblicher Aufarbeitung des Reaktionsgemisches erhält man die Titelverbindung. $R_f$(B) = 0,19.

Herstellung des Ausgangsmaterials:

a) Z-Gly-6-tert-butoxycarbonylamino-hexylamid

Analog Beispiel 1d) wird durch Umsetzung von 2,09 g (10 mMol) Z-Gly-OH, 2,53 g (10 mMol) 6-tert-Butoxyarbonylamino-n-hexylamin und 1,53 g (10 mMol) HOBt in 60 ml DMF mit 1,01 g (10 mMol) N-Methylmorpholin und 2,68 g (13 mMol) DCCI und üblicher Aufarbeitung die Titelverbindung a) erhalten. $R_f$(T) = 0,26.

b) Glycin-6-tert-butoxycarbonylamino-hexylamid

Analog Beispiel 1e) wird durch Hydrierung von 1,22 g Z-Gly-6-tert-butoxycarbonylamino-hexylamid in Gegenwart von 150 mg Pd-Kohle (10% Pd) in 20 ml 90%igem Methanol unter $CO_2$-Absorption die Titelverbindung b) hergestellt. $R_f$(E) = 0,22.

c) Z-Leu$\overset{red}{\text{—}}$Val-Gly-Gly-6-tert-butoxycarbonylamino-hexylamid

Analog Beispiel 1d) wird durch Umsetzung von 408 mg (1,0 mMol) Z-Leu$\overset{red}{\text{—}}$Val-Gly-OH (Herstellung siehe Beispiel 5c)), 355 mg Glycin-6-tert-butoxycarbonylamino-hexylamid und 153 mg HOBt in 10 ml DMF und 268 mg (1,3 mMol) DCCI und üblicher Aufarbeitung die Titelverbindung erhalten. $R_f(F) = 0,10$.

d) H-Leu$\overset{red}{\text{—}}$Val-Gly-Gly-6-tert-butoxycarbonylamino-hexylamid

Analog Beispiel 1e) wird durch Hydrierung von 300 mg Z-Leu$\overset{red}{\text{—}}$Val-Gly-Gly-6-tert-butoxycarbonylamino-hexylamid in Gegenwart von 50 mg Pd-Kohle (10% Pd) die Titelverbindung d) hergestellt und roh weiterverarbeitet.

Beispiel 8:

Z-Phe-His-Leu$\overset{red}{\text{—}}$Val-Gly-Gly-6-aminohexylamid

21 mg Z-Phe-His-Leu$\overset{red}{\text{—}}$Val-Gly-Gly-6-tert-butoxycarbonylamino-hexylamid werden mit 2 ml Trifluoressigsäure versetzt und 5 Min. bei Raumtemperatur stehengelassen. Anschliessend wird das Lösungsmittel abgedampft und der Rückstand durch Flash-Chromatographie im System B gereinigt. Die produkthaltigen Fraktionen werden vereinigt und eingedampft, der Rückstand in wenig Methylenchlorid gelöst, filtriert und das Filtrat erneut eingedampft. Nach Trocknen im Hochvakuum erhält man die Titelverbindung als farblosen Schaum. $R_f(B) = 0,19$.

Beispiel 9:

Z-Phe-His-Leu$\overset{red}{\text{—}}$Val-Gly-His-Lys-(BOC)-$OCH_3$

181 mg (0,277 mMol) Z-Phe-His-OH, 127 mg (0,291 mMol) H-Leu$\overset{red}{\text{—}}$Val-Gly-His-Lys-(BOC)-$OCH_3$ und 45 mg (0,291 mMol) HOBt in 5 ml DMF werden analog Beispiel 1 mit 75 mg (0,360 mMol) DCCI versetzt. Nach üblicher Aufarbeitung des Reaktionsgemisches erhält man die Titelverbindung. $R_f(C) = 0,10$.

Herstellung des Ausgangsmaterials:

a) H-His-Lys-(BOC)-$OCH_3$

1,6 g Z-His-Lys(BOC)-$OCH_3$ (S. Guttman et al., Helv.Chim.Acta 52, 1789 (1969)) werden in 33 ml Methanol gelöst, mit 3 ml 1N HCl und 160 mg Pd-Kohle (10% Pd) versetzt und unter $CO_2$-Absorption bis zur Sättigung hydriert. Nach Abfiltrieren des Katalysators wird eingeengt, der ölige Rückstand in 20 ml DMF gelöst und erneut bis auf ca. 10 ml eingeengt, worauf man H-His-Lys-(BOC)-$OCH_3$ in Form einer Lösung erhält, die sofort weiterverarbeitet wird.

b) Z-Leu$\overset{red}{—}$Val-Gly-His-Lys(BOC)-$OCH_3$

Analog Beispiel 1d) wird durch Umsetzung von 552 mg (1,355 mMol) Z-Leu$\overset{red}{—}$Val-Gly-OH (Herstellung siehe Beispiel 5c)), 651 mg (1,50 mMol) H-His-Lys-(BOC)-$OCH_3$ und 207 mg (1,355 mMol) HOBt in 15 ml DMF mit 152 mg (1,50 mMol) N-Methylmorpholin und 363 mg (1,76 mMol) DCCI und üblicher Aufarbeitung die Titelverbindung erhalten.

c) H-Leu$\overset{red}{—}$Val-Gly-His-Lys-(BOC)-$OCH_3$

Analog Beispiel 1e) wird durch Hydrierung von 393 mg Z-Leu$\overset{red}{—}$Val-Gly-His-Lys-(BOC)-$OCH_3$ mit 50 mg Pd-Kohle (10% Pd) als Katalysator in 10 ml 90%igem Methanol unter $CO_2$-Absorption die Titelverbindung c) hergestellt. $R_f$(B) = 0,24.

Beispiel 10:

Z-Phe-His-Leu$\overset{red}{—}$Val-Ile-His-$NH_2$

Ausgehend von 437 mg Z-Phe-His-OH, 466 mg H-Leu$\overset{red}{—}$Val-Ile-His-$NH_2$, 154 mg HOBt in 16 ml DMF und 268 mg DCCI wird analog Beispiel 1 die Titelverbindung erhalten. $R_f$(C) = 0,46; $R_f$(E) = 0,31.

Herstellung des Ausgangsmaterials:

a) Z-Leu$\overset{red}{\text{———}}$Val-Ile-His-$NH_2$

3,93 g Z-Leu$\overset{red}{\text{———}}$Val-OH, 3,0 g H-Ile-His-$NH_2$ und 1,72 g HOBt werden in 90 ml DMF gelöst und unter Rühren auf 0° gekühlt. Es werden 3,0 g DCCI zugegeben. Die Reaktionslösung wird anschliessend während 1 Std. bei 0° und während 68 Std. bei Raumtemperatur gerührt. Man filtriert den gebildeten DCH ab, entfernt das Lösungsmittel des Filtrats und löst den Rückstand in 220 ml Methanol/Wasser/Eisessig-Gemisch (94:3:3). Die Lösung wird während 60 Min. bei 60° unter Inertgas ($N_2$) gerührt und eingeengt. Der Kristalle enthaltende, ölige Rückstand wird anschliessend durch Flash-Chromatographie an 600 g Kieselgel im System Q gereinigt. Die DC-reinen Produktfraktionen werden vereinigt, vollständig eingedampft und der farblose Schaum über Nacht im Hochvakuum getrocknet. Man erhält die reine Titelverbindung a). $R_f$(B) = 0,45.

b) H-Leu$\overset{red}{\text{———}}$Val-Ile-His-$NH_2$

5,9 g Z-Leu$\overset{red}{\text{———}}$Val-Ile-His-$NH_2$ werden in 50 ml Methanol-Wasser (9:1) gelöst und in Gegenwart von 1 g Pd-Kohle (10% Pd) unter $CO_2$-Absorption bis zur Sättigung hydriert. Der Katalysator wird abfiltriert, das Filtrat zur Trockne eingedampft und der Rückstand durch Flash-Chromatographie an 220 g Kieselgel im System L gereinigt. Die produkthaltigen Fraktionen werden am Rotationsverdampfer eingeengt und im Hochvakuum getrocknet und ergeben dabei die farblose, pulverige Titelverbindung b). $R_f$(L) = 0,35.

c) H-Ile-His-$NH_2$

3,0 g Z-Ile-His-$OCH_3$ werden in 120 ml einer 5 N methanolischen Ammoniak-Lösung gelöst und 16 Std. lang bei Raumtemperatur gerührt. Der gebildete, farblose Niederschlag wird abfiltriert und im Hochvakuum getrocknet. Man erhält Z-Ile-His-$NH_2$. $R_f$(E) = 0,58, Smp.

204-205°. 2,5 g Z-Ile-His-$NH_2$ werden in 25 ml Methanol-Wasser (9:1) gelöst und in Gegenwart von 400 mg Pd-Kohle (10% Pd) unter $CO_2$-Absorption bis zur Sättigung hydriert. Der Katalysator wird abfiltriert, das Filtrat zur Trockne eingeengt und der Rückstand durch Flash-Chromatographie an Kieselgel im System L gereinigt. Die produkthaltigen Fraktionen ergeben nach Einengen im Rotationsverdampfer und Trocknen im Hochvakuum H-Ile-His-$NH_2$. $R_f$(E) = 0,12.

Beispiel 11:

BOC-Phe-His-Leu$\overset{red}{—}$Val-Ile-His-$NH_2$

Ausgehend von 170 mg BOC-Phe-His-OH (Herstellung siehe US-Patentschrift 4,025,499), 200 mg H-Leu$\overset{red}{—}$Val-Ile-His-$NH_2$, 64 mg HOBt und 110 mg DCCI, wird analog Beispiel 1 die Titelverbindung erhalten und durch Flash-Chromatographie im System L gereinigt. $R_f$(C) = 0,46; $R_f$(E) = 0,31.

Beispiel 12:

Z-Phe-His-Leu$\overset{red}{—}$Val-Ile-Phe-$NH_2$

Ausgehend von 180 mg Z-Phe-His-OH, 200 mg H-Leu$\overset{red}{—}$Val-Ile-Phe-$NH_2$, 64 mg HOBt und 111 mg DCCI wird analog Beispiel 1 die Titelverbindung erhalten und durch Flash-Chromatographie im System O gereinigt. $R_f$(L) = 0,66; $R_f$(O) = 0,36.

Herstellung des Ausgangsmaterials:

a) Z-Ile-Phe-$NH_2$

Eine Lösung von 28,45 g Z-Ile-Phe-$OCH_3$ (Herstellung siehe Chem. Pharm.Bull. 27, 3015 (1979)) in 600 ml Methanol, das mit Ammoniakgas gesättigt ist, wird 8 Std. lang bei Raumtemperatur gerührt. Das Produkt beginnt nach wenigen Minuten auszufallen. Nach beendeter

Reaktion wird die Suspension zur Trockne eingeengt und der Rückstand in 120 ml Methylenchlorid während 30 Min. bei Raumtemperatur aufgerührt. Nach Filtration und Trocknen des Rückstandes erhält man Z-Ile-Phe-$NH_2$ als farbloses Pulver. $R_f(M) = 0,13$; $R_f(X) = 0,48$.

b) H-Ile-Phe-$NH_2$

20,5 g Z-Ile-Phe-$NH_2$ werden in 370 ml Methanol/Wasser (95:5) in Gegenwart von 3,7 g Palladium-Kohle (10% Pd) bei Raumtemperatur 4 Std. lang unter Normaldruck hydriert. Nach Abfiltrieren des Katalysators wird das Lösungsmittel abgedampft und das so erhaltene Rohprodukt durch Mitteldruckchromatographie aufgetrennt (Lichroprep® Si 60, 25-40µm, Fraktionen zu ca. 200 ml, Laufmittel Methylenchlorid/Methanol/Wasser 500:50:1). Nach Eindampfen der produkthaltigen Fraktionen erhält man H-Ile-Phe-$NH_2$ als farbloses Pulver. $R_f(CH_2Cl_2/MeOH/H_2O\ 500:50:1) = 0,15$.

c) Z-Leu$\overset{red}{\text{———}}$Val-Ile-Phe-$NH_2$

Ausgehend von 1,38 g Z-Leu$\overset{red}{\text{———}}$Val-OH und 1,2 g H-Ile-Phe-$NH_2$ 603 mg HOBt in 32 ml DMF und 1,05 g DCCI wird analog Beispiel 10a) die Titelverbindung c) erhalten und durch Flash-Chromatographie im System Q gereinigt. $R_f(F) = 0,68$; $R_f(E) = 0,87$.

d) H-Leu$\overset{red}{\text{———}}$Val-Ile-Phe-$NH_2$

Ausgehend von 1,7 g Z-Leu$\overset{red}{\text{———}}$Val-Ile-Phe-$NH_2$ wird durch Hydrierung in Gegenwart von 200 mg Pd-Kohle (10% Pd) analog Beispiel 10b) die Titelverbindung d) erhalten und durch Flash-Chromatographie im System Q gereinigt. $R_f(Q) = 0,30$; $R_f(L) = 0,68$.

Beispiel 13:

BOC-Phe-His-Leu$\overset{red}{—}$Val-Ile-Phe-$NH_2$

Ausgehend von 170 mg BOC-Phe-His-OH, 200 mg H-Leu$\overset{red}{—}$Val-Ile-Phe-$NH_2$, 64 mg HOBt und 110 mg DCCI wird analog Beispiel 1 die Titelverbindung erhalten und durch Flash-Chromatographie im System P gereinigt. $R_f(Q) = 0{,}42$; $R_f(P) = 0{,}28$.

Beispiel 14:

Z-Phe-His-Leu$\overset{red}{—}$Val-Ile-His-$OCH_3$

Ausgehend von 136 mg Z-Phe-His-OH, 150 mg H-Leu$\overset{red}{—}$Val-Ile-His-$OCH_3$, 47 mg HOBt und 80 mg DCCI wird analog Beispiel 1 die Titelverbindung erhalten und durch Flash-Chromatographie im System P gereinigt. $R_f(Q) = 0{,}35$.

Herstellung des Ausgangsmaterials:

a) Z-Leu$\overset{red}{—}$Val-Ile-tert-butylester

7,02 g Z-Leu$\overset{red}{—}$Val-OH und 3,4 ml Aethyldiiospropylamin werden in 60 ml Methylenchlorid gelöst, bei Raumtemperatur mit 5,36 g Phenyl-N-phenylphosphoramidochloridat (Synthesis 1982, 288) versetzt und 20 Min. lang gerührt. Anschliessend wird eine Lösung aus 4,92 g L-Isoleucin-tert-butylester-hydrochlorid und 7,48 ml Aethyldiisopropylamin in 10 ml Methylenchlorid innerhalb von 10 Min. zugetropft. Nach 17 Std. Rühren wird das Reaktionsgemisch vollständig eingedampft und der Rückstand durch Filtration an einer 150 g Kieselgelsäule mit Hexan/Essigester (2:1) vorgereinigt. Die produkthaltigen Fraktionen werden eingedampft und durch Flash-Chromatographie an 230 g Kieselgel mit Hexan/Aether/Methylenchlorid (2:1:1) als Elutionsmittel weiter gereinigt. Nach Eindampfen der produkthaltigen Fraktionen erhält man die Titelverbindung. $R_f(H) = 0{,}11$; $R_f(G) = 0{,}35$.

b) Z-Leu$\overset{red}{\text{——}}$Val-Ile-OH

6,7 g Z-Leu$\overset{red}{\text{——}}$Val-Ile-tert-butylester werden in 65 ml mit HCl-Gas gesättigter Essigsäure gelöst. Die Lösung wird während 1 Std. bei Raumtemperatur gerührt. Anschliessend wird eingedampft, der Rückstand in 65 ml Eiswasser suspendiert und mit 1N Natriumhydroxid-Lösung auf pH 5,5 gebracht. Diese wässrige Suspension wird mehrmals mit je 300 ml Essigester extrahiert, die organische Phase mit 200 ml Wasser nachgewaschen und eingeengt, wobei man ein gelbliches Oel erhält. Dieses Rohprodukt wird an 240 g Kieselgel im System R chromatographiert. Nach Eindampfen der produkthaltigen Fraktionen erhält man die Titelverbindung b) als amorphen Festkörper. $R_f$(C) = 0,43; $R_f$(E) = 0,76.

c) Z-Leu$\overset{red}{\text{——}}$Val-Ile-His-$OCH_3$

Ausgehend von 695 mg Z-Leu$\overset{red}{\text{——}}$Val-Ile-OH, 400 mg L-Histidinmethylester-hydrochlorid, 230 mg HOBt, 408 mg DCCI und 0,56 ml Aethyldiisopropylamin wird analog Beispiel 1 die Titelverbindung c) erhalten und durch Flash-Chromatographie im System R gereinigt. $R_f$(R) = 0,36; $R_f$(E) = 0,80.

d) H-Leu$\overset{red}{\text{——}}$Val-Ile-His-$OCH_3$

700 mg Z-Leu$\overset{red}{\text{——}}$Val-Ile-His-$OCH_3$ werden in Gegenwart von 200 mg Pd-Kohle (10% Pd) analog Beispiel 10b) hydriert und die Titelverbindung d) nach Flash-Chromatographie im System L erhalten. $R_f$(L) = 0,53; $R_f$(K) = 0,16.

Beispiel 15:

Z-Phe-His-Leu$\overset{red}{\text{——}}$Val-Ile-Phe-$OCH_3$

Ausgehend von 360 mg Z-Phe-His-OH, 400 mg H-Leu$\overset{red}{\text{——}}$Val-Ile-Phe-$OCH_3$, 126 mg HOBt und 213 mg DCCI wird analog Beispiel 1 die Titelverbindung erhalten und durch Flash-Chromatographie im System R gereinigt. $R_f$(R) = 0,31; $R_f$(L) = 0,68.

Herstellung des Ausgangsmaterials:

a) Z-Leu$\overset{red}{—}$Val-Ile-Phe-$OCH_3$

Ausgehend von 464 mg Z-Leu$\overset{red}{—}$Val-Ile-OH (Herstellung siehe Beispiel 14b)), 237 mg L-Phenylalaninmethylester-hydrochlorid, 153 mg HOBt, 272 mg DCCI und 0,2 ml Aethyldiisopropylamin wird analog Beispiel 1 die Titelverbindung a) erhalten und durch Flash-Chromatographie im System R gereinigt. $R_f(G) = 0{,}25$; $R_f(R) = 0{,}65$.

b) H-Leu$\overset{red}{—}$Val-Ile-Phe-$OCH_3$

1,00 g Z-Leu$\overset{red}{—}$Val-Ile-Phe-$OCH_3$ werden in Gegenwart von 200 mg Pd-Kohle-Katalysator analog Beispiel 10b) hydriert und die Titelverbindung b) durch Flash-Chromatographie im System R erhalten. $R_f(E) = 0{,}50$; $R_f(L) = 0{,}83$.

Beispiel 16:

Z-Phe-His-Leu$\overset{red}{—}$Val-Ile-Phe-2-hydroxyäthylamid

Ausgehend von 168 mg Z-Phe-His-OH, 170 mg H-Leu$\overset{red}{—}$Val-Ile-Phe-2-hydroxyäthylamid, 59 mg HOBt und 100 mg DCCI wird analog Beispiel 1 die Titelverbindung erhalten und durch Flash-Chromatographie im System O gereinigt. $R_f(Q) = 0{,}43$.

Herstellung des Ausgangsmaterials:

a) H-Ile-Phe-2-hydroxyäthylamid

2,0 g Z-Ile-Phe-$OCH_3$ und 2,0 ml Aethanolamin werden in 20 ml Methanol 20 Std. lang bei 50° gerührt. Anschliessend wird die Mischung eingeengt, in 100 ml Eiswasser suspendiert und mehrmals mit Methylenchlorid extrahiert. Nach Einengen erhält man einen Feststoff. $R_f(R) = 0,45$. Dieser wird anschliessend in 400 ml Methanol/Wasser (9:1) gelöst und in Gegenwart von 500 mg Pd-Kohle (10% Pd) analog Beispiel 10b) hydriert und durch Flash-Chromatographie im System S gereinigt. $R_f(S) = 0,38$; $R_f(R) = 0,27$.

b) Z-Leu$\overset{red}{\text{——}}$Val-Ile-Phe-2-hydroxyäthylamid
Ausgehend von 350 mg Z-Leu$\overset{red}{\text{——}}$Val-OH und 353 mg H-Ile-Phe-2-hydroxyäthylamid, 153 mg HOBt und 268 mg DCCI wird analog Beispiel 1d) die Titelverbindung b) erhalten und durch Flash-Chromatographie im System R gereinigt. $R_f(R) = 0,44$.

c) H-Leu$\overset{red}{\text{——}}$Val-Ile-Phe-2-hydroxyäthylamid
400 mg Z-Leu$\overset{red}{\text{——}}$Val-Ile-Phe-2-hydroxyäthylamid werden in Gegenwart von 50 g Pd-Kohle (10% Pd)-Katalysator hydriert und die Titelverbindung c) durch Flash-Chromatographie im System K erhalten. $R_f(K) = 0,44$; $R_f(L) = 0,66$.

Beispiel 17:

Z-Phe-His-Leu$\overset{red}{\text{——}}$Val-Ile-1-benzylpiperid-4-ylamid
Ausgehend von 106 mg Z-Phe-His-OH und 110 mg H-Leu$\overset{red}{\text{——}}$Val-Ile-1-benzylpiperid-4-ylamid, 37 mg HOBt und 63 mg DCCI wird analog Beispiel 1 die Titelverbindung erhalten und durch Flash-Chromatographie im System R gereinigt. $R_f(R) = 0,25$.

Herstellung des Ausgangsmaterials:

a) Z-Leu$\overset{red}{\text{——}}$Val-Ile-1-benzylpiperid-4-ylamid
Ausgehend von 232 mg Z-Leu$\overset{red}{\text{——}}$Val-Ile-OH (Herstellung siehe Beispiel 14b)), 96 mg 4-Amino-1-benzylpiperidin, 77 mg HOBt und 134 mg DCCI wird analog Beispiel 1d) die Titelverbindung erhalten und durch Flash-Chromatographie im System R gereinigt. $R_f(R) = 0,30$.

b) H-Leu$\stackrel{red}{—}$Val-Ile-1-benzylpiperid-4-ylamid
250 mg Z-Leu$\stackrel{red}{—}$Val-Ile-(1-Benzylpiperid-4-ylamid werden in Gegenwart von 50 mg Pd-Kohle (10% Pd)-Katalysator hydriert und die Titelverbindung c) nach Flash-Chromatographie im System R erhalten. $R_f(R) = 0,30$.

Beispiel 18:

Z-Phe-His-Leu$\stackrel{red}{—}$Val-Ile-2-dimethylaminoäthylamid
Ausgehend von 117 mg Z-Phe-His-OH, 100 mg H-Leu$\stackrel{red}{—}$Val-Ile-2-dimethylaminoäthylamid, 41 mg HOBt und 69 mg DCCI wird analog Beispiel 1 die Titelverbindung erhalten und durch Flash-Chromatographie im System O gereinigt. $R_f(O) = 0,34$.

Herstellung des Ausgangsmaterials:

a) Z-Leu$\stackrel{red}{—}$Val-Ile-2-dimethylaminoäthylamid
Ausgehend von 233 mg Z-Leu$\stackrel{red}{—}$Val-Ile-OH (Herstellung siehe Beispiel 14b)), 66 µl Dimethylaminoäthylamin, 77 mg HOBt und 134 mg DCCI wird analog Beispiel 1d) die Titelverbindung erhalten und durch Flash-Chromatographie im System O gereinigt. $R_f(O) = 0,33$.

b) H-Leu$\stackrel{red}{—}$Val-Ile-2-dimethylaminoäthylamid
280 mg Z-Leu$\stackrel{red}{—}$Val-Ile-2-dimethylaminoäthylamid werden in Gegenwart von 40 mg Pd-Kohle (10% Pd)-Katalysator hydriert und die Titelverbindung b) nach Flash-Chromatographie im System O erhalten. $R_f(O) = 0,18$.

Beispiel 19:

Z-Phe-His-Leu$\stackrel{red}{—}$Val-Ile-2-dimethylaminopropylamid
Ausgehend von 152 mg Z-Phe-His-OH, 100 mg H-Leu$\stackrel{red}{—}$Val-Ile-2-dimethylaminopropylamid, 53 mg HOBt und 90 mg DCCI wird analog Beispiel 1 die Titelverbindung erhalten und durch Flash-Chromatographie im System O gereinigt. $R_f(O) = 0,24$.

Herstellung des Ausgangsmaterials:

a) Z-Leu$\overset{red}{—}$Val-Ile-2-dimethylaminopropylamid

Ausgehend von 210 mg Z-Leu$\overset{red}{—}$Val-Ile-OH (Herstellung siehe Beispiel 14b)), 74 µl Dimethylamino-n-propylamin, 69 mg HOBt und 117 mg DCCI wird analog Beispiel 1d) die Titelverbindung erhalten und durch Flash-Chromatographie im System O gereinigt. $R_f$(O) = 0,26.

b) H-Leu$\overset{red}{—}$Val-Ile-2-dimethylaminopropylamid

200 mg Z-Leu$\overset{red}{—}$Val-Ile-2-dimethylaminopropylamid werden in Gegenwart von 30 mg Pd-Kohle (10% Pd)-Katalysator hydriert und die Titelverbindung b) nach Flash-Chromatographie im System C erhalten. $R_f$(C) = 0,45.

Beispiel 20:

Z-Phe-Gly-Leu$\overset{red}{—}$Val-Ile-His-$NH_2$

Ausgehend von 145 mg Z-Phe-Gly-OH (Fluka AG, Buchs, Schweiz), 190 mg H-Leu$\overset{red}{—}$Val-Ile-His-$NH_2$ (Herstellung siehe Beispiel 10b)), 62 mg HOBt und 109 mg DCCI wird analog Beispiel 1 die Titelverbindung erhalten und durch Flash-Chromatographie im System Q gereinigt. $R_f$(S) = 0,29; $R_f$(E) = 0,56.

Beispiel 21:

Z-Phe-Gly-Leu$\overset{red}{—}$Val-Ile-2-hydroxyäthylamid

Ausgehend von 139 mg Z-Phe-Gly-OH, 145 mg H-Leu$\overset{red}{—}$Val-Ile-2-hydroxyäthylamid, 60 mg HOBt und 104 mg DCCI wird analog Beispiel 1 die Titelverbindung erhalten und durch Flash-Chromatographie im System R gereinigt. $R_f$(R) = 0,37.

Herstellung des Ausgangsmaterials:

a) Z-Leu$\overset{red}{-}$Val-Ile-2-hydroxyäthylamid

Ausgehend von 315 mg Z-Leu$\overset{red}{-}$Val-Ile-OH (Herstellung siehe Beispiel 14b)), 50 mg Aethanolamin, 104 mg HOBt und 182 mg DCCI wird analog Beispiel 1d) die Titelverbindung erhalten und durch Flash-Chromatographie im System R gereinigt. $R_f(R) = 0,42$.

b) H-Leu$\overset{red}{-}$Val-Ile-2-hydroxyäthylamid

200 mg Z-Leu$\overset{red}{-}$Val-Ile-2-hydroxyäthylamid werden in Gegenwart von 30 mg Pd-Kohle (10% Pd)-Katalysator hydriert und die Titelverbindung b) nach Flash-Chromatographie im System L erhalten. $R_f(L) = 0,57$.

Beispiel 22:

Z-Phe-His-Leu$\overset{red}{-}$Val-Ile-His-OH

130 mg Z-Phe-His-Leu$\overset{red}{-}$Val-Ile-His-$OCH_3$ (Beispiel 14) werden in 2,5 ml Wasser und 2,5 ml Methanol mit 0,22 ml 1 N NaOH-Lösung verseift. Anschliessend wird das Gemisch mit 0,22 ml 1 N HCl-Lösung versetzt, am Rotationsverdampfer vollständig eingedampft und durch Flash-Chromatographie im System E gereinigt. $R_f(E) = 0,32$.

Beispiel 23:

Ac-Phe-His-Leu$\overset{red}{-}$Val-Ile-His-$NH_2$

23 mg Acetyl-L-Phenylalanin, 60 mg H-His-Leu$\overset{red}{-}$Val-Ile-His-$NH_2$ und 16 mg HOBt werden in 2 ml DMF gelöst. Bei 0° werden 27 mg DCCI zugegeben. Die Lösung wird anschliessend während 2 Std. bei 0° und während 16 Std. bei Raumtemperatur gerührt. Es wird vom gebildeten DCH abfiltriert, das Lösungsmittel des Filtrats abgedampft und der Rückstand in 5 ml eines Methanol/Wasser/Eisessig-Gemisches (94:3:3) gelöst. Die Lösung wird während 60 Min. bei 60° gerührt, eingedampft und der Rückstand in wenig Methanol suspendiert. Der erneut gebildete Harnstoff wird abfiltriert und das Filtrat am Rotationsverdampfer zur Trockne eingedampft. Der Rückstand wird durch Flash-Chromatographie an 30 g Kieselgel mit System S gereinigt. Die produkthaltigen Fraktionen werden vereinigt, vom Lösungsmitel befreit, der Rückstand in wenig Methanol gelöst, die Lösung filtriert und das Filtrat erneut eingedampft. Nach Trocknen im Hochvakuum erhält man die Titelverbindung als farbloses Pulver. $R_f(E) = 0{,}11$.

Herstellung des Ausgangsmaterials:

a) Z-His-Leu $\overset{red}{—}$ Val-Ile-His-$NH_2$

Ausgehend von 370 mg Benzyloxycarbonyl-L-Histidin, 600 mg H-Leu $\overset{red}{—}$ Val-Ile-His-$NH_2$ (Herstellung siehe Beispiel 10 b)), 196 mg HOBt und 343 mg DCCI wird analog Beispiel 1) die Titelverbindung erhalten und durch Flash-Chromatographie im System Q gereinigt. $R_f(Q) = 0{,}25$; $R_f(L) = 0{,}46$.

b) H-His-Leu $\overset{red}{—}$ Val-Ile-His-$NH_2$

740 mg Z-His-Leu $\overset{red}{—}$ Val-Ile-His-$NH_2$ werden in Gegenwart von 100 mg Pd-Kohle (10% Pd)-Katalysator hydriert und die Titelverbindung b) nach Flash-Chromatographie im System L erhalten. $R_f(L) = 0{,}24$.

Beispiel 24:

H-Phe-His-Leu $\overset{red}{—}$ Val-Gly-Sar-n-hexylamid

Eine Lösung von 150 mg Z-Phe-His-Leu $\overset{red}{—}$ Val-Gly-Sar-n-hexylamid (siehe Beispiel 5) in 10 ml Methanol/Wasser 9:1 wird in Gegenwart von 30 mg Pd-Kohle (10% Pd) unter $CO_2$-Absorption bis zur Sättigung hydriert. Der Katalysator wird abfiltriert, das Filtrat zur Trockne eingedampft und der Rückstand durch Flash-Chromatographie an 80 g Kieselgel im System E gereinigt. Die DC-einheitlichen Fraktionen

werden vereinigt, eingedampft, der Rückstand in wenig Methanol gelöst, die Lösung filtriert, das Filtrat erneut eingedampft und der Rückstand im Hochvakuum getrocknet. Die Titelverbindung wird als farbloser Schaum erhalten. $R_f(E) = 0{,}23$.

Beispiel 25:

(2S)-5-Oxopyrrolidinyl-2-carbonyl-Phe-His-Leu$\overset{red}{—}$Val-Ile-His-$NH_2$

Ausgehend von 55 mg (2S)-5-Oxopyrrolidinyl-2-carbonyl-L-phenylalanin, 100 mg H-His-Leu$\overset{red}{—}$Val-Ile-His-$NH_2$ (Beispiel 23 b)), 36 mg HOBt und 58 mg DCCI wird analog Beispiel 23 nach Flash-Chromatographie an 65 g Kieselgel mit System O die Titelverbindung erhalten. $R_f(O) = 0{,}33$.

Herstellung des Ausgangsmaterials:

a) (2S)-5-Oxopyrrolidinyl-2-carbonyl-L-phenylalanin-methylester
Ausgehend von 3,49 g L-Pyroglutaminsäure, 5,82 g L-Phenylalanin-methylester-hydrochlorid, 4,13 g HOBt, 2,73 g N-Methylmorpholin und 7,23 g DCCI wird analog Beispiel 1 nach Flash-Chromatographie mit System F die Titelverbindung a) erhalten. $R_f(F) = 0{,}29$.

b) (2S)-5-Oxopyrrolidinyl-2-carbonyl-L-phenylalanin
6,83 g (2S)-5-Oxopyrrolidinyl-2-carbonyl-L-phenylalanin-methylester werden in 400 ml Methanol-Wasser 1:1 gelöst, mit 35,4 ml 1N NaOH versetzt und 2 Std. bei Raumtemperatur stehen gelassen. Anschliessend wird 35,4 ml 1N HCl zugesetzt, am Rotationsverdampfer eingeengt, der Rückstand mit 70 ml Dichlormethan-Aethanol 10:1 verrührt und das ausgefallene NaCl abfiltriert. Die Titelverbindung wird durch Eindampfen des Filtrats erhalten. $R_f(L) = 0{,}08$.

Beispiel 26:

tert-Butylaminocarbonyl-Phe-His-Leu$\overset{red}{\text{——}}$Val-Ile-His-$NH_2$

Ausgehend von 66 mg tert-Butylaminocarbonyl-L-phenylalanin, 100 mg H-His-Leu$\overset{red}{\text{——}}$Val-Ile-His-$NH_2$ (Beispiel 23 b)), 38 mg HOBt und 52 mg DCCI wird analog Beispiel 23 nach Flash-Chromatographie an 65 g Kieselgel mit System L die Titelverbindung erhalten. $R_f(L) = 0,36$.

Herstellung des Ausgangsmaterials:

a) tert-Butylaminocarbonyl-L-phenylalanin-methylester
1,08 g L-Phenylalanin-methylester-hydrochlorid und 0,86 ml Aethyldiisopropylamin werden in 50 ml Methylenchlorid gelöst und bei 0° mit 0,65 ml tert-Butylisocyanat versetzt. Das Reaktionsgemisch wird eingeengt und durch Flash-Chromatographie mit System J gereinigt. $R_f(J) = 0,4$.

b) tert-Butylaminocarbonyl-L-phenylalanin
wird durch Verseifung von 1,4 g tert-Butylaminocarbonyl-L-phenylalanin-methylester mit 7 ml 1N NaOH analog Beispiel 25b) erhalten und durch Flash-Chromatographie mit System S gereinigt. $R_f(S) = 0,20$.

Beispiel 27:

1,1-Dimethyl-2,2,2-trichloräthoxycarbonyl-Phe-His-Leu$\overset{red}{\text{——}}$Val-Ile-His-$NH_2$

Ausgehend von 67 mg 1,1-Dimethyl-2,2,2-trichloräthoxycarbonyl-L-phenylalanin, 100 mg H-His-Leu$\overset{red}{\text{——}}$Val-Ile-His-$NH_2$ (Beispiel 23 b)), 28 mg HOBt und 45 mg DCCI wird analog Beispiel 23 nach Flash-Chromatographie an 65 g Kieselgel mit System L die Titelverbindung erhalten. $R_f(L) = 0,33$; $R_f(O) = 0,21$.

Herstellung des Ausgangsmaterials:

a) 1,1-Dimethyl-2,2,2-trichloräthoxycarbonyl-L-phenylalanin
wird ausgehend von 5,74 g L-Phenylalanin, 10,0 g Chlorameisensäure-1,1-dimethyl-2,2,2-trichloräthylester und 38,2 ml 2N NaOH in einer Schotten-Baumann-Reaktion nach der allgemeinen Vorschrift in "Houben-Weyl, Methoden der organischen Chemie", Band XV/1, Seite 49 (4. Auflage 1974), hergestellt. $R_f(L) = 0,27$.

Beispiel 28:

Cyclopropylcarbonyl-Phe-His-Leu$\overset{red}{—}$Val-Ile-His-$NH_2$

Ausgehend von 40 mg Cyclopropylcarbonyl-L-phenylalanin, 80 mg H-His-Leu$\overset{red}{—}$Val-Ile-His-$NH_2$ (Beispiel 23 b)), 26 mg HOBt und 36 mg DCCI wird analog Beispiel 23 nach Flash-Chromatographie an 65 g Kieselgel mit System T die Titelverbindung erhalten. $R_f(T) = 0,20$; $R_f(L) = 0,38$.

Herstellung des Ausgangsmaterials:

a) Cyclopropylcarbonyl-L-phenylalanin
wird ausgehend von 5,0 g L-Phenylalanin, 3,8 g Cyclopropancarbonsäurechlorid und 33,2 ml 2N NaOH in einer Schotten-Baumann-Reaktion nach der allgemeinen Vorschrift in "Houben-Weyl, Methoden der organischen Chemie", Band XV/1, Seite 49 (4. Auflage 1974), hergestellt. $R_f(L) = 0,16$; $R_f(E) = 0,55$.

Beispiel 29:

H-Phe-His-Leu$\overset{red}{—}$Val-Ile-His-$NH_2$

150 mg Z-Phe-His-Leu$\overset{red}{—}$Val-Ile-His-$NH_2$ (Beispiel 10)), werden in Gegenwart von 30 mg Pd-Kohle (10% Pd) analog Beispiel 24 hydriert und durch Flash-Chromatographie mit System L gereinigt. $R_f$(L) = 0,26.

Beispiel 30:

Pyrrolyl-2-carbonyl-Phe-His-Leu$\overset{red}{—}$Val-Ile-His-$NH_2$

Ausgehend von 16 mg 2-Pyrrolcarbonsäure, 100 mg H-Phe-His-Leu$\overset{red}{—}$Val-Ile-His-$NH_2$ (Beispiel 29)), 23 mg HOBt und 58 mg DCCI wird analog Beispiel 23 nach Flash-Chromatographie an 65 g Kieselgel mit System L die Titelverbindung erhalten. $R_f$(L) = 0,39; $R_f$(O) = 0,18.

Beispiel 31:

Z-Phe-His-Leu$\overset{red}{—}$Val-Ile-2-(4-imidazolyl)-äthylamid

Ausgehend von 100 mg Z-Phe-His-OH, 90 mg H-Leu$\overset{red}{—}$Val-Ile-2-(4-imidazolyl)-äthylamid, 36 mg HOBt und 58 mg DCCI wird analog Beispiel 1 nach Flash-Chromatographie an 65 g Kieselgel mit System O die Titelverbindung erhalten. $R_f$(O) = 0,17; $R_f$(T) = 0,38.

Herstellung des Ausgangsmaterials:

a) Z-Leu$\overset{red}{—}$Val-Ile-2-(4-imidazolyl)-äthylamid

wird ausgehend von 200 mg Z-Phe-His-Leu$\overset{red}{—}$Val-Ile-OH (Beispiel 14b)), 90 mg Histamin-dihydrochlorid, 0,17 µl Aethyldiisopropylamin, 66 mg HOBt und 120 mg DCCI analog Beispiel 1 nach Flash-Chromatographie an 65 g Kieselgel mit System O erhalten. $R_f$(O) = 0,35; $R_f$(L) = 0,70.

b) H-Leu$\overset{red}{—}$Val-Ile-2-(4-imidazolyl)-äthylamid
wird durch Hydrierung von 183 mg Z-Leu$\overset{red}{—}$Val-Ile-2-(4-imidazolyl)-äthylamid in Gegenwart von 40 mg Pd-Kohle (10% Pd) analog Beispiel 10b) erhalten und durch Flash-Chromatographie an 65 g Kieselgel mit System T gereinigt. $R_f$(T) = 0,30.

Beispiel 32:

BOC-Phe-His-Leu$\overset{red}{—}$Val-Ile-3-(4-morpholinyl)-propylamid
Ausgehend von 124 mg BOC-Phe-His-OH (US-Patent 4'025'499), 140 mg H-Leu$\overset{red}{—}$Val-Ile-3-(4-morpholinyl)-propylamid, 62 mg HOBt und 89 mg DCCI wird analog Beispiel 1 nach Flash-Chromatographie an 65 g Kieselgel mit System R die Titelverbindung erhalten. $R_f$(O) = 0,35; $R_f$(F) = 0,28.

Herstellung des Ausgangsmaterials:

a) Z-Leu$\overset{red}{—}$Val-Ile-3-(4-morpholinyl)-propylamid
wird ausgehend von 260 mg Z-Leu$\overset{red}{—}$Val-Ile-OH (Beispiel 14b)), 0,1 ml 4-(3-Aminopropyl)-morpholin, 103 mg HOBt und 162 mg DCCI analog Beispiel 1 nach Flash-Chromatographie an 65 g Kieselgel mit System U erhalten. $R_f$(U) = 0,35; $R_f$(R) = 0,70.

b) H-Leu$\overset{red}{—}$Val-Ile-3-(4-morpholinyl)-propylamid
wird durch Hydrierung von 350 mg Z-Leu$\overset{red}{—}$Val-Ile-3-(4-morpholinyl)-propylamid in Gegenwart von 70 mg Pd-Kohle (10% Pd) analog Beispiel 10b) erhalten und durch Flash-Chromatographie an 65 g Kieselgel mit System O gereinigt. $R_f$(O) = 0,26.

Beispiel 33:

BOC-Phe-His-Leu$\overset{red}{—}$Val-Ile-2-(4-morpholinyl)-äthylamid

Ausgehend von 170 mg BOC-Phe-His-OH, 170 mg H-Leu$\overset{red}{—}$Val-Ile-2-(4-morpholinyl)-äthylamid, 71 mg HOBt und 119 mg DCCI wird analog Beispiel 1 nach Flash-Chromatographie an 65 g Kieselgel mit System O die Titelverbindung erhalten. $R_f(O) = 0,29$; $R_f(L) = 0,49$.

Herstellung des Ausgangsmaterials:

a) Z-Leu$\overset{red}{—}$Val-Ile-2-(4-morpholinyl)-äthylamid
wird ausgehend von 370 mg Z-Leu$\overset{red}{—}$Val-Ile-OH (Beispiel 14b)), 0,12 ml 4-(2-Aminoäthyl)-morpholin, 135 mg HOBt und 214 mg DCCI analog Beispiel 1 nach Flash-Chromatographie an 150 g Kieselgel mit System R erhalten. $R_f(R) = 0,35$; $R_f(O) = 0,54$.

b) H-Leu$\overset{red}{—}$Val-Ile-2-(4-morpholinyl)-äthylamid
wird durch Hydrierung von 380 mg Z-Leu$\overset{red}{—}$Val-Ile-2-(4-morpholinyl)-äthylamid in Gegenwart von 50 mg Pd-Kohle (10% Pd) analog Beispiel 10b) erhalten und durch Flash-Chromatographie an 65 g Kieselgel mit System O gereinigt. $R_f(O) = 0,28$.

Beispiel 34:

Z-Phe-His-Leu$\overset{red}{—}$Val-Ile-3-methoxycarbonylbenzylamid

Ausgehend von 112 mg Z-Phe-His-OH, 94 mg H-Leu$\overset{red}{—}$Val-Ile-3-methoxycarbonylbenzylamid, 46 mg HOBt und 65 mg DCCI wird analog Beispiel 1 nach Flash-Chromatographie an 65 g Kieselgel mit System U die Titelverbindung erhalten. $R_f(U) = 0,22$; $R_f(R) = 0,49$.

Herstellung des Ausgangsmaterials:

a) Z-Leu$\overset{red}{-}$Val-Ile-3-methoxycarbonylbenzylamid
wird ausgehend von 150 mg Z-Leu$\overset{red}{-}$Val-Ile-OH (Beispiel 14b)), 80 mg 3-Aminomethylbenzoesäuremethylester, 74 mg HOBt und 107 mg DCCI analog Beispiel 1 nach Flash-Chromatographie an 65 g Kieselgel mit System N erhalten. $R_f(N) = 0,36$; $R_f(R) = 0,68$.

b) H-Leu$\overset{red}{-}$Val-Ile-3-methoxycarbonylbenzylamid
wird durch Hydrierung von 135 mg Z-Leu$\overset{red}{-}$Val-Ile-3-methoxycarbonylbenzylamid in Gegenwart von 30 mg Pd-Kohle (10% Pd) analog Beispiel 10b) erhalten und durch Flash-Chromatographie an 65 g Kieselgel mit System O gereinigt. $R_f(O) = 0,28$; $R_f(L) = 0,66$.

Beispiel 35:

Z-Phe-His-Leu$\overset{red}{-}$Val-Ile-3-carbamoylbenzylamid
Ausgehend von 33 mg Z-Phe-His-OH, 32 mg H-Leu$\overset{red}{-}$Val-Ile-3-carbamoylbenzylamid, 12 mg HOBt und 19 mg DCCI wird analog Beispiel 1 nach Flash-Chromatographie an 65 g Kieselgel mit System O die Titelverbindung erhalten. $R_f(O) = 0,25$; $R_f(L) = 0,46$.

Herstellung des Ausgangsmaterials:

a) Z-Leu$\overset{red}{-}$Val-Ile-3-carbamoylbenzylamid
wird ausgehend von 100 mg Z-Leu$\overset{red}{-}$Val-Ile-OH (Beispiel 14b)), 36 mg 3-Aminomethyl-benzoesäureamid, 30 mg HOBt und 58 mg DCCI analog Beispiel 1 nach Flash-Chromatographie an 65 g Kieselgel mit System R erhalten. $R_f(R) = 0,34$; $R_f(T) = 0,53$.

b) H-Leu$\overset{red}{-}$Val-Ile-3-carbamoylbenzylamid
wird durch Hydrierung von 92 mg Z-Leu$\overset{red}{-}$Val-Ile-3-carbamoylbenzylamid in Gegenwart von 20 mg Pd-Kohle (10% Pd) analog Beispiel 10b) erhalten und durch Flash-Chromatographie an 65 g Kieselgel mit System O gereinigt. $R_f(O) = 0,28$; $R_f(L) = 0,45$.

Beispiel 36:

BOC-Phe-His-Leu$\overset{red}{-\!-\!-}$Val-Ala-Sta-$OCH_3$

Ausgehend von 74 mg BOC-Phe-His-OH, 70 mg H-Leu$\overset{red}{-\!-\!-}$Val-Ala-Sta-$OCH_3$, 31 mg HOBt und 51 mg DCCI wird analog Beispiel 1 nach Flash-Chromatographie an 65 g Kieselgel mit System R die Titelverbindung erhalten. $R_f(R) = 0,21$; $R_f(S) = 0,55$.

Herstellung des Ausgangsmaterials:

a) Z-Leu$\overset{red}{-\!-\!-}$Val-Ala-tert-butylester

Ausgehend von 740 mg Z-Leu$\overset{red}{-\!-\!-}$Val-OH (Beispiel 1i)), 423 mg L-Alanin-tert-butylester-hydrochlorid, 0,4 ml Aethyldiisopropylamin, 388 mg HOBt und 610 mg DCCI wird analog Beispiel 1 nach Flash-Chromatographie an 200 g Kieselgel mit System F die Titelverbindung erhalten. $R_f(F) = 0,53$.

b) Z-Leu$\overset{red}{-\!-\!-}$Val-Ala-OH

600 mg Z-Leu$\overset{red}{-\!-\!-}$Val-Ala-tert-butylester werden in 50 ml Trifluoressigsäure während 4 Std. bei Raumtemperatur gerührt. Die Reaktionslösung wird am Rotationsverdampfer eingeengt und der Rückstand durch Flash-Chromatographie an 65 g Kieselgel mit System L gereinigt. $R_f(L) = 0,37$.

c) Z-Leu$\overset{red}{-\!-\!-}$Val-Ala-Sta-$OCH_3$

wird ausgehend von 270 mg Z-Leu$\overset{red}{-\!-\!-}$Val-Ala-OH, 144 mg Statinmethylester-hydrochlorid (hergestellt wie beschrieben in der Europäischen Patent-Anmeldung 111'266, $R_f(E) = 0,34$), 0,11 ml Aethyldiisopropylamin, 118 mg HOBt und 172 mg DCCI analog Beispiel 1 nach Flash-Chromatographie an 65 g Kieselgel mit System F erhalten. $R_f(F) = 0,28$; $R_f(R) = 0,45$.

d) H-Leu$\overset{red}{—}$Val-Ala-Sta-$OCH_3$

wird durch Hydrierung von 190 mg Z-Leu$\overset{red}{—}$Val-Ala-Sta-$OCH_3$ in Gegenwart von 50 mg Pd-Kohle (10% Pd) analog Beispiel 10b) erhalten und durch Flash-Chromatographie an 65 g Kieselgel mit System O gereinigt. $R_f(O) = 0,25$; $R_f(L) = 0,66$.

Beispiel 37:

BOC-Phe-His-Leu$\overset{red}{—}$Val-Ala-Sta-$NH_2$

Ausgehend von 60 mg BOC-Phe-His-Leu$\overset{red}{—}$Val-Ala-Sta-$OCH_3$ (Beispiel 36)) wird durch Ammonolyse in 5 ml einer 5 N Lösung von Ammoniak in Methanol während 24 Std. bei Raumtemperatur und anschliessendem Einengen am Hochvakuum die Titelverbindung erhalten und durch Flash-Chromatographie im System O gereinigt. $R_f(O) = 0,25$; $R_f(R) = 0,12$.

Beispiel 38:

BOC-Phe-His-Leu$\overset{red}{—}$Val-Ile-Sta-$NH_2$

Ausgehend von 81 mg BOC-Phe-His-OH, 89 mg H-Leu$\overset{red}{—}$Val-Ile-Sta-$NH_2$, 31 mg HOBt und 49 mg DCCI wird analog Beispiel 1 nach Flash-Chromatographie an 65 g Kieselgel mit System O die Titelverbindung erhalten. $R_f(O) = 0,22$; $R_f(L) = 0,65$.

Herstellung des Ausgangsmaterials:

a) Z-Sta-$NH_2$

wird durch Ammonolyse von 1,00 g Z-Sta-$OCH_3$ (hergestellt wie beschrieben in der Europäischen Patentanmeldung 111'166) in 20 ml 5N Ammoniak-Lösung in Methanol bei Raumtemperatur in 24 Std. erhalten. Die Reaktionslösung wird eingeengt und die Titelverbindung durch Flash-Chromatographie mit System F gereinigt. $R_f(F) = 0,15$.

b) Statinamid

wird ausgehend von 900 mg Z-Sta-$NH_2$ durch Hydrierung in Gegenwart von 120 mg Palladium-Kohle (10% Pd) analog Beispiel 10b) erhalten und durch Flash-Chromatographie mit System L gereinigt. $R_f(L)$ = 0,23.

c) Z-Ile-Sta-$NH_2$

wird ausgehend von 983 mg Z-Ile-OH-Dicyclohexylaminsalz, 348 mg Statinamid, 367 mg HOBt und 536 mg DCCI analog Beispiel 1 nach Flash-Chromatographie an 200 g Kieselgel mit System R erhalten. $R_f(R)$ = 0,37; $R_f(S)$ = 0,63.

d) H-Ile-Sta-$NH_2$

wird durch Hydrierung von 700 mg Z-Ile-Sta-$NH_2$ in Gegenwart von 100 mg Pd-Kohle (10% Pd) analog Beispiel 10b) erhalten und durch Flash-Chromatographie mit System O gereinigt. $R_f(O)$ = 0,20.

e) Z-Leu$\overset{\text{red}}{\text{——}}$Val-Ile-Sta-$NH_2$

wird ausgehend von 159 mg Z-Leu$\overset{\text{red}}{\text{——}}$Val-OH (Beispiel 1i), 108 mg H-Ile-Sta-$NH_2$, 75 mg HOBt und 116 mg DCCI analog Beispiel 1 nach Flash-Chromatographie an 65 g Kieselgel im System R erhalten. $R_f(R)$ = 0,28; $R_f(F)$ = 0,15.

f) H-Leu$\overset{\text{red}}{\text{——}}$Val-Ile-Sta-$NH_2$

wird durch Hydrierung von 180 mg Z-Leu$\overset{\text{red}}{\text{——}}$Val-Ile-Sta-$NH_2$ in Gegenwart von 30 mg Pd-Kohle (10% Pd) analog Beispiel 10b) erhalten und durch Flash-Chromatographie an 65 g Kieselgel mit System O gereinigt. $R_f(O)$ = 0,11; $R_f(L)$ = 0,32.

Beispiel 39:

2-(2-Methoxyäthoxy)-äthoxyacetyl-Phe-His-Leu$\overset{red}{—}$Val-Ile-His-$NH_2$

Ausgehend von 36 mg 2-(2-Methoxyäthoxy)-äthoxyacetyl-L-phenylalanin, 60 mg H-His-Leu$\overset{red}{—}$Val-Ile-His-$NH_2$ (Beispiel 23 b)), 16 mg HOBt und 27 mg DCCI wird analog Beispiel 1 nach Flash-Chromatographie an Kieselgel mit System V die Titelverbindung erhalten. $R_f$(L) = 0,44; $R_f$(E) = 0,15.

Herstellung des Ausgangsmaterials:

a) 2-(Methoxyäthoxy)-äthoxyessigsäure-methylester
24 g Diäthylenglykolmonomethyläther und 300 ml THF abs. werden bei 0°C in Portionen während 15 Min. mit 9,16 g Natriumhydriddispersion (ca. 55%) versetzt. Nach 2 Std. wird bei 0°C eine Lösung von 30,6 g Bromessigsäuremethylester in 100 ml THF zugetropft. Das Reaktionsgemisch wird auf Eis/Wasser gegossen und mehrmals mit Essigester extrahiert. Die organischen Phasen werden getrocknet, eingeengt und der Rückstand bei 129-132°C/20 mbar destilliert, wobei die Titelverbindung als farloses Oel erhalten wird.

b) 2-(2-Methoxyäthoxy)-äthoxyessigsäure
1,0 g 2-(2-Methoxyäthoxy)-äthoxyessigsäure-methylester werden in 25 ml Methanol und 25 ml gesättigter Kaliumcarbonatlösung während 3 Tagen gerührt und die Reaktionsmischung anschliessend filtriert. Das Filtrat wird mit 1N HCl-Lösung angesäuert und mit Essigester extrahiert. Nach dem Trocknen und Eindampfen wird die Titelverbindung durch Destillation bei 170°C/0,2 mbar als leicht gelbliches Oel erhalten.

c) 2-(2-Methoxyäthoxy)-äthoxyacetyl-L-phenylalanin-methylester

Analog Beispiel 1 wird aus 713 mg 2-(2-Methoxyäthoxy)-äthoxyessigsäure, 863 mg L-Phenylalanin-methylester, 612 mg HOBt, 404 mg 4-Methylmorpholin und 1,07 g DCCI die Titelverbindung nach Flash-Chromatographie mit System J erhalten. $R_g(E) = 0{,}80$.

d) 2-(2-Methoxyäthoxy)-äthoxyacetyl-L-phenylalanin

1,05 g 2-(2-Methoxyäthoxy)-äthoxyacetyl-L-phenylalanin-methylester werden in 5 ml Methanol und 10 ml Wasser mit 4,5 ml 1N NaOH-Lösung gerührt und nach 20 Min. mit 4,50 ml 1N HCl-Lösung versetzt. Die Lösung wird eingeengt und mit Essigester extrahiert. Nach dem Trocknen der organischen Phase wird die Titelverbindung als farbloses Oel erhalten. $R_f(E) = 0{,}42$.

Beispiel 40:

Methoxycarbonyl-Phe-His-Leu$\overset{red}{\text{———}}$Val-Ile-His-$NH_2$

Ausgehend von 25 mg Methoxycarbonyl-L-phenylalanin (hergestellt aus L-Phenylalanin und Chlorameisensäuremethylester analog Beispiel 27a)), 60 mg H-His-Leu$\overset{red}{\text{———}}$Val-Ile-His-$NH_2$ (Beispiel 23 b)), 16 mg HOBt und 27 mg DCCI wird analog Beispiel 1 nach Flash-Chromatographie an 65 g Kieselgel mit System V die Titelverbindung erhalten. $R_f(L) = 0{,}40$; $R_f(E) = 0{,}12$.

Beispiel 41:

1-Adamantyloxycarbonyl-Phe-His-Leu$\overset{red}{\text{———}}$Val-Ile-His-$NH_2$

Ausgehend von 53 mg 1-Adamantyloxycarbonyl-L-phenylalanin, 84 mg H-His-Leu$\overset{red}{\text{———}}$Val-Ile-His-$NH_2$ (Beispiel 23 b)), 22 mg HOBt und 37 mg DCCI wird analog Beispiel 1 nach Flash-Chromatographie an 65 g Kieselgel mit System V und E die Titelverbindung als Triacetat erhalten. $R_f(L) = 0{,}48$; $R_f(E) = 0{,}20$.

Herstellung des Ausgangsmaterials:

a) 1-Adamantyloxycarbonyl-L-phenylalanin

3,3 g L-Phenylalanin, 20 ml 1N NaOH-Lösung, 20 ml Wasser und 1,06 g Natriumcarbonat werden bei 0°C während 15 Min. mit 3,97 g Fluorameisensäure-1-adamantylester in 20 ml Aether versetzt. Die Wasserphase wird mit 50 ml 1N HCl-Lösung versetzt und anschliessend mit Essigester extrahiert. Die organische Phase wird eingedampft und getrocknet, wobei die Titelverbindung als weisser Schaum erhalten wird. $R_f(E) = 0{,}50$.

Beispiel 42:

Benzoyl-Phe-His-Leu$\overset{red}{—}$Val-Ile-His-$NH_2$

Ausgehend von 30 mg Benzoyl-L-phenylalanin, 60 mg H-His-Leu$\overset{red}{—}$Val-Ile-His-$NH_2$ (Beispiel 23 b)), 16 mg HOBt und 27 mg DCCI wird analog Beispiel 1 nach Flash-Chromatographie an 65 g Kieselgel mit System V die Titelverbindung erhalten. $R_f(L) = 0{,}46$; $R_f(E) = 0{,}15$.

Herstellung des Ausgangsmaterials:

a) Benzoyl-L-phenylalanin

3,3 g L-Phenylalanin und 20 ml 1N NaOH-Lösung werden bei 0°C gleichzeitig während 30 Min. mit 30 ml 1N NaOH-Lösung und 3,94 g Benzoylchlorid in 30 ml Aether versetzt. Nach 30 Min. Rühren bei Raumtemperatur wird die wässrige Phase mit 50 ml 1N HCl-Lösung versetzt und mit Essigester extrahiert. Die organischen Phasen werden getrocknet und eingeengt. Die Titelverbindung kristallisiert aus Aether/Hexan als weisses Pulver, Smp. 139-140°C.

Beispiel 43:

Pivaloyl-Phe-His-Leu$\overset{red}{\text{—}}$Val-Ile-His-$NH_2$

Ausgehend von 28 mg Pivaloyl-L-phenylalanin (hergestellt nach Chem. Pharm. Bull. 15, 1948 (1967)), 60 mg H-His-Leu$\overset{red}{\text{—}}$Val-Ile-His-$NH_2$ (Beispiel 23 b)), 16 mg HOBt und 27 mg DCCI wird analog Beispiel 1 nach Flash-Chromatographie an 65 g Kieselgel mit System V die Titelverbindung erhalten. $R_f$(L) = 0,46; $R_f$(E) = 0,17.

Beispiel 44:

n-Decanoyl-Phe-His-Leu$\overset{red}{\text{—}}$Val-Ile-His-$NH_2$

Ausgehend von 35 mg n-Decanoyl-L-phenylalanin, 60 mg H-His-Leu$\overset{red}{\text{—}}$Val-Ile-His-$NH_2$ (Beispiel 23 b)), 16 mg HOBt und 27 mg DCCI wird analog Beispiel 1 nach Flash-Chromatographie an 65 g Kieselgel mit System V die Titelverbindung erhalten. $R_f$(L) = 0,53; $R_f$ (E) = 0,19.

Herstellung des Ausgangsmaterials:

a) n-Decanoyl-L-phenylalanin

3,3 g L-Phenylalanin und 20 ml 1N NaOH-Lösung werden bei 0°C gleichzeitig während 30 Min. mit 30 ml 1N NaOH-Lösung und 5,72 g Caprinsäurechlorid in 30 ml Aether versetzt. Mach 30 Min. Rühren bei Raumtemperatur wird die wässrige Phase mit 50 ml 1N HCl-Lösung versetzt und mit Essigester extrahiert. Die organischen Phasen werden getrocknet und eingeengt. Die Titelverbindung kristallisiert aus Aether/Hexan als weisses Pulver, Smp. 101-102°C.

Beispiel 45:

Dimethylaminoacetyl-Phe-His-Leu$\overset{red}{—}$Val-Ile-His-$NH_2$

Ausgehend von 28 mg Dimethylaminoacetyl-L-phenylalanin, 60 mg H-His-Leu$\overset{red}{—}$Val-Ile-His-$NH_2$ (Beispiel 23 b)), 16 mg HOBt und 27 mg DCCI wird analog Beispiel 1 nach Flash-Chromatographie an Kieselgel mit System V die Titelverbindung erhalten. $R_f$(L) = 0,42; $R_f$(E) = 0,03.

Herstellung des Ausgangsmaterials:

a) Dimethylaminoacetyl-L-phenylalanin-methylester
Analog Beispiel 1 wird aus 3,09 g N,N-Dimethylglycin und 6,47 g L-Phenylalanin-methylester-hydrochlorid nach Zugabe von 4,59 g HOBt, 3,03 g 4-Methylmorpholin und 8,03 g DCCI und Flash-Chromatographie mit System R die Titelverbindung erhalten. $R_f$(R) = 0,40.

b) Dimethylaminoacetyl-L-phenylalanin
2,64 g Dimethylaminoacetyl-L-phenylalanin-methylester wird in 10 ml Methanol und 20 ml Wasser mit 15 ml 1N NaOH-Lösung verseift. Nach 30 Min. wird die Lösung mit 15 ml 1N HCl-Lösung versetzt, eingeengt und mit Essigester extrahiert. Nach dem Trocknen der organischen Phase wird die Titelverbindung erhalten. $R_f$(E) = 0,12.

Beispiel 46:

BOC-Phe-His-N-methyl-Leu$\overset{red}{—}$Val-Ile-His-$NH_2$

Ausgehend von 99 mg BOC-Phe-His-OH, 117 mg H-N-Methyl-Leu$\overset{red}{—}$Val-Ile-His-$NH_2$, 37 mg HOBt und 65 mg DCCI wird analog Beispiel 1 nach Flash-Chromatographie an Kieselgel mit System E die Titelverbindung erhalten. $R_f$(L) = 0,52; $R_f$(E) = 0,29.

Herstellung des Ausgangsmaterials:

a) Z-N-Methyl-Leu$\overset{red}{—}$Val-$OCH_3$

701 mg Z-Leu$\overset{red}{—}$Val-OH (Beispiel 1i) in 10 ml Methanol und 1 ml Wasser werden mit 2,5 ml $Cs_2CO_3$-Lösung (20%ig in Wasser) auf pH 9,5 gestellt. Nach 10 Min. wird eingeengt und der Rückstand noch zweimal mit 10 ml DMF aufgenommen und wieder eingeengt. Der Rückstand wird in 10 ml DMF unter Rühren mit 140 µl Methyljodid versetzt und 16 Std. bei Raumtemperatur gerührt. Die Lösung wird zur Trockene eingedampft, mit Wasser aufgenommen und mit Essigester extrahiert. Die organische Phase wird getrocknet und eingeengt. Der Rückstand wird anschliessend im System H an 35 g Kieselgel chromatographiert. Neben drei weiteren Methylierungsprodukten wird die Titelverbindung als farbloses Oel erhalten. $R_f$(H) = 0,20.

b) Z-N-Methyl-Leu$\overset{red}{—}$Val-OH

379 mg Z-N-Methyl-Leu$\overset{red}{—}$Val-$OCH_3$ werden in 10 ml Methanol und 5 ml Wasser mit 1,5 ml 1N NaOH-Lösung verseift. Nach 8 Tagen wird mit 1,5 ml 1N HCl-Lösung versetzt, eingedampft und getrocknet, wobei die Titelverbindung erhalten wird. $R_f$(B) = 0,32.

c) Z-N-Methyl-Leu$\overset{red}{—}$Val-Ile-His-$NH_2$

Analog Beispiel 1 wird aus 399 mg Z-N-Methyl-Leu$\overset{red}{—}$Val-OH und 268 mg H-Ile-His-$NH_2$ (Beispiel 10c)) nach Zugabe von 153 mg HOBt und 268 mg DCCI die Titelverbindung erhalten und durch Flash-Chromatographie mit System B gereinigt. $R_f$(B) = 0,72.

d) H-N-Methyl-Leu$\overset{red}{—}$Val-Ile-His-$NH_2$

Analog Beispiel 1e wird durch Hydrierung von 150 mg Z-N-Methyl-Leu$\overset{red}{—}$Val-Ile-His-$NH_2$ in Gegenwart von 50 mg Pd-Kohle (10% Pd) in 10 ml Methanol/Wasser 9:1 die Titelverbindung erhalten. $R_f$(E) = 0,23.

Beispiel 47:

Cyclopentylcarbonyl-Phe-His-Leu$\overset{red}{—}$Val-Ile-2-(4-imidazolyl)-äthylamid

198 mg Cyclopentylcarbonyl-Phe-His-OH, 127 mg H-Leu$\overset{red}{—}$Val-Ile-2-(4-imidazolyl)-äthylamid (Beispiel 31b)) und 70 mg HOBt werden in 15 ml DMF bei 0°C unter Rühren gelöst und mit 103 mg DCCI versetzt. Das Reaktionsgemisch wird 1 Std. lang bei 0°C und 16 Std. bei Raumtemperatur gerührt. Der ausgefallene DCH wird abfiltriert und das DMF im Hochvakuum entfernt. Der Rückstand wird in 20 ml eines Methanol/Wasser/Eisessig-Gemisches (94:3:3) am Rückfluss 15 Min. lang gekocht. Nach Abdampfen des Lösungsmittels wird der Rückstand an SEPHADEX® LH 20 (Eluiermittel Methanol) chromatographiert. Das so erhaltene Produkt wird in 90% Dioxan lyophilsiert. $R_f(Z_2) = 0{,}63$; $R_f(Z_3) = 0{,}48$.

Herstellung des Ausgangsmaterials:

a) H-Phe-His-$OCH_3$-Hydrochlorid

1,35 g Z-Phe-His-$OCH_3$ (Herstellung siehe H.A. Dewald et al., J. Med. Pharm. Chem. 7, 50 (1964)) werden in 50 ml Methanol und 3 ml 1N Salzsäure gelöst und mit 200 mg Pd-Kohle (10% Pd) versetzt. Das Gemisch wird unter $CO_2$-Asorption bis zur Sättigung hydriert. Der Katalysator und das Lösungsmittel werden entfernt. Man erhält die Titelverbindung als farbloses Oel. Dieses wird ohne weitere Reinigung in die nächste Stufe eingesetzt. $R_f(Y) = 0{,}21$.

b) Cyclopentylcarbonyl-Phe-His-$OCH_3$

1,06 g H-Phe-His-$OCH_3$-Hydrochlorid und 405 mg N-Methylmorpholin werden in 10 ml DMF gelöst und mit 1,06 g Cyclopentancarbonsäure-4-nitrophenylester versetzt. Die Reaktionslösung wird 20 Std. lang bei Raumtemperatur stehen gelassen. Das DMF wird im Hochvakuum

entfernt, und der Rückstand an SEPHADEX® LH 20 (Eluiermittel Methanol) chromatographiert. Das erhaltene reine Produkt wird in 15 ml Dioxan suspendiert und gefriergetrocknet. $R_f(Y) = 0,49$; $R_f(Z_4) = 0,67$.

c) Cyclopentylcarbonyl-Phe-His-OH

990 mg Cyclopentylcarbonyl-Phe-His-$OCH_3$ werden in 12 ml Dioxan und 12 ml Wasser unter Rühren gelöst und mit 7,2 ml 1N Natronlauge versetzt. Nach 3 Min. werden 3 ml 1N Salzsäure zugegeben. Das Lösungsmittel wird abgedampft, und der Rückstand an BIOGEL $P_2$® chromatographiert. Die erhaltene Substanz wird in 30 ml Wasser lyophilsiert. $R_f(Z_2) = 0,5$; $R_f(Z_3) = 0,35$; $R_f(Z_4) = 0,33$.

Beispiel 48:

Cyclopentylcarbonyl-Phe-$N^{\alpha}$-methyl-His-Leu$\overset{red}{—}$Val-Ile-2-(4-imidazolyl)-äthylamid

101 mg H-Phe-$N^{\alpha}$-methyl-His-Leu$\overset{red}{—}$Val-Ile-2-(4-imidazolyl)-äthylamid werden in 2 ml DMF gelöst und mit 95 mg Cyclopentancarbonsäure-4-nitrophenylester versetzt. Die Reaktionslösung wird 4 Std. lang bei Raumtemperatur stehen gelassen. Das Lösungsmittel wird am Hochvakuum entfernt und der Rückstand an Sephadex® LH 20 mit Methanol und anschliessend an Kieselgel mit Chloroform/Methanol (7:3) chromatographiert. Das erhaltene Produkt wird in 90% Dioxan lyophilisiert. $R_f(Z) = 0,31$; $R_f(Z_3) = 0,45$.

Herstellung des Ausgangsmaterials:

a) BOC-$N^{\alpha}$-methyl-His-Leu$\overset{red}{—}$Val-Ile-2-(4-imidazolyl)-äthylamid

210 mg tert-Butoxycarbonyl-L-$N^{\alpha}$-methylhystidin, 211 mg H-Leu$\overset{red}{—}$Val-Ile-2-(4-imidazolyl)-äthylamid (Beispiel 31b) und 135 mg HOBt werden unter Rühren in 6 ml DMF gelöst. Es werden 145 mg DCCI zugegeben. Die Reaktionslösung wird 15 Std. lang bei Raumtemperatur stehen gelassen. Man entfernt das DMF im Hochvakuum und kocht den

Rückstand in 30 ml eines Methanol/Wasser/Eisessig-Gemisches (94:3:3) 45 Min. lang am Rückfluss. Nach Entfernen des Lösungsmittels wird der Rückstand an SEPHADEX® LH 20 chromatographiert (Eluiermittel Methanol). Die Titelverbindung wird durch Lyophilsierung aus 90% Dioxan erhalten. $R_f(Z) = 0,35$; $R_f(Z_3) = 0,46$.

b) H-N$^{\alpha}$-Methyl-His-Leu$\overset{red}{\text{——}}$Val-Ile-2-(4-imidazolyl)-äthyl-amid-hydrochlorid

135 mg BOC-N$^{\alpha}$-methyl-His-Leu$\overset{red}{\text{——}}$Val-Ile-2-(4-imidazolyl)-äthyl-amid werden in 2 ml 4,3N HCl in Dioxan suspendiert. Nach 60 Min. wird das Reaktionsgemisch zur Trockene eingedampft und der Rückstand im Exsikkator über Natriumhydroxid-Plätzchen getrocknet. $R_f(Z) = 0,09$; $R_f(Z_3) = 0,32$.

c) Z-Phe-N$^{\alpha}$-methyl-His-Leu$\overset{red}{\text{——}}$Val-Ile-2-(4-Imidazolyl)-äthylamid

210 mg Benzyloxycarbonyl-L-phenylalanin-4-nitrophenylester und 122 mg H-N$^{\alpha}$-Methyl-His-Leu$\overset{red}{\text{——}}$Val-Ile-2-(4-imidazolyl)-äthylamid-hydrochlorid werden in 6 ml DMF gelöst und nach Zugabe von 500 µl N-Methylmorpholin 16 Std. lang reagieren gelassen. Man entfernt das DMF im Hochvakuum und chromatographiert den Rückstand an SEPHADEX® LH 20 (Eluiermittel Methanol) und an Kieselgel (Eluiermittel Chloroform/Methanol 7:3). Das so erhaltene gewünschte Produkt wird in 10 ml 90% Dioxan lyophilisiert. $R_f(Z) = 0,40$; $R_f(Z_3) = 0,55$.

d) H-Phe-N$^{\alpha}$-methyl-His-Leu$\overset{red}{\text{——}}$Val-Ile-2-(4-imidazolyl)-äthylamid

150 mg Z-Phe-N$^{\alpha}$-methyl-His-Leu$\overset{red}{\text{——}}$Val-Ile-2-(4-imidazolyl)-äthyl-amid werden in 12 ml Methanol in Gegenwart von 30 mg Pd-Kohle (10% Pd) unter $CO_2$-Absorption zur Sättigung hydriert. Der Katalysator wird abfiltriert und das Lösungsmitel abgedampft, wobei die Titelverbindung als farbloses Oel erhalten wird. $R_f(Z) = 0,21$; $R_f(Z_3) = 0,33$.

Beispiel 49:

Cyclopentylcarbonyl-Phe-His-(erythro-2-amino-1-hydroxy-1-phenyl-propyl)-D- oder L-Ala-Ile-2-(4-imidazolyl)-äthylamid (Diastereomere A I und A II)

180 mg Cyclopentylcarbonyl-L-phenylalanin-N-hydroxysuccinimidester, 180 mg H-His-(erythro-2-amino-1-hydroxy-1-phenyl-propyl)-D- oder L-Ala-Ile-2-(4-imidazolyl)-äthylamid-hydrochlorid (Diastereoisomerenpaar A) und 0,5 ml einer 1M N-Methylmorpholin-Lösung werden in 6 ml DMF gelöst und während 15 Std. reagieren gelassen. Das DMF wird im Hochvakuum entfernt und der Rückstand an SEPHADEX® LH 20 (Eluiermittel Methanol) und an Kieselgel (Eluiermittel Chloroform/Methanol 7:3) chromatographiert. Das lipophilere Diastereoisomere A I und das hydrophilere Diastereoisomere A II werden durch mehrere Chromatogramme an Kieselgel rein erhalten und in 90% Dioxan lyophilisiert.
Diastereoisomeres A I : $R_f(Z_1) = 0,37$;
Diastereoisomeres A II: $R_f(Z_1) = 0,27$.

Herstellung des Ausgangsmaterials:

a) α-Brom-ß-amino-propiophenon-hydrobromid
36 g (0,129 Mol) ß-Phthalimido-propiophenon (hergestellt nach A. Butenandt und U. Renner, Z. Naturforsch. 8b, 454 (1963)) werden in 80 ml Eisessig gelöst und bei 75-80°C innert 45 Min. mit einer Lösung von 22,5 g (0,28 Mol) Brom in 10 ml Eisessig versetzt. Anschliessend wird 2 Std. unter Rückfluss zum Sieden erhitzt und der Ueberschuss an Brom durch Einblasen von Stickstoff während 15 Min. beseitigt. Man versetzt die Reaktionslösung mit weiteren 50 ml Eisessig und 100 ml 48% Bromwasserstoffsäure und erhitzt 18 Std. zum Rückfluss. Nach Abkühlen im Eisbad wird von abgeschiedener Phthalsäure abgesaugt, mit wenig Wasser nachgewaschen und das Filtrat zur Trockene eingedampft. Der Rückstand wird in Wasser aufgenommen,

Ungelöstes (Phthalsäure) abfiltriert und das Filtrat wiederum zur Trockene eingedampft. Der Rückstand wird durch Behandeln mit Essigester/Aceton in eine farblose, filtrierbare Kristallmasse übergeführt. Smp. 188-190° (unter Zersetzung).

b) α-Azido-ß-tert-butoxycarbonylamino-propiophenon
155 g (0,5 Mol) α-Brom-ß-amino-propiophenon-hydrobromid und 80 g Di-tert-butylcarbonat werden in 500 ml DMF gelöst und der Lösung unter Rühren bei 0-4°C innert 45 Min. eine Lösung von 20 g Natriumhydroxid in 100 ml Wasser zugesetzt. Anschliessend werden bei 0-5°C 35 g (0,54 Mol) Natriumazid eingetragen, wobei die Temperatur auf 10°C ansteigt. Man rührt noch 4 Std. bei Raumtemperatur, giesst das Reaktionsgemisch auf Wasser und extrahiert mit Aether. Der Aetherextrakt wird mit Wasser gewaschen, über Natriumsulfat getrocknet und eingedampft. Der ölige Rückstand kristallisiert beim Anreiben mit Aether/Hexan. Smp. 68-69°.

c) α-Benzyloxycarbonylamino-ß-tert-butoxycarbonylamino-propiophenon
11,6 g (0,04 Mol) α-Azido-ß-tert-butoxycarbonylamino-propiophenon und 10,1 g (0,16 Mol) Ammoniumformiat werden unter Rühren in eine unter Stickstoff bereitete Suspension von 1,2 g Palladiumkohle (5%) in 200 ml Methanol eingetragen. Die Reaktion setzt sofort unter Gasentwicklung ein. Man rührt 4 Std. bei Raumtemperatur, filtriert den Katalysator ab, kühlt die Lösung auf 0-4°C ab und versetzt innert 30 Min. mit 15 ml Chlorameisensäurebenzylester (50% in Toluol) und anschliessend mit 15 ml Wasser. Man rührt noch 1 Std. bei Raumtemperatur, filtriert von unlöslichen Nebenprodukten ab, dampft das Filtrat zur Trockene ein und nimmt den Rückstand in Essigester auf. Die Essigesterlösung wird mit Wasser gewaschen, über Natriumsulfat getrocknet und eingedampft. Den Rückstand nimmt man in Aether auf, klärt die Lösung durch Filtration, dampft den Aether ab und kristallisiert das Reaktionsprodukt aus Aether/Hexan. Smp. 105-106°.

d) 2-Benzyloxycarbonylamino-3-tert-butoxycarbonylamino-1-phenyl-1-propanol

21 g (0,0527 Mol) Benzyloxycarbonylamino-ß-tert-butoxycarbonylamino-propiophenon werden in 250 ml Methanol gelöst und der Lösung bei 0-3°C innert 30 Min. portionenweise 2,1 g Natriumborhydrid zugesetzt. Man rührt noch 15 Min., engt im Vakuum ein, nimmt den Rückstand in Essigester auf, wäscht mit Wasser neutral, trocknet die Lösung über Natriumsulfat und dampft zur Trockne ein. Der Rückstand kristallisiert zum Teil beim Anreiben mit Aether. Das Kristallisat, das d,l-erythro-Isomere der Titelverbindung, wird durch Filtration abgetrennt. Es schmilzt bei 143-145°. Aus dem Filtrat kann durch fraktionierte Kristallisation eine weitere Fraktion des erythro-Isomeren und das threo-Isomere vom Smp. 75-78° gewonnen werden.

e) d,l-erythro-3-Amino-2-benzyloxycarbonylamino-1-phenyl-1-propanol-hydrochlorid

2 g (0,005 Mol) d,l-erythro-2-Benzyloxycarbonylamino-3-tert-butyl-oxycarbonylamino-1-phenyl-1-propanol werden in 50 ml Aethanol gelöst, mit 50 ml 0,1 N Salzsäure versetzt und die Lösung 6 Std. auf 80°C erhitzt. Man dampft im Vakuum zur Trockene ein und entfernt restliches Wasser durch azeotrope Destillation mit Toluol. Das als kristalliner Rückstand verbleibende Reaktionsprodukt schmilzt bei 204-206°. Die aus dem Hydrochlorid freigesetzte Base kristallisiert aus Aether, Smp. 132-134°.

f) N-[d,l-erythro-2-Benzyloxycarbonylamino-1-hydroxy-1-phenyl-propyl]-D,L-alanin-äthylester (Diastereomere A und B)

15,8 g (0,047 Mol) d,l-erythro-3-Amino-2-benzyloxycarbonylamino-1-phenyl-1-propanol-hydrochlorid werden in 250 ml absolutem Methanol gelöst, 5,5 ml (0,049 Mol) Brenztraubensäureäthylester und 2 g Natriumcyanoborhydrid zugesetzt und das Gemisch bei Raumtemperatur gerührt. Nach 1 Tag werden weitere 1 ml Brenztraubensäureäthylester

und 1 g Natriumcyanoborhydrid zugesetzt, ebenso nach 2 Tagen. Nach 3 1/2 Tagen wird mit 2N Salzsäure angesäuert, das Reaktionsgemisch eingedampft und der Rückstand mit Essigester und 2N Ammoniaklösung verrührt. Die Essigesterphase wird abgetrennt, mit Wasser gewaschen, über Natriumsulfat getrocknet und eingedampft. Der Rückstand, ein farbloses Oel, wird an Kieselgel chromatographiert. Durch Elution mit Hexan/Essigester 9:1 erhält man zunächst ein weniger polares, aus Aether/Hexan mit Smp. 75-77° kristallisierendes Diastereomeres B des Reaktionsproduktes, spätere Fraktionen enthalten das polarere Diastereomere A, das aus Aether/Hexan mit Smp. 73-75° kristallsiert.

g) N-[d,l-erythro-2-Benzyloxycarbonylamino-1-hydroxy-1-phenyl-propyl]-D,L-alanin (Diastereomeres Razemat A)

1,15 g (2,87 mMol) N-[d,l-erythro-2-Benzyloxycarbonylamino-1-hydroxy-1-phenyl-propyl]-D,L-alanin-äthylester (Diastereomeres A) werden in 15 ml Aethanol gelöst und nach Versetzen mit 2,9 ml 1N Natronlauge 6 Std. bei Raumtemperatur gerührt. Man säuert mit 2 ml 2N Salzsäure an, versetzt mit 0,5 ml Propylenoxid und rührt 14 Std. bei Raumtemperatur. Nach Einengen der Lösung im Vakuum wird das auskristallisierte Reaktionsprodukt abgesaugt. Smp. 202-205° (unter Zersetzung).

h) N-(erythro-2-Benzyloxycarbonylamino-1-hydroxy-1-phenyl-propyl)-D- oder L-Ala-Ile-2-(4-imidazolyl)-äthylamid (Diastereomerenpaar A)

373 mg N-(d,l-erythro-2-Benzyloxycarbonylamino-1-hydroxy-1-phenyl-propyl)-D,L-alanin (Diastereoisomeres Razemat A), 224 mg L-Isoleucin-2-(4-imidazolyl)-äthylamid (Herstellung siehe unten) und 270 mg HOBt werden in 6 ml DMF gelöst und mit 309 mg DCCI versetzt. Das Reaktionsgemisch wird 20 Stunden lang bei Raumtemperatur gerührt. Der gebildete DCH wird abfiltriert und das Filtrat zur Trockene eingedampft. Der Rückstand wird in 20 ml eines Methanol/-Wasser/Eisessig-Gemisches (94:3:3) am Rückfluss 60 Min. lang gekocht. Nach Abdampfen des Lösungsmittels wird der Rückstand an SEPHADEX® LH 20 (Eluiermittel Methanol) chromatographiert. Das Produkt wird in 90% Dioxan lyophilisiert. $R_f(Z) = 0,38$; $R_f(Z_6) = 0,51$.

i) N-(erythro-2-Amino-1-hydroxy-1-phenyl-propyl)-D- oder L-Ala-Ile-2-(4-imidazolyl)-äthylamid (Diastereomerenpaar A)

290 mg N-(erythro-2-Benzyloxycarbonylamino-1-hydroxy-1-phenyl-propyl)-D- oder L-Ala-Ile-2-(4-imidazolyl)-äthylamid werden in 10 ml Methanol gelöst und mit 50 mg Pd-Kohle (10% Pd) versetzt. Das Gemisch wird unter $CO_2$-Absorption bis zur Sättigung hydriert. Der Katalysator wird abfiltriert und das Filtrat zur Trockene eingeengt. Das Produkt wird in dieser Form in die nächste Stufe eingesetzt. $R_f(Z_6)$ = 0,24.

j) BOC-His-(erythro-2-amino-1-hydroxy-1-phenyl-propyl)-D- oder L-Ala-Ile-2-(4-imidazolyl)-äthylamid (Diastereomerenpaar A)

191 mg BOC-His-OH, 222 mg N-(erythro-2-Amino-1-hydroxy-1-phenyl-propyl)-D- oder L-Ala-Ile-2-(4-imidazolyl)-äthylamid und 135 mg HOBt werden in 6 ml DMF gelöst und mit 155 mg DCCI versetzt. Nach 22 Std. wird der ausgefallene DCH abfiltriert und das Filtrat zur Trockene eingedampft. Der Rückstand wird an SEPHADEX® LH 20 (Eluiermittel Methanol) chromatographiert und das so erhaltene Produkt in 15 ml Dioxan lyophilisiert. $R_f(Z)$ = 0,32; $R_f(Z_3)$ = 0,37.

k) H-His-(erythro-2-amino-1-hydroxy-1-phenyl-propyl)-D- oder L-Ala-Ile-2-(4-imidazolyl)-äthylamid-hydrochlorid (Diastereomerenpaar A)

205 mg BOC-His-(erythro-2-amino-1-hydroxy-1-phenyl-propyl)-D- oder L-Ala-Ile-2-(4-imidazolyl)-äthylamid werden in 2 ml 4,3N HCl in Dioxan suspendiert und während 1 Std. bei Raumtemperatur reagieren gelassen. Das Reaktionsgemisch wird zur Trockene eingeengt und der Rückstand im Exsikkator in Gegenwart von Natriumhydroxid-Plätzchen getrocknet. Das Produkt wird in wenig 90% Dioxan lyophilisiert. $R_f(Z)$ = 0,07.

l) Z-Ile-2-(4-imidazolyl)-äthylamid

1,84 g Histamindihydrochlorid und 2,03 g N-Methylmorpholin werden bei 0° unter Rühren in 30 ml DMF gelöst. Es werden 4,35 g Z-Ile-N-Hydroxysuccinimidester zugegeben. Das Reaktionsgemisch wird 2 Std. lang bei 0° und 20 Std. bei Raumtemperatur gerührt. Das gebildete

N-Methylmorpholiniumchlorid wird abfiltriert und das DMF im Hochvakuum entfernt. Der Rückstand wird an SEPHADEX® LH 20 (Eluiermittel Methanol) und an Kieselgel (Eluiermittel Chloroform/Methanol 8:2) chromatographiert. Das erhaltene Produkt wird aus Methanol/Essigester 1:2 auskristallisiert. $R_f(Z) = 0,51$; $R_f(Y) = 0,44$; $R_f(Z_4) = 0,54$.

m) L-Isoleucin-2-(4-imidazolyl)-äthylamid

360 mg Z-Ile-2-(4-imidazolyl)-äthylamid werden in 10 ml Methanol gelöst und nach Zugabe von 50 mg Pd-Kohle (10% Pd) unter $CO_2$-Absorption bis zur Sättigung hydriert. Der Katalysator wird abfiltriert und das Filtrat eingeengt. Das Produkt wird in der anfallenden öligen Form in Stufe h) eingesetzt. $R_f(Z) = 0,2$; $R_f(Z_4) = 0,12$.

Beispiel 50:

Cyclopentylcarbonyl-Phe-His-(erythro-2-amino-1-hydroxy-1-phenyl-propyl)-D- oder L-Ala-Ile-2-(4-imidazolyl)-äthylamid (Diastereomere B I und B II)

Analog Beispiel 49 wird durch Umsetzung von 180 mg H-His-(erythro-2-amino-1-hydroxy-1-phenyl-propyl)-D- oder L-Ala-Ile-2-(4-imidazolyl)-äthylamid (Diastereomerenpaar B) mit 180 mg Cyclopentylcarbonyl-L-phenylalanin-N-hydroxysuccinimidester und 0,5 ml einer 1M N-Methylmorpholin-Lösung die Titelverbindung hergestellt. Das lipophilere Diastereomere B I und das hydrophilere Diastereomere B II werden durch eine Craig-Verteilung im System tert-Butanol/-Toluol/Puffer (5,7 ml Eisessig und 0,77 g Ammoniumacetat pro Liter Wasser) 44:1:5 getrennt.

Diastereoisomeres B I : $R_f(Z_1) = 0,39$;
Diastereoisomeres B II : $R_f(Z_1) = 0,34$.

Die Ausgangsmaterialien der Diastereomerenreihe B werden analog Beispiel 49g) bis 49k) hergestellt:

N-(d,l-erythro-2-Benzyloxycarbonylamino-l-hydroxy-l-phenyl-propyl)-D,L-alanin (Diastereomeres Razemat B), Smp. (unter Zersetzung) 201-205°C.

N-(erythro-2-Benzyloxycarbonylamino-l-hydroxy-l-phenyl-propyl)-D- oder L-Ala-Ile-2-(4-imidazolyl)-äthylamid (Diastereomerenpaar B), $R_f(Z) = 0,38$; $R_f(Y) = 0,22$.

N-(erythro-2-Amino-l-hydroxy-l-phenyl-propyl)-D- oder L-Ala-Ile-2-(4-imidazolyl)-äthylamid (Diastereomerenpaar B), $R_f(Z_6) = 0,29$.

BOC-His-(erythro-2-amino-l-hydroxy-l-phenyl-propyl)-D- oder L-Ala-Ile-2-(4-imidazolyl)-äthylamid (Diastereomerenpaar B), $R_f(Z) = 0,31$; $R_f(Z_3) = 0,48$.

H-His-(erythro-2-amino-l-hydroxy-l-phenyl-propyl)-D- oder L-Ala-Ile-2-(4-imidazolyl)-äthylamid-hydrochlorid (Diastereomerenpaar B), $R_f(Z_3) = 0,1$.

Beispiel 51:

BOC-Phe-His-Leu$\overset{red}{\text{—}}$Ser-Ile-His-$NH_2$

Ausgehend von 241 mg BOC-Phe-His-OH, 272 mg H-Leu$\overset{red}{\text{—}}$Ser-Ile-His-$NH_2$, 92 mg HOBt und 162 mg DCCI wird analog Beispiel 1, jedoch nach einer Reaktionszeit von 24 Std., die Titelverbindung hergestellt und nach Chromatographie mit System E als Triacetat erhalten. $R_f(E) = 0,16$; $R_f(L) = 0,27$.

Herstellung des Ausgangsmaterials:

a) Z-Leu$\overset{red}{\text{—}}$Ser-$CH_3$

Zu einer Lösung von 3,48 g 2(S)-Benzyloxycarbonylamino-4-methylpentanal (Herstellung siehe Chem. Pharm. Bull. 23, 3081 (1975)) in 50 ml abs. THF, welches 20 g Molekularsieb enthält, wird 2,17 g

L-Serin-methylester-hydrochlorid in 20 ml Methanol und anschliessend 1,54 ml N-Methylmorpholin zugegeben. Das Reaktionsgemisch wird 20 Std. bei Raumtemperatur unter Stickstoffatmosphäre stehen gelassen, anschliessend mit Eis gekühlt und danach mit 1,05 g Natriumcyanoborhydrid in 8 ml Methanol versetzt. Das Gemisch wird bei Raumtemperatur 4 Std. gerührt, filtriert und das Filtrat eingedampft. Der Rückstand wird in Essigester gelöst, die Lösung mit gesättigter Natriumbicarbonatlösung und Sole gewaschen, mit Natriumsulfat getrocknet und eingedampft. Die Kristallisation des Rückstandes aus Cyclohexan ergibt die Titelverbindung als weisse Nadeln vom Smp. 103-106°. $R_f(K) = 0,78$.

b) Z-Leu$\overset{red}{\text{—}}$Ser-OH

264,3 mg Z-Leu$\overset{red}{\text{—}}$Ser-$OCH_3$ werden in 15 ml Methanol gelöst, bei Raumtemperatur mit 15 ml 0,1N Natronlauge versetzt und 2 Std. lang gerührt. Anschliessend wird 0,1N Salzsäure zugegeben und das Gemisch eine Std. im Eisbad gerührt. Die weisse Suspension wird abfiltriert und mit wenig Wasser gewaschen. Nach Trocknen im Hochvakuum erhält man die Titelverbindung mit einem Smp. von 208-209° (Zersetzung). $R_f(E) = 0,82$; $R_f(L) = 0,17$.

c) Z-Leu$\overset{red}{\text{—}}$Ser-Ile-His-$NH_2$

Ausgehend von 406,1 mg Z-Leu$\overset{red}{\text{—}}$Ser-OH, 320,8 mg H-Ile-His-$NH_2$, 184 mg HOBt und 322 mg DCCI wird analog Beispiel 10a), jedoch nach einer Reaktionszeit von 140 Std., die Titelverbindung nach Flash-Chromatographie mit System O erhalten. $R_f(L) = 0,41$; $R_f(E) = 0,29$.

d) H-Leu$\overset{red}{\text{—}}$Ser-Ile-His-$NH_2$

Ausgehend von 559,6 mg Z-Leu$\overset{red}{\text{—}}$Ser-Ile-His-$NH_2$ wird durch Hydrierung in Gegenwart von 100 mg Pd-Kohle (10% Pd) analog Beispiel 10b) die Titelverbindung d) erhalten und durch Flash-Chromatographie mit System L gereinigt. $R_f(L) = 0,05$; $R_f(E) = 0,05$.

Beispiel 52:

BOC-Phe-His-(2(S)-amino-4-methyl-pentyl)-2(R,S)-amino-4-benzyloxy-butyryl-Ile-His-$NH_2$

Ausgehend von 140,9 mg BOC-Phe-His-OH, 195,2 mg (2(S)-Amino-4-methyl-pentyl)-2(R,S)-amino-4-benzyloxy-butyryl-Ile-His-$NH_2$, 54 mg HOBt und 94 mg DCCI wird analog Beispiel 1 bei einer Reaktionszeit von 24 Std. die Titelverbindung nach Flash-Chromatographie mit System O erhalten. $R_f$(L) = 0,52; $R_f$(E) = 0,40.

Herstellung ds Ausgangsmaterials:

a) 2-Acetylamino-2-benzyloxyäthyl-malonsäurediäthylester

2,3 g Natrium werden portionenweise in 250 ml absoluten Aethanol gelöst. Dazu werden 21,7 g Acetylaminomalonsäurediäthylester und dann unter Eiskühlung innert 15 Min. 21,5 g Benzyl-2-bromäthyläther (Herstellung siehe D. Klamman, P. Weyerstahl, Liebig's Ann. Chem. 710, 59-70, (1967)) zugetropft. Die Lösung wird 24 Std. gekocht und eingedampft. Der Rückstand wird in Aether aufgenommen, mit Wasser gewaschen, mit Natriumsulfat getrocknet und eingedampft. Der Rückstand wird an 650 g Kieselgel (Lösungsmittel: Essigester-Hexan 1:3) durch Flash-Chromatographie gereinigt. Die produkthaltigen Fraktionen werden vereinigt und eingedampft. Die Kristallisation des Rückstandes aus Aether/n-Hexan ergibt die Titelverbindung als weisse Nadeln vom Smp. 55-57°. $R_f$(Essigester/Hexan 2:3) = 0,20.

b) 2-Amino-4-benzyloxybuttersäure (O-Benzyl-D,L-Homoserin)

13,0 g 2-Acetylamino-2-benzyloxyäthyl-malonsäurediäthylester werden in 110 ml 2N Natronlauge und 100 ml Wasser suspendiert und 16 Std. gekocht. Die Lösung wird mit 110 ml 2N HCl angesäuert, 16 Std. gekocht und anschliessend eingedampft. Der Rückstand wird in 1 Liter 0,1N HCl gelöst, 24 Std. gekocht und auf 400 ml eingedampft. Die Lösung wird mit 2N Natronlauge auf pH 5 gebracht und auf 0° gekühlt,

wobei weisse Kristalle nach wenigen Min. auszufallen beginnen. Die Mutterlauge wird entfernt und die Kristalle aus Wasser umkristallisiert. Nach Trocknen im Hochvakuum erhält man die Titelverbindung als weisse Platten vom Smp. 230-235° (Zersetzung). $R_f$(E) = 0,29.

c) 2-Amino-4-benzyloxybuttersäuremethylester-hydrochlorid

Zu 20 ml Methanol werden bei -20° 1,3 ml Thionylchlorid zugetropft und anschliessend 3,14 g 2-Amino-4-benzyloxybuttersäure zugegeben. Die Lösung wird 10 Min. bei -20° und 16 Std. bei Raumtemperatur stehen gelassen, dann eingedampft. Der Rückstand wird im Hochvakuum getrocknet und ergibt die Titelverbindung als farbloses Oel. $R_f$(E) = 0,81.

d) (2(S)-tert-Butoxycarbonylamino-4-methyl-pentyl)-2(R,S)-amino-4-benzyloxy-buttersäuremethylester

Zu einer Lösung von 2,45 g 2(S)-tert-Butoxycarbonylamino-4-pentyl-pentanal in 50 ml abs. THF, welches 20 g Molekularsieb enthält, werden 2,96 g 2-Amino-4-benzyloxbuttersäuremethylester-hydrochlorid in 20 ml Methanol und anschliessend 1,25 ml N-Methylmorpholin zugegeben. Das Reaktionsgemisch wird 18 Std. bei Raumtemperatur unter Stickstoffatmosphäre stehen gelassen, anschliessend mit Eis gekühlt und danach mit 195 mg Natriumcyanoborhydrid in 4 ml Methanol versetzt. Das Gemisch wird bei Raumtemperatur 1 Std. gerührt, filtriert und das Filtrat eingedampft. Der Rückstand wird in Essigester gelöst und die Lösung mit gesättigter Natriumcarbonatlösung und Sole gewaschen, mit Natriumsulfat getrocknet und eingedampft. Der Rückstand wird an 170 g Kieselgel (Lösungsmittel: Essigester/Hexan 1:4) durch Flash-Chromatographie gereinigt. Die produkthaltigen Fraktionen werden vereinigt und eingedampft. Nach Trocknen im Hochvakuum erhält man die Titelverbindung als gelbes Oel. $R_f$(Essigester/Hexan 3:7) = 0,55.

e) (2(S)-tert-Butoxycarbonylamino-4-methyl-pentyl)-2(R,S)-amino-4-benzyloxybuttersäure

1,5 g (2(S)-tert-Butoxycarbonylamino-4-methyl-pentyl)-2(R,S)-amino-4-benzyloxy-buttersäuremethylester werden in 75 ml Methanol gelöst und bei Raumtemperatur mit 5,3 ml 1N Natronlauge und 10 ml Wasser versetzt. Nach 115 Std. werden 5,3 ml 1N HCl zugegeben und das Gemisch eingedampft. Der Rückstand wird an 100 g Kieselgel (Lösungsmittel: Methylenchlorid/Methanol/Wasser 360:40:1) durch Flash-Chromatographie gereinigt. die produkthaltigen Fraktionen werden vereinigt und eingedampft. Nach Trocknen im Hochvakuum erhält man die Titelverbindung als farbloses Pulver. $R_f$(V) = 0,17.

f) (2(S)-tert-Butoxycarbonylamino-4-methyl-pentyl)-2(R,S)-amino-4-benzyloxy-butyryl-Ile-His-$NH_2$

Ausgehend von 612,8 mg (2(S)-tert-Butoxycarbonylamino-4-methyl-pentyl)-2(R,S)-amino-4-benzyloxybuttersäure, 401 mg H-Ile-His-$NH_2$, 230 mg HOBt und 402 mg DCCI wird analog Beispiel 1 die Titelverbindung nach Flash-Chromatographie mit System K erhalten. $R_f$(K) = 0,28; $R_f$(E) = 0,44.

g) (2(S)-Amino-4-methyl-pentyl)-2(R,S)-amino-4-benzyloxy-butyryl-Ile-His-$NH_2$

493,4 mg (2(S)-tert-Butoxycarbonylamino-4-methyl-pentyl)-2(R,S)-amino-4-benzyloxy-butyryl-Ile-His-$NH_2$ werden in 10 ml Trifluoressigsäure gelöst, 5 Min. bei Raumtemperatur gerührt und das Gemisch eingedampft. Der Rückstand wird in n-Butanol aufgenommen, die Lösung mit gesättigter Natriumbicarbonatlösung gewaschen und eingedampft. Der Rückstand wird an 30 g Kieselgel im System L durch Flash-Chromatographie gereinigt. Die produkthaltigen Fraktionen werden vereinigt und eingedampft. Nach Trocknen im Hochvakuum erhält man die Titelverbindung als farbloses Pulver. $R_f$(E) = 0,18.

Beispiel 53:

BOC-Phe-His-(2(S)-amino-4-methyl-pentyl)-2(R,S)-amino-4-hydroxybutyryl-Ile-His-$NH_2$

94,2 mg BOC-Phe-His-(2(S)-amino-4-methyl-pentyl)-2(R,S)-amino-4-benzyloxybutyryl-Ile-His-$NH_2$ werden in 10 ml Methanol/Essigsäure (9:1) gelöst und in Gegenwart von 50 mg Palladium-Kohle (10% Pd) während 68 Std. hydriert, wobei nach jeweils 24 Std. weitere 50 mg Pd-Kohle zugegeben werden. Der Katalysator wird abfiltriert, das Filtrat eingedampft und der Rückstand durch Flash-Chromatographie mit System O gereinigt. Die produkthaltigen Fraktionen werden vereinigt und eingedampft. Nach Trocknen im Hochvakuum erhält man die Titelverbindung als farbloses Pulver. $R_f$(L) = 0,37 und 0,42; $R_f$(E) = 0,15 und 0,20.

Beispiel 54:

Analog der vorstehenden Beispiele werden erhalten:

H-Phe-His-Leu$\overset{red}{—}$Val-Ile-Phe-$NH_2$, $R_f$(Q) = 0,30.

H-Phe-His-Leu$\overset{red}{—}$Val-Ile-Phe-$OCH_3$, $R_f$(O) = 0,51.

H-Phe-Gly-Leu$\overset{red}{—}$Val-Ile-His-$NH_2$, $R_f$(C) = 0,63.

Z-Phe-Ala-Leu$\overset{\text{red}}{\text{——}}$Val-Ile-His-$NH_2$, $R_f(S) = 0,37$.

Z-Phe-His-Leu$\overset{\text{red}}{\text{——}}$Val-Ile-His-2-hydroxyäthylamid, $R_f(T) = 0,28$.

Z-Phe-His-Phe$\overset{\text{red}}{\text{——}}$Phe-Ile-His-$NH_2$, $R_f(T) = 0,38$.

α-Carboxymethyl-ß,ß-dimethylacryloyl-Phe-His-Leu$\overset{\text{red}}{\text{——}}$Val-Ile-His-$NH_2$, $R_f(L) = 0,10$.

Z-Phe-His-(2(S)-amino-4-methyl-pentyl)-(2R,S)-2-amino-2-phenyl-acetyl-Ile-His-$NH_2$, $R_f(T) = 0,25$.

Beispiel 55: Gelatine-Lösung

Eine sterilfiltrierte wässrige Lösung von Z-Phe-His-Leu$\overset{\text{red}}{\text{——}}$Val-Ile-His-$NH_2$ wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| Z-Phe-His-Leu$\overset{\text{red}}{\text{——}}$Val-Ile-His-$NH_2$ | 3 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel 56: Sterile Trockensubstanz zur Injektion

Man löst 5 mg Z-Phe-His-Leu$\overset{\text{red}}{\text{——}}$Val-Ile-His-$NH_2$ in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel 57: Nasenspray

In einer Mischung von 3,5 ml "Myglyol 812" und 0,08 g Benzylalkohol werden 500 mg fein gemahlenes (<5,0 µm) Z-Phe-His-Leu$\overset{red}{—}$Val-Ile-His-$NH_2$ suspendiert. Diese Suspension wird in einen Behälter mit Dosierventil eingefüllt. Es werden 5,0 g "Freon 12" unter Druck durch das Ventil in den Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

<u>Patentansprüche</u> für die Vertragsstaaten: BE, DE, FR, IT, CH, LI, LU, NL, SE, GB

1. Substituierte Aethylendiaminderivate der Formel

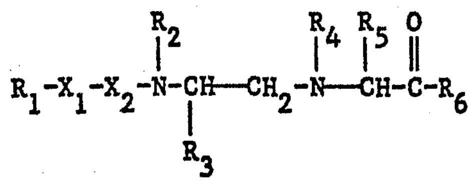

(I),

worin $R_1$ Wasserstoff oder Acyl, $X_1$ einen gegebenenfalls N-alkylierten natürlichen Aminosäurerest, der N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2-$ verbunden ist, $R_2$ Wasserstoff oder Niederalkyl, $R_3$ und $R_5$ unabhängig voneinander Wasserstoff, unsubstituiertes oder substituiertes Niederalkyl, unsubstituiertes oder substituiertes Arylniederalkyl oder Aryl, $R_4$ Wasserstoff, Niederalkyl oder Acyl und $R_6$ substituiertes Amino oder substituiertes Hydroxy darstellen, mit Ausnahme der Verbindungen, worin $R_1$ einen gegebenenfalls N-acylierten Rest der Aminosäure L-Prolin oder worin $X_1$ und $X_2$ zusammen -Arg-Pro-, -Tyr-Gly- oder -Gly-Gly- bedeuten, ferner Salze von solchen Verbindungen mit salzbildenden Gruppen.

2. Verbindungen gemäss Anspruch 1 der Formel I, worin $R_1$ und $R_4$ unabhängig voneinander Wasserstoff oder Acyl mit den Teilformeln $R^b-CO-$, $R^a-O-CO-$ oder $(R^b)(R^b)N-CO-$, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18 C-Atomen oder einen unsubstituierten oder substituierten, aromatischen, heteroaromatischen, aromatisch-aliphatischen oder heteroaromatisch-aliphatischen Kohlenwasserstoffrest bis einschliesslich 18 C-Atomen oder einen unsubstituierten oder substituierten, gesättigten, fünf-oder sechsgliedrigen Heterocyclus darstellt und $R^b$ Wasserstoff bedeutet oder die Bedeutungen von $R^a$ hat, $X_1$ einen natürlichen Aminosäurerest, der

N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2$-verbunden ist, $R_2$ Wasserstoff, $R_3$ und $R_5$ unabhängig voneinander Wasserstoff, unsubstituiertes oder substituiertes Niederalkyl, unsubstituiertes oder substituiertes Arylniederalkyl oder Aryl und $R_6$ eine Amino-oder Hydroxygruppe, welche durch einen oder gegebenenfalls zwei unsubstituierte oder substituierte, gesättigte oder ungesättigte, aliphatische Kohlenwasserstoffreste bis einschliesslich 18 C-Atomen oder einen aromatischen, heteroaromatischen, aromatisch-aliphatischen oder heteroaromatisch-aliphatischen Kohlenwasserstoffrest bis einschliesslich 18 C-Atomen substituiert ist, oder einen N-terminal mit der Gruppe -CO- verbundenen und C-terminal gegebenenfalls amidierten oder veresterten Rest einer natürlichen α-Aminosäure oder einen N-terminal mit der Gruppe -CO-verbundenen und C-terminal gegebenenfalls amidierten oder veresterten Di- oder Tripeptidrest, der aus natürlichen α-Aminosäuren und gewünschtenfalls aus Statin besteht, darstellen, mit Ausnahme der Verbindungen, worin $R_1$ einen gegebenenfalls N-acylierten Rest der Aminosäure L-Prolin oder worin $X_1$ und $X_2$ zusammen -Arg-Pro-, -Tyr-Gly- oder -Gly-Gly- bedeuten, ferner pharmazeutisch annehmbare Salze von Verbindungen der Formel I mit salzbildenden Gruppen.

3. Verbindungen gemäss Anspruch 1 der Formel I, worin $R_1$ und $R_4$ unabhängig voneinander Wasserstoff oder Acyl mit den Teilformeln $R^b$-CO-, $R^a$-O-CO-, $(R^b)(R^b)$N-CO-, $(R^b)(R^b)$-N-CS-, $R^b$-$SO_2$-oder $(R^b)(R^b)$N-$SO_2$-, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18, bevorzugt bis einschliesslich 10, C-Atomen oder einen unsubstituierten oder substituierten, aromatischen oder aromatischh-aliphatischen Kohlenwasserstoffrest bis einschliesslich 18, bevorzugt bis einschliesslich 10, C-Atomen oder einen unsubstituierten oder substituierten, fünf- oder sechsgliedrigen Heterocyclus darstellt und $R^b$ Wasserstoff bedeutet oder die Bedeutungen

von $R^a$ hat, $X_1$ einen natürlichen Aminosäurerest, der N-terminal mit $R_1$ und C-terminal mit $X_2$ verbunden ist, $X_2$ einen natürlichen Aminosäurerest, der N-terminal mit $X_1$ und C-terminal mit der Gruppe $-NR_2$-verbunden ist, $R_2$ Wasserstoff, $R_3$ und $R_5$ unabhängig voneinander Wasserstoff, Niederalkyl, Arylniederalkyl oder Aryl und $R_6$ eine Amino- oder Hydroxygruppe, welche durch einen unsubstituierten oder substituierten, gesättigten oder ungesättigten, aliphatischen Kohlenwasserstoffrest bis einschliesslich 18, bevorzugt bis einschliesslich 10, C-Atomen oder einen aromatischen, aromatisch-aliphatischen Kohlenwasserstoffrest bis einschliesslich 18, bevorzugt bis einschliesslich 10, C-Atomen substituiert ist, oder einen N-terminal mit der Gruppe -CO- verbundenen und C-terminal gegebenenfalls amidierten oder veresterten Rest einer Aminosäure oder einen N-terminal mit der Gruppe -CO- verbundenen und C-terminal gegebenenfalls amidierten oder veresterten Di-oder Tripeptidrest, darstellen, mit Ausnahme der Verbindungen, worin $R_1$ einen gegebenenfalls N-acylierten Rest der Aminosäure L-Prolin oder worin $X_1$ und $X_2$ zusammen -Arg-Pro-, Tyr-Gly- oder -Gly-Gly- bedeuten, ferner pharmazeutisch annehmbare Salze von Verbindungen der Formel I mit salzbildenden Gruppen.

4. Verbindungen gemäss Anspruch 1 oder 2 der Formel I, worin $R_1$ Wasserstoff, Alkanoyl, Niederalkoxyniederalkanoyl, Niederalkoxyniederalkoxyniederalkoxyniederalkanoyl, Halogenniederalkanoyl, Carboxyniederalkanoyl, Niederalkoxycarbonylniederalkanoyl, Diniederalkylaminoniederalkanoyl, Niederalkenoyl, Niederalkinoyl, Cycloalkylcarbonyl, Benzoyl oder durch Halogen, Niederalkoxy und/oder Nitro substituiertes Benzoyl, Pyrrolyl-2-carbonyl, 5-Oxopyrrolidinyl-2-carbonyl, Niederalkoxycarbonyl, 2-Halogenniederalkoxycarbonyl, 1-Adamantyloxycarbonyl, Arylniederalkoxycarbonyl mit einem oder zwei Arylresten, worin Aryl gegebenenfalls durchNiederalkyl, Niederalkoxy, Hydroxy, Halogen und/oder Nitro mono-, di- oder trisubstituiertes Phenyl ist, ferner Niederalkylaminocarbonyl,

$X_1$ Phenylalanin, welches N-terminal mit $R_1$ verbunden ist, $X_2$ Histidin, Glycin, Alanin oder Phenylalanin, welche am N-Atom durch Niederalkyl substituiert sein können und N-terminal mit $X_1$ und C-terminal mit der Gruppe $-NR_2$-verbunden sind, $R_2$ Wasserstoff oder Niederalkyl, $R_3$ und $R_5$ unabhängig voneinander Niederalkyl, Hydroxyniederalkyl, veräthertes Hydroxyniederalkyl, verestertes Hydroxyniederalkyl, Arylniederalkyl, Aryl-hydroxy-niederalkyl oder Phenyl, $R_4$ Wasserstoff und

$R_6$ Alkylamino, Diniederalkylamino, Hydroxyniederalkylamino, Niederalkoxyniederalkylamino, Carboxyalkylamino, Niederalkoxycarbonylalkylamino, physiologisch spaltbares verestertes Carboxyalkylamino, Aminoalkylamino, Diniederalkylaminoniederalkylamino, Acylaminoniederalkylamino, Carboxybenzylamino, Niederalkoxycarbonylbenzylamino, Carbamoylbenzylamino, Niederalkoxy, oder physiologisch spaltbares Alkoxy, ferner Histidin, Phenylalanin, Alanin oder Isoleucin, C-terminal gewünschtenfalls substituiert durch Amino, Alkylamino, Diniederalkylamino, Hydroxyniederalkylamino, Carboxyalkylamino, Niederalkoxycarbonylalkylamino, physiologisch spaltbares, verestertes Carboxyalkylamino, Aminoalkylamino, Diniederalkylaminoniederalkylamino, über ein Stickstoff-Atom gebundenes gesättigtes, fünf- oder sechsgliedriges Heterocyclylniederalkylamino, Acylaminoniederalkylamino, Phenylniederalkylamino, Carboxybenzylamino, Carbamoylbenzylamino, Pyridylniederalkylamino, Imidazolylniederalkylamino, 1-Benzylpiperidin-2-, -3- oder -4-ylamino, Niederalkoxy, oder physiologisch spaltbares substituiertes Alkoxy, ferner -Ile-His-, Ile-Phe-, -Ala-His-, -Gly-Gly-, -Gly-His-, -Gly-Ala-, -Ala-Sta-, -Ile-Sta-, -Gly-Gly-Gly-, -Gly-His-Lys-, -Gly-Ala-Gly- oder Ile-His-Lys, worin die Aminosäurereste N-terminal durch Niederalkyl substituiert sein können und worin die Aminofunktion in der Seitenkette von -Lys- gewünschtenfalls durch Niederalkanoyl, durch Niederalkoxycarbonyl, oder durch Arylniederalkoxycarbonyl acyliert ist, C-terminal gewünschtenfalls substituiert durch Amino, Alkylamino, Diniederalkylamino, Hydroxyniederalkylamino, Aminoalkylamino, Acylaminoalkylamino, Niederalkoxy, oder physiologisch spaltbares substituiertes Alkoxy,

darstellt, sowie pharmazeutisch annehmbare Salze von diesen Verbindungen mit salzbildenden Gruppen.

5. Verbindungen gemäss Anspruch 1, 2 oder 4 der Formel I, worin $R_1$ Wasserstoff, Niederalkanoyl, 2-(2-Methoxyäthoxy)-äthoxyacetyl, Carboxyniederalkanoyl, Niederalkoxycarbonylniederalkanoyl, Diniederalkylaminoniederalkanoyl, α-Carboxymethyl-ß,ß-dimethyl-acryloyl, Cycloalkylcarbonyl, Benzoyl, Pyrrolyl-2-carbonyl, 5-Oxopyrrolidinyl-2-carbonyl, Niederalkoxycarbonyl, 2-Halogenniederalkoxycarbonyl, 1-Adamantyloxycarbonyl, Benzyloxycarbonyl oder Niederalkylaminocarbonyl,

$X_1$ und $X_2$ zusammen -Phe-His-, -Phe-(N-methyl-His)- oder Phe-Gly, welche N-terminal mit $R_1$ und C-terminal mit der Gruppe $-NR_2-$ verbunden sind, $R_2$ Wasserstoff oder Niederalkyl, $R_3$ Isobutyl, Benzyl oder α-Hydroxybenzyl, $R_4$ Wasserstoff, $R_5$ Methyl, Isopropyl, Hydroxymethyl, 2-Hydroxyäthyl, 2-Benzyloxyäthyl, Benzyl oder Phenyl, und

$R_6$ Alkylamino, Hydroxyniederalkylamino, Carboxyalkylamino, Niederalkoxycarbonylalkylamino, Carboxybenzylamino, Niederalkoxycarbonylbenzylamino, ferner Isoleucin, C-terminal substituiert durch Amino, Alkylamino, Hydroxyniederalkylamino, Aminoalkylamino, Diniederalkylaminoniederalkylamino, Morpholin-4-yl-niederalkylamino, Benzylamino, Carboxybenzylamino, Niederalkoxycarbonylbenzylamino, Carbamoylbenzylamino, Imidazolylniederalkylamino, 1-Benzylpiperidin-2-, -3- oder -4-ylamino, ferner -Ile-His-, -Ile-Phe-, -Gly-Gly-, -Gly-(N-methyl-Gly)-, -Ala-Sta-, -Ile-Sta-, -Gly-Gly-Gly- oder -Gly-His-Lys-, worin die Amino-Funktion in der Seitenkette von -Lys- gewünschtenfalls

durch tert-Butoxycarbonyl oder Benzyloxycarbonyl acyliert ist, C-terminal substituiert durch Amino, Alkylamino, Hydroxyniederalkylamino, Aminoalkylamino, tert-Butoxycarbonylaminoalkylamino, Benzyloxycarbonylamino-alkylamino oder Niederalkoxy,

darstellt, sowie pharmazeutisch annehmbare Salze von diesen Verbindungen mit salzbildenden Gruppen.

6. Verbindungen gemäss Anspruch 1, 2, 4 oder 5 der Formel I, worin $R_1$ Wasserstoff, Niederalkanoyl, 2-(2-Methoxyäthoxy)-äthoxyacetyl, Diniederalkylaminoniederalkanoyl, α-Carboxymethyl-ß,ß-dimethylacryloyl, Cycloalkylcarbonyl, Benzoyl, Pyrrolyl-2-carbonyl, 5-Oxopyrrolidinyl-2-carbonyl, Niederalkoxycarbonyl, 1,1-Dimethyl-2,2,2-trichloräthoxycarbonyl, 1-Adamantyloxycarbonyl, Benzyloxycarbonyl oder Niederalkylaminocarbonyl, $X_1$ und $X_2$ zusammen -Phe-His- oder -Phe-(N-methyl-His)-, welche N-terminal mit $R_1$ und C-terminal mit der Gruppe $-NR_2-$ verbunden sind, $R_2$ Wasserstoff oder Niederalkyl, $R_3$ Isobutyl, Benzyl oder α-Hydroxybenzyl, $R_4$ Wasserstoff, $R_5$ Methyl, Isopropyl, Hydroxymethyl, 2-Hydroxyäthyl, 2-Benzyloxyäthyl, Benzyl oder Phenyl, und $R_6$ Carboxyalkylamino, Niederalkoxycarbonylalkylamino, Carboxybenzylamino, Niederalkoxycarbonylbenzylamino, ferner Isoleucin, C-terminal substituiert durch Amino, Alkylamino, Hydroxyniederalkylamino, Diniederalkylaminoniederalkylamino, Morpholin-4-yl-niederalkylamino oder Carbamoylbenzylamino, ferner -Ile-His-, -Ile-Phe-, -Gly-Gly-, -Gly-(N-methyl-Gly)- oder -Ile-Sta-, C-terminal substituiert durch Amino, Alkylamino oder Hydroxyniederalkylamino, oder -Ala-Sta-, C-terminal substituiert durch Niederalkoxy, darstellt, sowie pharmazeutisch annehmbare Salze von diesen Verbindungen mit salzbildenden Gruppen.

7. Verbindungen gemäss einem der Ansprüche 1 bis 6 der Formel I, worin $R_1$ Wasserstoff, Niederalkanoyl, 2-(2-Methoxyäthoxy)-äthoxyacetyl, Diniederalkylaminoniederalkanoyl, Cycloalkylcarbonyl, Benzoyl, 5-Oxopyrrolidinyl-2-carbonyl, Niederalkoxycarbonyl, 1-Adamantyloxycarbonyl, Benzyloxycarbonyl oder Niederalkylaminocar-

bonyl, $X_1$ und $X_2$ zusammen -Phe-His-, welche N-terminal mit $R_1$ und C-terminal mit der Gruppe $-NR_2-$ verbunden sind, $R_2$ und $R_4$ Wasserstoff, $R_3$ Isobutyl, $R_5$ Isopropyl, $R_6$ Isoleucin, C-terminal substituiert durch Morpholin-4-yl-niederalkylamino oder Carbamoylbenzylamino, oder -Ile-His-, -Ile-Phe- oder -Ile-Sta-, C-terminal substituiert durch Amino oder Hydroxyniederalkylamino, oder -Ala-Sta-, C-terminal substituiert durch Niederalkoxy, darstellt, sowie pharmazeutisch annehmbare Salze von diesen Verbindungen mit salzbildenden Gruppen.

8. Benzyloxycarbonyl-Phe-His-(2(S)-amino-4-methyl-pentyl)-Val-Ile-His-$NH_2$ gemäss einem der Ansprüche 1 bis 7.

9. tert-Butylaminocarbonyl-Phe-His-(2(S)-amino-4-methyl-pentyl)-Val-Ile-His-$NH_2$ gemäss einem der Ansprüche 1 bis 7.

10. Benzyloxycarbonyl-Phe-His-(2(S)-amino-4-methyl-pentyl)-Val-Ile-3-carbamoylbenzylamid gemäss einem der Ansprüche 1 bis 7.

11. Benzyloxycarbonyl-Phe-His-(2(S)-amino-4-methyl-pentyl)-Val-Ala-Sta-$OCH_3$ gemäss einem der Ansprüche 1 bis 7.

12. tert-Butoxycarbonyl-Phe-His-(2(S)-amino-4-methyl-pentyl)-Val-Ile-Sta-$NH_2$ gemäss einem der Ansprüche 1 bis 7.

13. Pivaloyl-Phe-His-(2(S)-amino-4-methyl-pentyl)-Val-Ile-His-$NH_2$, gemäss einem der Ansprüche 1 bis 7.

14. tert-Butoxycarbonyl-Phe-His-(2(S)-amino-4-methyl-pentyl)-Ser-Ile-His-$NH_2$ gemäss einem der Ansprüche 1, 2, 4, 5 oder 6.

15. Pharmazeutische Präparate enthaltend Verbindungen der Formel I oder pharmazeutisch verwendbare Salze von Verbindungen der Formel I mit salzbildenden Gruppen zusammen mit einem pharmazeutischen Trägermaterial.

16. Verbindungen der Formel I oder pharmazeutisch verwendbare Salze von Verbindungen der Formel I zur Anwendung in einem Verfahren zur therapeutischen Behandlung des menschlichen oder tierischen Körpers.

17. Verwendung von Verbindungen der Formel I oder deren pharmazeutisch verwendbaren Salzen zur Hemmung der Wirkung des Enzyms Renin.

18. Verwendung von Verbindungen der Formel I, deren Hydraten oder deren pharmazeutisch verwendbaren Salzen zur Herstellung von pharmazeutischen Präparaten.

19. Verfahren zur Herstellung von Verbindungen der Formel I, worin die Substituenten die in Anspruch 1 genannten Bedeutungen haben, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung einer Verbindung der Formel I, worin $R_6$ substituiertes Amino darstellt, in einer Verbindung der Formel

$$R_1 - X_1 - X_2 - \underset{}{\overset{R_2}{\overset{|}{N}}} - \underset{\underset{R^3}{|}}{CH} - CH_2 - \overset{R_4}{\overset{|}{N}} - \overset{R_5}{\overset{|}{CH}} - CN \quad (III),$$

bzw. in einer Verbindung der Formel

$$R_1 - X_1 - X_2 - \overset{R_2}{\overset{|}{N}} - \underset{\underset{R_3}{|}}{CH} - CH_2 - \overset{R_4}{\overset{|}{N}} - \overset{R_5}{\overset{|}{CH}} - \overset{O}{\overset{\|}{C}} - R'_6 - CN \quad (IV),$$

worin die Gruppe $R_6'$-CN den Rest einer natürlichen α-Aminosäure, von Statin, eines Dipeptids oder eines Tripeptids, worin jeweils die terminale -COOH-Gruppe durch -CN ersetzt ist, bedeutet, die übrigen Substituenten die genannten Bedeutungen haben und vorhandene funktionelle Gruppen gegebenenfalls in geschützter Form vorliegen, die Cyanogruppe in eine N-substituierte oder gegebenenfalls unsubstituierte Carboxamidogruppe überführt, oder

c) eine Verbindung der Formel

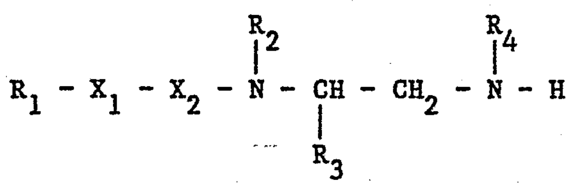 (V)

mit einem α-substituierten Carbonsäurederivat der Formel

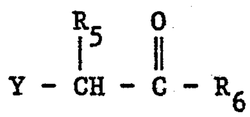 (VI),

worin die Substituenten die angegebenen Bedeutungen haben, Y eine Abgangsgruppe bedeutet und freie funktionelle Gruppen gegebenenfalls in geschützter Form vorliegen, umsetzt, oder

d) eine Verbindung der Formel

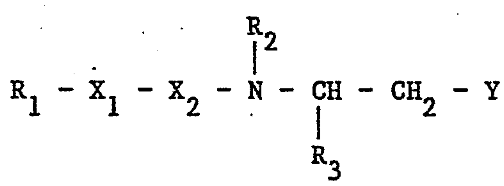 (VII)

mit einem α-Aminocarbonsäurederivat der Formel

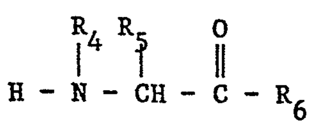 (VIII),

worin die Substituenten die genannten Bedeutungen haben, Y eine Abgangsgruppe bedeutet und freie funktionelle Gruppen gegebenenfalls in geschützter Form vorliegen, umsetzt, oder

e) eine Verbindung der Formel V mit einem α-Oxocarbonsäurederivat der Formel

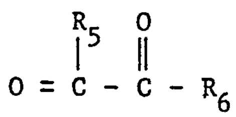 (IX),

worin die Substituenten die genannten Bedeutungen haben und freie funktionelle Gruppen gegebenenfalls in geschützter Form vorliegen, kondensiert und das Kondensationsprodukt mit einem Reduktionsmittel behandelt, oder

f) eine Verbindung der Formel

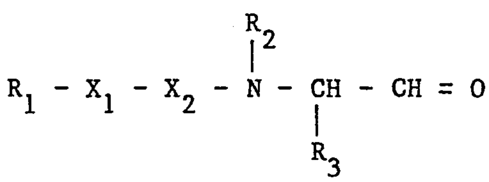 (X)

mit einem α-Aminocarbonsäurederivat der Formel VIII, worin die Substituenten die genannten Bedeutungen haben und freie funktionelle Gruppen gegebenenfalls in geschützter Form vorliegen, kondensiert und das Kondensationsprodukt mit einem Reduktionsmittel behandelt,

und gewünschtenfalls in einer erhältlichen Verbindung vorhandene Schutzgruppen abspaltet und/oder gewünschtenfalls nach Ausführung eines der vorstehend genannten Verfahren oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

20. Die nach einem der Verfahren des Anspruchs 19 erhältlichen Verbindungen und ihre Salze.

21. Verbindungen der Formel

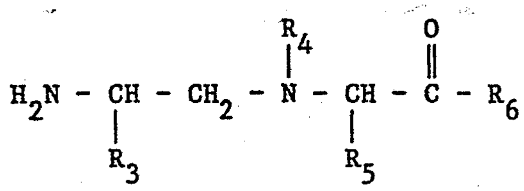 (XI),

worin $R_3$, $R_4$, $R_5$ und $R_6$ die unter Formel I genannten Bedeutungen haben.

22. Verfahren zur Herstellung von Verbindungen der Formel XI, worin die Substituenten die genannten Bedeutungen haben, dadurch gekennzeichnet, dass man eine Verbindung der Formel

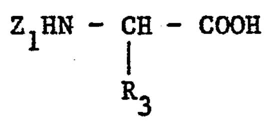 (XII),

worin $Z_1$ eine Aminoschutzgruppe ist und $R_3$ die unter Formel I genannten Bedeutungen hat, oder ein reaktionsfähiges, funktionelles Derivat davon mit einer Verbindung der Formel

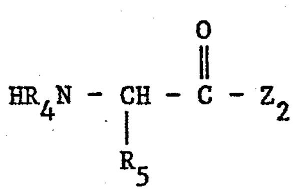 (XIII),

worin $R_4$ und $R_5$ die genannten Bedeutungen haben und $Z_2$ eine Carboxyschutzgruppe ist, unter Bildung einer Amidbindung kondensiert und in einer erhältlichen Verbindung der Formel

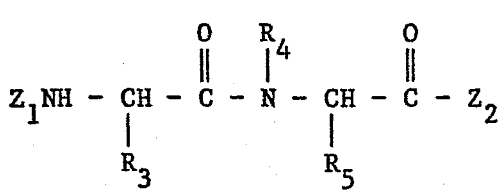 (XIV),

worin die Substituenten die genannten Bedeutungen haben, die Carbonylgruppe der Amidbindung zu einer $-CH_2-$ Gruppe reduziert, in einer erhältlichen Verbindung der Formel

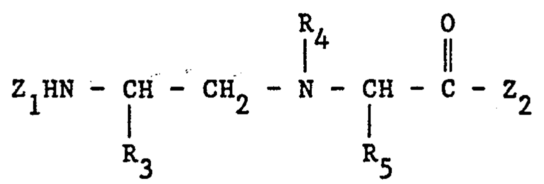

(XV)

in beliebiger Reihenfolge der Reaktionsschritte oder gleichzeitig die Schutzgruppen $Z_1$ und $Z_2$ abspaltet, gewünschtenfalls ein erhältliches Isomerengemisch in die einzelnen Isomere auftrennt und die erhältliche Verbindung mit einer freien Carboxygruppe je nach Bedeutung des einzuführenden Rests $R_6$ amidiert, verestert oder mit einem C-terminal gegebenenfalls amidierten oder veresterten Rest einer Aminosäure, eines Dipeptids oder eines Tripeptids substituiert.

FO 7.4/KB/gs*

0144290

Patentansprüche für den Vertragsstaat: AT

1. Verfahren zur Herstellung von substituierten Aethylendiaminderivaten der Formel

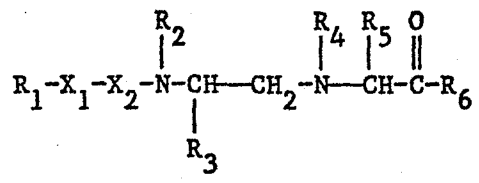

(I),

worin $R_1$ Wasserstoff oder Acyl, $X_1$ einen gegebenenfalls N-alkylierten natürlichen Aminosäurerest, der N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2-$ verbunden ist, $R_2$ Wasserstoff oder Niederalkyl, $R_3$ und $R_5$ unabhängig voneinander Wasserstoff, unsubstituiertes oder substituiertes Niederalkyl, unsubstituiertes oder substituiertes Arylniederalkyl oder Aryl, $R_4$ Wasserstoff, Niederalkyl oder Acyl und $R_6$ substituiertes Amino oder substituiertes Hydroxy darstellen, mit Ausnahme der Verbindungen, worin $R_1$ einen gegebenenfalls N-acylierten Rest der Aminosäure L-Prolin oder worin $X_1$ und $X_2$ zusammen -Arg-Pro-, -Tyr-Gly- oder -Gly-Gly- bedeuten, ferner von Salzen von solchen Verbindungen mit salzbildenden Gruppen, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

 

b) zur Herstellung einer Verbindung der Formel I, worin $R_6$ substituiertes Amino darstellt, in einer Verbindung der Formel

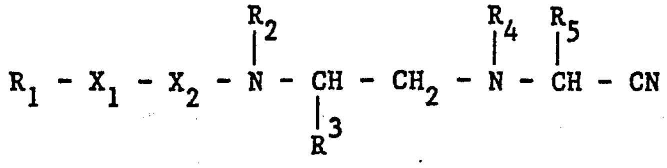 (III),

bzw. in einer Verbindung der Formel

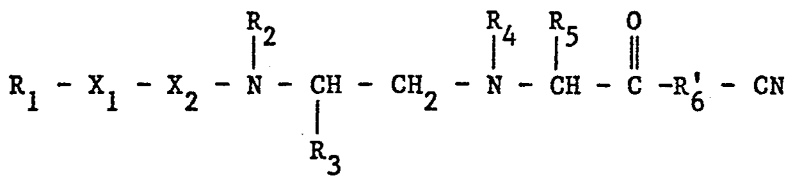 (IV),

worin die Gruppe $R_6'$-CN den Rest einer natürlichen α-Aminosäure, von Statin, eines Dipeptids oder eines Tripeptids, worin jeweils die terminale -COOH-Gruppe durch -CN ersetzt ist, bedeutet, die übrigen Substituenten die genannten Bedeutungen haben und vorhandene funktionelle Gruppen gegebenenfalls in geschützter Form vorliegen, die Cyanogruppe in eine N-substituierte oder gegebenenfalls unsubstituierte Carboxamidogruppe überführt, oder

c) eine Verbindung der Formel

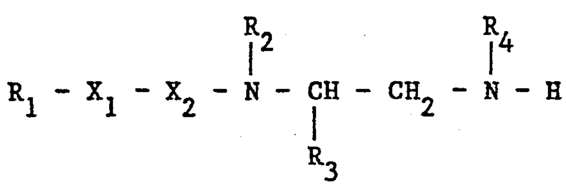 (V)

mit einem α-substituierten Carbonsäurederivat der Formel

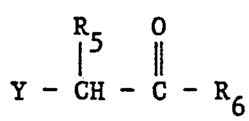 (VI),

worin die Substituenten die angegebenen Bedeutungen haben, Y eine Abgangsgruppe bedeutet und freie funktionelle Gruppen gegebenenfalls in geschützter Form vorliegen, umsetzt, oder

d) eine Verbindung der Formel

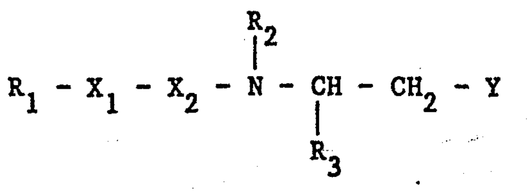 (VII)

mit einem α-Aminocarbonsäurederivat der Formel

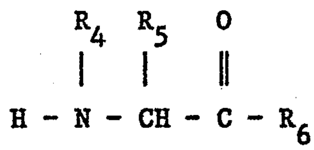 (VIII),

worin die Substituenten die genannten Bedeutungen haben, Y eine Abgangsgruppe bedeutet und freie funktionelle Gruppen gegebenenfalls in geschützter Form vorliegen, umsetzt, oder

e) eine Verbindung der Formel V mit einem α-Oxocarbonsäurederivat der Formel

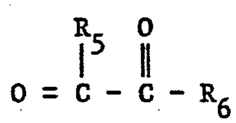 (IX),

worin die Substituenten die genannten Bedeutungen haben und freie funktionelle Gruppen gegebenenfalls in geschützter Form vorliegen, kondensiert und das Kondensationsprodukt mit einem Reduktionsmittel behandelt, oder

f) eine Verbindung der Formel

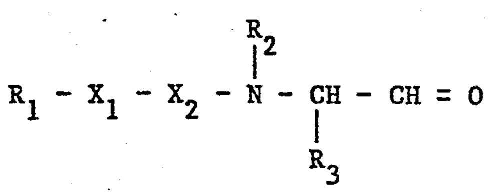 (X)

mit einem α-Aminocarbonsäurederivat der Formel VIII, worin die Substituenten die genannten Bedeutungen haben und freie funktionelle Gruppen gegebenenfalls in geschützter Form vorliegen, kondensiert und das Kondensationsprodukt mit einem Reduktionsmittel behandelt,

und gewünschtenfalls in einer erhältlichen Verbindung vorhandene Schutzgruppen abspaltet und/oder gewünschtenfalls nach Ausführung eines der vorstehend genannten Verfahren oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

2. Verfahren zur Herstellung von Verbindungen der Formel I gemäss Anspruch 1, dadurch gekennzeichnet, dass man ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert und gewünschtenfalls in einer erhältlichen Verbindung vorhandene Schutzgruppen durch Behandlung mit Säure oder mit Base oder durch Hydrogenolyse abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

3. Verfahren zur Herstellung von Verbindungen der Formel I gemäss Anspruch 1, dadurch gekennzeichnet, dass man ein Fragment einer Verbindung der Formel I mit einer endständigen Carboxygruppe oder ein davon abgeleiteter aktivierter Ester, reaktionsfähiges Anhydrid oder reaktionsfähiges cyclisches Amid mit einem zur Verbindung der Formel I komplementären Fragment mit einer freien Aminogruppe, wobei in den Reaktionskomponenten vorhandene freie funktionelle Gruppen mit Ausnahme der an der Reaktion teilnehmenden Gruppen gegebenenfalls in geschützter Form vorliegen, in Gegenwart eines Kondensationsmittels ausgewählt aus einem Carbodiimid, Carbonyldiimidazol, 1,2-Oxazoliumverbindungen, einem 1,2-Dihydrochinolin oder einem aktivierten Phosphat, und gewünschtenfalls in Gegenwart einer organischen Base oder von Alkalimetallcarbonaten in einem inerten, polaren, aprotischen Lösungsmittel in einem Temperaturbereich von -40°C bis +100°C kondensiert, wobei aktivierte Ester vom Amino- oder Amidoester-Typ gewünschtenfalls in situ durch Zugabe einer N-Hydroxyamino- oder N-Hydroxyamido-Verbindung gebildet werden, und gewünschtenfalls in einer erhältlichen Verbindung vorhandene Schutzgruppen durch Behandlung mit Säure oder mit Base oder durch Hydrogenolyse abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

4. Verfahren gemäss einem der Ansprüche 1-3, dadurch gekennzeichnet, dass man solche Ausgangsstoffe wählt, dass Verbindungen der Formel I, worin $R_1$ und $R_4$ unabhängig voneinander Wasserstoff oder Acyl mit den Teilformeln $R^b$-CO-, $R^a$-O-CO- oder $(R^b)(R^b)$N-CO-, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18 C-Atomen oder einen unsubstituierten oder substituierten, aromatischen, heteroaromatischen, aromatisch-aliphatischen oder heteroaromatisch-aliphatischen Kohlenwasserstoffrest bis einschliesslich 18 C-Atomen oder einen unsubstituierten oder substituierten, gesättigten, fünf- oder sechsgliedrigen Heterocyclus darstellt und $R^b$ Wasserstoff bedeutet oder die Bedeutungen von $R^a$ hat, $X_1$ einen natürlichen Aminosäurerest, der N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2-$verbunden ist, $R_2$ Wasserstoff, $R_3$ und $R_5$ unabhängig voneinander Wasserstoff, unsubstituiertes oder substituiertes Niederalkyl, unsubstituiertes oder substituiertes Arylniederalkyl oder Aryl und $R_6$ eine Amino-oder Hydroxygruppe, welche durch einen oder gegebenenfalls zwei unsubstituierte oder substituierte, gesättigte oder ungesättigte, aliphatische Kohlenwasserstoffreste bis einschliesslich 18 C-Atomen oder einen aromatischen, heteroaromatischen, aromatisch-aliphatischen oder heteroaromatisch-aliphatischen Kohlenwasserstoffrest bis einschliesslich 18 C-Atomen substituiert ist, oder einen N-terminal mit der Gruppe -CO- verbundenen und C-terminal gegebenenfalls amidierten oder veresterten Rest einer natürlichen α-Aminosäure oder einen N-terminal mit der Gruppe -CO-verbundenen und C-terminal gegebenenfalls amidierten oder veresterten Di- oder Tripeptidrest, der aus natürlichen α-Aminosäuren und gewünschtenfalls aus Statin besteht, darstellen, mit Ausnahme der Verbindungen, worin $R_1$ einen gegebenenfalls N-acylierten Rest der Aminosäure L-Prolin oder worin $X_1$ und $X_2$ zusammen -Arg-Pro-, -Tyr-Gly- oder -Gly-Gly- bedeuten, ferner pharmazeutisch annehmbare Salze von Verbindungen der Formel I mit salzbildenden Gruppen, gebildet werden.

5. Verfahren gemäss einem der Ansprüche 1-3, dadurch gekennzeichnet, dass man solche Ausgangsstoffe wählt, dass Verbindungen der Formel I, worin $R_1$ und $R_4$ unabhängig voneinander Wasserstoff oder Acyl mit den Teilformeln $R^b$-CO-, $R^a$-O-CO-, $(R^b)(R^b)$N-CO-, $(R^b)(R^b)$-N-CS-, $R^b$-$SO_2$-oder $(R^b)(R^b)$N-$SO_2$-, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18, bevorzugt bis einschliesslich 10, C-Atomen oder einen unsubstituierten oder substituierten, aromatischen oder aromatischh-aliphatischen Kohlenwasserstoffrest bis einschliesslich 18, bevorzugt bis einschliesslich 10, C-Atomen oder einen unsubstituierten oder substituierten, fünf- oder sechsgliedrigen Heterocyclus darstellt und $R^b$ Wasserstoff bedeutet oder die Bedeutungen von $R^a$ hat, $X_1$ einen natürlichen Aminosäurerest, der N-terminal mit $R_1$ und C-terminal mit $X_2$ verbunden ist, $X_2$ einen natürlichen Aminosäurerest, der N-terminal mit $X_1$ und C-terminal mit der Gruppe $-NR_2-$verbunden ist, $R_2$ Wasserstoff, $R_3$ und $R_5$ unabhängig voneinander Wasserstoff, Niederalkyl, Arylniederalkyl oder Aryl und $R_6$ eine Amino- oder Hydroxygruppe, welche durch einen unsubstituierten oder substituierten, gesättigten oder ungesättigten, aliphatischen Kohlenwasserstoffrest bis einschliesslich 18, bevorzugt bis einschliesslich 10, C-Atomen oder einen aromatischen, aromatisch-aliphatischen Kohlenwasserstoffrest bis einschliesslich 18, bevorzugt bis einschliesslich 10, C-Atomen substituiert ist, oder einen N-terminal mit der Gruppe -CO- verbundenen und C-terminal gegebenenfalls amidierten oder veresterten Rest einer Aminosäure oder einen N-terminal mit der Gruppe -CO- verbundenen und C-terminal gegebenenfalls amidierten oder veresterten Di-oder Tripeptidrest, darstellen, mit Ausnahme der Verbindungen, worin $R_1$ einen gegebenenfalls N-acylierten Rest der Aminosäure L-Prolin oder worin $X_1$ und $X_2$ zusammen -Arg-Pro-, -Tyr-Gly- oder -Gly-Gly- bedeuten, fernerpharmazeutisch annehmbare Salze von Verbindungen der Formel I mit salzbildenden Gruppen, gebildet werden.

6. Verfahren gemäss einem der Ansprüche 1-3, dadurch gekennzeichnet, dass man solche Ausgangsstoffe wählt, dass Verbindungen der Formel I, worin $R_1$ Wasserstoff, Alkanoyl, Niederalkoxyniederalkanoyl, Niederalkoxyniederalkoxyniederalkoxyniederalkanoyl, Halogenniederalkanoyl, Carboxyniederalkanoyl, Niederalkoxycarbonylniederalkanoyl, Diniederalkylaminoniederalkanoyl, Niederalkenoyl, Niederalkinoyl, Cycloalkylcarbonyl, Benzoyl oder durch Halogen, Niederalkoxy und/oder Nitro substituiertes Benzoyl, Pyrrolyl-2-carbonyl, 5-Oxo-

pyrrolidinyl-2-carbonyl, Niederalkoxycarbonyl, 2-Halogenniederalkoxycarbonyl, 1-Adamantyloxycarbonyl, Arylniederalkoxycarbonyl mit einem oder zwei Arylresten, worin Aryl gegebenenfalls durch Niederalkyl, Hydroxy, Halogen und/oder Nitro mono-, di- oder trisubstituiertes Phenyl ist, ferner Niederalkylaminocarbonyl,

$X_1$ Phenylalanin, welches N-terminal mit $R_1$ verbunden ist, $X_2$ Histidin, Glycin, Alanin oder Phenylalanin, welche am N-Atom durch Niederalkyl substituiert sein können und N-terminal mit $X_1$ und C-terminal mit der Gruppe $-NR_2$-verbunden sind, $R_2$ Wasserstoff oder Niederalkyl, $R_3$ und $R_5$ unabhängig voneinander Niederalkyl, Hydroxyniederalkyl, veräthertes Hydroxyniederalkyl, verestertes Hydroxyniederalkyl, Arylniederalkyl, Aryl-hydroxy-niederalkyl oder Phenyl, $R_4$ Wasserstoff und

$R_6$ Alkylamino, Diniederalkylamino, Hydroxyniederalkylamino, Niederalkoxyniederalkylamino, Carboxyalkylamino, Niederalkoxycarbonylalkylamino, physiologisch spaltbares verestertes Carboxyalkylamino, Aminoalkylamino, Diniederalkylaminoniederalkylamino, Acylaminoniederalkylamino, Carboxybenzylamino, Niederalkoxycarbonylbenzylamino, Carbamoylbenzylamino, Niederalkoxy, oder physiologisch spaltbares Alkoxy, ferner Histidin, Phenylalanin, Alanin oder Isoleucin, C-terminal gewünschtenfalls substituiert durch Amino, Alkylamino, Diniederalkylamino, Hydroxyniederalkylamino, Carboxyalkylamino, -Niederalkoxycarbonylalkylamino, physiologisch spaltbares, verestertes Carboxyalkylamino, Aminoalkylamino, Diniederalkylaminoniederalkylamino, über ein Stickstoff-Atom gebundenes gesättigtes, fünf- oder sechsgliedriges Heterocyclylniederalkylamino, Acylaminoniederalkylamino, Phenylniederalkylamino, Carboxybenzylamino, Carbamoylbenzylamino, Pyridylniederalkylamino, Imidazolylniederalkylamino, -1-Benzylpiperidin-2-, -3- oder -4-ylamino, Niederalkoxy, oder physiologisch spaltbares substituiertes Alkoxy, ferner -Ile-His-, Ile-Phe-, -Ala-His-, -Gly-Gly-, -Gly-His-, -Gly-Ala-, -Ala-Sta-, -Ile-Sta-, -Gly-Gly-Gly-, -Gly-His-Lys-, -Gly-Ala-Gly- oder Ile-His-Lys, worin die Aminosäurereste N-terminal durch Niederalkyl

substituiert sein können und worin die Aminofunktion in der Seitenkette von -Lys- gewünschtenfalls durch Niederalkanoyl, durch Niederalkoxycarbonyl oder durch Arylniederalkoxycarbonyl acyliert ist, C-terminal gewünschtenfalls substituiert durch Amino, Alkylamino, Diniederalkylamino, Hydroxyniederalkylamino, Aminoalkylamino, Acylaminoalkylamino, oder physiologisch spaltbares substituiertes Alkoxy,

darstellt, sowie pharmazeutisch annehmbare Salze von diesen Verbindungen mit salzbildenden Gruppen, gebildet werden.

7. Verfahren gemäss einem der Ansprüche 1-3, dadurch gekennzeichnet, dass man solche Ausgangsstoffe wählt, dass Verbindungen der Formel I, worin $R_1$ Wasserstoff, Niederalkanoyl, 2-(2-Methoxyäthoxy)-äthoxyacetyl, Carboxyniederalkanoyl, Niederalkoxycarbonylniederalkanoyl, Diniederalkylaminoniederalkanoyl, α-Carboxymethyl-ß,ß-dimethylacryloyl, Cycloalkylcarbonyl, Benzoyl, Pyrrolyl-2-carbonyl, 5-Oxopyrrolidinyl-2-carbonyl, Niederalkoxycarbonyl, 2-Halogenniederalkoxycarbonyl, 1-Adamantyloxycarbonyl, Benzyloxycarbonyl oder Niederalkylaminocarbonyl,

$X_1$ und $X_2$ zusammen -Phe-His-, -Phe-(N-methyl-His)- oder Phe-Gly, welche N-terminal mit $R_1$ und C-terminal mit der Gruppe $-NR_2-$ verbunden sind, $R_2$ Wasserstoff oder Niederalkyl, $R_3$ Isobutyl, Benzyl oder α-Hydroxybenzyl, $R_4$ Wasserstoff, $R_5$ Methyl, Isopropyl, Hydroxymethyl, 2-Hydroxyäthyl, 2-Benzyloxyäthyl, Benzyl oder Phenyl, und

$R_6$ Alkylamino, Hydroxyniederalkylamino, Carboxyalkylamino, Niederalkoxycarbonylalkylamino, Carboxybenzylamino, Niederalkoxycarbonylbenzylamino, ferner Isoleucin, C-terminal substituiert durch Amino, Alkylamino, Hydroxyniederalkylamino, Aminoalkylamino, Diniederalkylaminoniederalkylamino, Morpholin-4-yl-niederalkylamino, Benzylamino, Carboxybenzylamino, Niederalkoxycarbonylbenzylamino, Carbamoylbenzylamino, Imidazolylniederalkylamino, 1-Benzylpiperidin-2-, -3- oder

-4-ylamino, ferner -Ile-His-, -Ile-Phe-, -Gly-Gly-, -Gly-(N-methyl-Gly)-, -Ala-Sta-, -Ile-Sta-, -Gly-Gly-Gly- oder -Gly-His-Lys-, worin die Amino-Funktion in der Seitenkette von -Lys- gewünschtenfalls durch tert-Butoxycarbonyl oder Benzyloxycarbonyl acyliert ist, C-terminal substituiert durch Amino, Alkylamino, Hydroxyniederalkyl-amino, Aminoalkylamino, tert-Butoxycarbonylaminoalkylamino, Benzyl-oxycarbonylamino-alkylamino oder Niederalkoxy,

darstellt, sowie pharmazeutisch annehmbare Salze von diesen Verbindungen mit salzbildenden Gruppen, gebildet werden.

8. Verfahren gemäss einem der Ansprüche 1-3, dadurch gekennzeichnet, dass man solche Ausgangsstoffe wählt, dass Verbindungen der Formel I, worin $R_1$ Wasserstoff, Niederalkanoyl, 2-(2-Methoxyäthoxy)-äthoxy-acetyl, Diniederalkylaminoniederalkanoyl, α-Carboxymethyl-ß,ß-dimethyl-acryloyl, Cycloalkylcarbonyl, Benzoyl, Pyrrolyl-2-carbonyl, 5-Oxopyrrolidinyl-2-carbonyl, Niederalkoxycarbonyl, 1,1-Dimethyl-2,2,2-trichloräthoxycarbonyl, 1-Adamantyloxycarbonyl, Benzyloxy-carbonyl oder Niederalkylaminocarbonyl, $X_1$ und $X_2$ zusammen -Phe-His-oder -Phe-(N-methyl-His)-, welche N-terminal mit $R_1$ und C-terminal mit der Gruppe $-NR_2-$ verbunden sind, $R_2$ Wasserstoff oder Nieder-alkyl, $R_3$ Isobutyl, Benzyl oder α-Hydroxybenzyl, $R_4$ Wasserstoff, $R_5$ Methyl, Isopropyl, Hydroxymethyl, 2-Hydroxyäthyl, 2-Benzyloxyäthyl, Benzyl oder Phenyl, und $R_6$ Carboxyalkylamino, Niederalkoxycarbonyl-alkylamino, Carboxybenzylamino, Niederalkoxycarbonylbenzylamino, ferner Isoleucin, C-terminal substituiert durch Amino, Alkylamino, Hydroxyniederalkylamino, Diniederalkylaminoniederalkylamino, Morpholin-4-yl-niederalkylamino oder Carbamoylbenzylamino, ferner -Ile-His-, -Ile-Phe-, -Gly-Gly-, -Gly-(N-methyl-Gly)- oder -Ile-Sta-, C-terminal substituiert durch Amino, Alkylamino oder Hydroxyniederalkylamino, oder -Ala-Sta-, C-terminal substituiert durch Niederalkoxy, darstellt, sowie pharmazeutisch annehmbare Salze von diesen Verbindungen mit salzbildenden Gruppen, gebildet werden.

9. Verfahren gemäss einem der Ansprüche 1-3, dadurch gekennzeichnet, dass man solche Ausgangsstoffe wählt, dass Verbindungen der Formel I, worin $R_1$ Wasserstoff, Niederalkanoyl, 2-(2-Methoxyäthoxy)-äthoxyacetyl, Diniederalkylaminoniederalkanoyl, Cycloalkylcarbonyl, Benzoyl, 5-Oxopyrrolidinyl-2-carbonyl, Niederalkoxycarbonyl, 1-Adamantyloxycarbonyl, Benzyloxycarbonyl oder Niederalkylaminocarbonyl, $X_1$ und $X_2$ zusammen -Phe-His-, welche N-terminal mit $R_1$ und C-terminal mit der Gruppe $-NR_2-$ verbunden sind, $R_2$ und $R_4$ Wasserstoff, $R_3$ Isobutyl, $R_5$ Isopropyl, $R_6$ Isoleucin, C-terminal substituiert durch Morpholin-4-yl-niederalkylamino oder Carbamoylbenzylamino, oder -Ile-His-, -Ile-Phe- oder -Ile-Sta-, C-terminal substituiert durch Amino oder Hydroxyniederalkylamino, oder -Ala-Sta-, C-terminal substituiert durch Niederalkoxy, darstellt, sowie pharmazeutisch annehmbare Salze von diesen Verbindungen mit salzbildenden Gruppen, gebildet werden.

10. Verfahren gemäss einem der Ansprüche 1-3, dadurch gekennzeichnet, dass man solche Ausgangsstoffe wählt, dass Benzyloxycarbonyl-Phe-His-(2(S)-amino-4-methyl-pentyl)-Val-Ile-His-$NH_2$ gebildet wird.

11. Verfahren gemäss einem der Ansprüche 1-3, dadurch gekennzeichnet, dass man solche Ausgangsstoffe wählt, dass tert-Butylaminocarbonyl-Phe-His-(2(S)-amino-4-methyl-pentyl)-Val-Ile-His-$NH_2$ gebildet wird.

12. Verfahren gemäss einem der Ansprüche 1-3, dadurch gekennzeichnet, dass man solche Ausgangsstoffe wählt, dass Benzyloxycarbonyl-Phe-His-(2(S)-amino-4-methyl-pentyl)-Val-Ile-3-carbamoylbenzylamid gebildet wird.

13. Verfahren gemäss einem der Ansprüche 1-3, dadurch gekennzeichnet, dass man solche Ausgangsstoffe wählt, dass Benzyloxycarbonyl-Phe-His-(2(S)-amino-4-methyl-pentyl)-Val-Ala-Sta-$OCH_3$ gebildet wird.

14. Verfahren gemäss einem der Ansprüche 1-3, dadurch gekennzeichnet, dass man solche Ausgangsstoffe wählt, dass tert-Butoxycarbonyl-Phe-His-(2(S)-amino-4-methyl-pentyl)-Val-Ile-Sta-$NH_2$ gebildet wird.

15. Verfahren gemäss einem der Ansprüche 1-3, dadurch gekennzeichnet, dass man solche Ausgangsstoffe wählt, dass Pivaloyl-Phe-His-(2(S)-amino-4-methyl-pentyl)-Val-Ile-His-$NH_2$ gebildet wird.

16. Verfahren gemäss einem der Ansprüche 1-3, dadurch gekennzeichnet, dass man solche Ausgangsstoffe wählt, dass tert-Butoxycarbonyl-Phe-His-(2(S)-amino-4-methyl-pentyl)-Ser-Ile-His-$NH_2$ gebildet wird.

17. Verfahren zur Herstellung von pharmazeutischen Präparaten, dadurch gekennzeichnet, dass man Verbindungen der Formel I oder pharmazeutisch verwendbare Salze von Verbindungen der Formel I mit salzbildenden Gruppen mit einem pharmazeutischen Trägermaterial mischt.

18. Verfahren zur Herstellung von Verbindungen der Formel

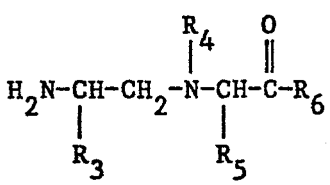

(XI),

worin die Substituenten die unter Formel I genannten Bedeutungen haben, dadurch gekennzeichnet, dass man eine Verbindung der Formel

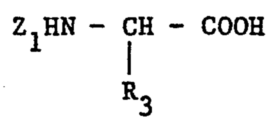

(XII),

worin $Z_1$ eine Aminoschutzgruppe ist und $R_3$ die unter Formel I genannten Bedeutungen hat, oder ein reaktionsfähiges, funktionelles Derivat davon mit einer Verbindung der Formel

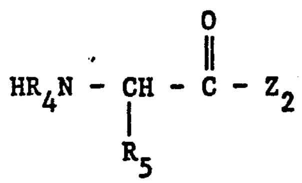 (XIII),

worin $R_4$ und $R_5$ die genannten Bedeutungen haben und $Z_2$ eine Carboxyschutzgruppe ist, unter Bildung einer Amidbindung kondensiert und in einer erhältlichen Verbindung der Formel

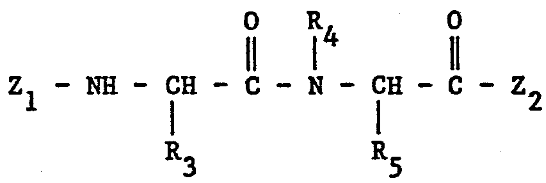 (XIV),

worin die Substituenten die genannten Bedeutungen haben, die Carbonylgruppe der Amidbindung zu einer $-CH_2-$ Gruppe reduziert, in einer erhältlichen Verbindung der Formel

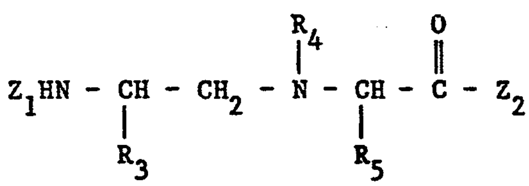 (XV)

in beliebiger Reihenfolge der Reaktionsschritte oder gleichzeitig die Schutzgruppen $Z_1$ und $Z_2$ abspaltet, gewünschtenfalls ein erhältliches Isomerengemisch in die einzelnen Isomere auftrennt und die erhältliche Verbindung mit einer freien Carboxygruppe je nach Bedeutung des einzuführenden Restes $R_6$ amidiert, verestert oder mit einem C-terminal gegebenenfalls amidierten oder veresterten Rest einer Aminosäure, eines Dipeptids oder eines Tripeptids substituiert.

FO 7.4/KB/gs*